# EUROPEAN PATENT SPECIFICATION

(11) **EP 3 169 771 B1**
(45) Date of publication and mention of the grant of the patent: **01.04.2020**
(21) Application number: 15821458.5
(22) Date of filing: 10.07.2015
(51) Int. Cl.: C12N 5/0775

(54) **A METHOD FOR THE REGENERATION AND DIFFERENTIATION OF HUMAN PERINEPHRIC FAT DERIVED MESENCHYMAL STROMAL CELLS INTO ASTROGLIAL, RENAL, NEURONAL AND PANCREATIC PROGENITOR CELLS**
VERFAHREN ZUR REGENERATION UND DIFFERENZIERUNG VON AUS MENSCHLICHEM PERINEPHRITISCHEM FETT STAMMENDEN MESENCHYMALEN STROMAZELLEN IN ASTROGLIALE, RENALE, NEURONALE UND PANKREAS-VORLÄUFERZELLEN
MÉTHODE DE RÉGÉNÉRATION ET DE DIFFÉRENCIATION DE CELLULES STROMALES MÉSENCHYMATEUSES DÉRIVÉES DU TISSU ADIPEUX PÉRINÉPHRIQUE HUMAIN DANS LES CELLULES PROGÉNITRICES ASTROGLIALES, RÉNALES, NEURONALES ET PANCRÉATIQUES

(30) Priority: 14.07.2014 IN 3463CH2014
(43) Date of publication of application: 24.05.2017
(73) Proprietor: Venkataramanaa, Neelam Krishnan, Bangalore, Karnataka 560038 (IN)
(72) Inventor: Venkataramanaa, Neelam Krishnan, Bangalore, Karnataka 560038 (IN)
(74) Representative: Hellmich, Wolfgang
(86) International application number: PCT/IN2015/000281
(87) International publication number: WO 2016/009446

(56) References cited:
- IN-A- 211 MU2 008
- MEINDERT J. CROP ET AL: "Donor-Derived Mesenchymal Stem Cells Suppress Alloreactivity of Kidney Transplant Patients :", TRANSPLANTATION, vol. 87, no. 6, 1 March 2009 (2009-03-01), pages 896-906, XP055434313, GB ISSN: 0041-1337, DOI: 10.1097/TP.0b013e31819b3d72
- ROSSUKON KAEWKHAW ET AL: "Anatomical site influences the differentiation of adipose-derived stem cells for Schwann-cell phenotype and function", GLIA, vol. 59, no. 5, 1 May 2011 (2011-05-01), pages 734-749, XP055436221, ISSN: 0894-1491, DOI: 10.1002/glia.21145
- ALIREZA ABDANIPOUR ET AL: "Induction of adipose-derived stem cell into motoneuron-like cells using selegiline as preinducer", BRAIN RESEARCH, ELSEVIER, AMSTERDAM, NL, vol. 1440, 26 December 2011 (2011-12-26), pages 23-33, XP028455006, ISSN: 0006-8993, DOI: 10.1016/J.BRAINRES.2011.12.051 [retrieved on 2012-01-08]
- M.J. HOOGDUIJN et al.: "Human Heart, Spleen, and Perirenal Fat-Derived Mesenchymal Stem Cells Have Immunomodulatory Capacities", Stem Cells and Development, vol. 16, 2007, pages 597-604, XP009098725, DOI: doi:10.1089/scd.2006.0110
- SCHINKÖTHE TIMO et al.: "In Vitro Secreting Profile of Human Mesenchymal Stem Cells", Stem Cells and Development, vol. 17, 2008, pages 199-205, XP055384250,
- ANA PAULA FRANCO LAMBERT et al.: "Differentiation of human adipose-derived adult stem cells into neuronal tissue: Does it work?", Differentiation, vol. 77, April 2009 (2009-04), pages 221-228, XP025967592, DOI: doi:10.1016/j.diff.2008.10.016
- JESSICA LUNDQVIST et al.: "Optimisation of culture conditions for differentiation of C17.2 neural stem cells to be used for in vitro toxicity tests", Toxicology in Vitro, vol. 27, 2013, pages 1565-1569, XP055384255,
- GREGORY J. BREWER et al.: "Neurobasal? medium/b27 supplement: a new serum-free Medium combination for survival of neurons", Focus, 1994, XP055385021, Retrieved from the Internet: URL:http://homepages.wmich.edu/ ~axm7996/Neurobasal.pdf
- MOHACSIK PETRA et al.: "Thyroid Hormone and the Neuroglia: Both Source and Target", Journal of Thyroid Research, 2011, pages 1-16, XP055385025,
- NARAYANAN KARTHIKEYAN et al.: "Human embryonic stem cells differentiate into functional renal proximal tubular-like cells", Kidney International, vol. 83, 2013, pages 593-603, XP055228557, DOI: doi:10.1038/ki.2012.442
- ARUMUGAM SARASA BHARATI et al.: "Detection of embryonic stem cell markers in adult human adipose tissue-derived stem cells", Indian Journal of Pathology and Microbiology, vol. 54, no. 3, 2011, pages 501-508, XP008185481,
- SACHIN KADAM: "Human amnion mesenchymal stem cells (hAMSCs) - isolation, characterization and islet neogenesis", Ph.D. Thesis NCCS Pune, 2009, pages 66-77, XP055511947,
- Patrick C. Baer ET AL: "Adipose-Derived Mesenchymal Stromal/Stem Cells: Tissue Localization, Characterization, and Heterogeneity", Stem Cells International, vol. 18, supplement, no. 4, 1 January 2012 (2012-01-01), pages S3-11, XP055226401, US ISSN: 1687-966X, DOI: 10.1155/2012/812693

## Description

### BACKGROUND

### Technical field

The embodiments herein generally relates to the field of stem cells. The embodiments herein particularly relates to the regeneration and differentiation of the stem cells into specific cell types. The embodiments herein more particularly relates to the isolation of mesenchymal stromal cells from human perinephric fat and for the regeneration and differentiation into the specific cell types using the isolated mesenchymal stem cells.

### Description of the Related Art

Stem cells are undifferentiated biological cells which can differentiate into specialized cells and can divide to produce more stem cells. The stem cells are found in multi-cellular organisms. In mammals, stem cells can be classified into embryonic, fetal and adult stem cells. The embryonic stem cells are isolated from the inner cell mass of the blastocyst stage during embryonic development. The fetal stem cells are derived from the extra-embryonic structures like umbilical cord, placenta, amnion, umbilical cord blood etc. The adult stem cells are isolated from the various tissues of adult human body, for example: bone marrow, adipose tissue and blood. These stem cells along with specialized progenitor cells act as a repair system for the body, during regular wear and tear and after an insult.

The organ and tissue generation from the stem cells and their subsequent transplantation provide the treatments for a number of pathologies, thereby making the stem cells a central focus of research in many fields. The stem cell technology provides a promising alternative therapy for diabetes, Parkinson's disease, liver disease, heart disease, kidney disorders and autoimmune disorders etc. But there are two major problems associated with organ and tissue transplantation.

The first major problem is a shortage of donor's for organs and tissues. The second major problem is in the potential incompatibility of the transplanted tissue with the immune system of the recipient. Because the donated organ or tissue is recognized as foreign by the host immune system, the immunosuppressive medications must be provided, to the patient at a significant cost, both financially and physically.

The most promising source of organs and tissues for transplantation therefore lies in the development of stem cell technology. The Stem cells undergo self renewing cell division to give rise to phenotypically and genotypically identical daughter cells for an indefinite time and may differentiate into different cell types.

The isolation of adult stem cells from autologous source by harvesting involves least risk. The autologous stem cells are obtained from one's own body. The stem cells find their application in the treatment of neurodegenerative disorders such as Parkinson's disease, amyotrophic lateral sclerosis (ALS) and Alzheimer's disease; brain and spinal cord injury; cardiac problems (especially the generation of heart muscle cells); stimulation of growth of new blood vessels to repopulate damaged heart tissue or other tissues in body; secretion of growth factors; blood cell formation; renal failure and disorders; hair regeneration and growth; blindness and vision impairment; and diabetes.

For the neurodegenerative disorders, there are no treatments for slowing the progress of degeneration or to stop the brain cell damage. The current therapies are symptomatic and are unable to treat the disease. The drugs such as Levodopa, Entacapone, Dopamine agonists, Monoamine oxidase inhibitors are mainly used to control the symptoms. Also these drugs have several side effects and lose their efficiency over the years. The side effects caused by these drugs include swelling, sleepiness or compulsive behaviors, elevated blood pressure etc.

The current treatment of chronic kidney disorder (CKD) and renal failure mainly involves kidney transplant, and haemo-dialysis. The costs of the kidney transplant and haemo-dialysis are high. For kidney transplant, a donor search and the judicial work is cumbersome and time consuming. Further, the patient and the patient's family are economically and mentally stressed.

The pancreatic malfunction leads to blood sugar disorder or diabetes. The treatments known for pancreatic malfunction mainly include administration of drugs, injection of insulin and the dietary changes for the diabetic patient. The most commonly used drugs to control diabetes are sulfonylureas, biguanides/metformin, alpha glucosidase inhibitors, thiazolidinediones, meglitinides. The drugs can cause many side effects on the health of the diabetic patient such ashypoglycemia, stomach upset, skin rash, itching, weight gain, kidney complications, dizziness, tiredness, risk of liver disease, anemia, swelling of legs or ankles etc. Further the cost of drugs and the financial costs involved have a huge burden on the patient and the family.

Attention is given to adipose derived stem cells (ASC) as an important source for the multilineage differentiation. Three major anatomical sites are used to derive the stem cells from the adipose tissue. They are (i) subcutaneous adipose tissue (ii) perinephric fat tissue, and (iii) epidymal adipose tissue.

The perinephric fat is a layer of adipose tissue present around the kidneys in the adult human body. When the donor kidney is prepared for transplantation, the perinephric fat tissue is surgically dissected and removed from around the kidney and collected in a sterile transport container. The adipose or mainly perinephric derived stem cells are advantageous over bone marrow, as there is abundance of the mesenchymal stem cells and ease of isolation of the perinephric fat tissue. The isolation of the perinephric fat is easy when compared to the complex procedure involved in the isolation of the bone marrow or blood stem cells. Further the perinephric fat derived stem cells act as an autologous source for the tissue and the stem cells. The perinephric fat derived stem cells secrete the growth factors namely vascular endothelial growth factor (VEGF), Granulocyte colony stimulating factor (G-CSF) and Platelet derived growth factor (PDGF). These growth factors have a specific role such as VEGF stimulates angiogenesis, G-CSF stimulates and mobilizes of stem cells, PDGF stimulates cell proliferation, stem cell recruitment and neo-vascularization. Hence, these growth factors stimulate the stem cells to differentiate into specific cell lineages.

Hence, there is a need for the isolation of the stem cells from the perinephric fat. Also there is a need for a method for subjecting the human perinephric fat cells derived mesenchymal stromal cells for the differentiation into renal progenitor cells, pancreatic islet like cluster cells, neural progenitor cells and astroglial progenitor cells.

The above mentioned shortcomings, disadvantages and problems are addressed herein and which will be understood by reading and studying the following specification.

### OBJECTIVES OF THE EMBODIMENTS

The primary objective of the present invention is to isolate the human perinephric fat derived mesenchymal stromal cells from the perinephric fat.

Another objective of the present invention is to subject the human perinephric fat derived mesenchymal stromal cells to expansion process.

Yet another objective of the present invention is characterizing the human perinephric fat derived mesenchymal stromal cells.

Yet another objective of the present invention is to confirm that the isolated human perinephric fat cells are mesenchymal stromal cells.

Yet another objective of the present invention is to analyze the secretome profiling of the human perinephric fat derived mesenchymal stromal cells.

Yet another objective of the present invention is to differentiate the human perinephric fat cells derived mesenchymal stromal cells into the renal progenitor cells, pancreatic islet like clusters, neural progenitor cells and the astroglial progenitor cells.

Yet another objective of the present invention is to target the specific pathways involved in differentiation of perinephric fat derived mesenchymal stromal cells into specific cell lineages.

Yet another objective of the present invention is to target the Wnt pathway for inducing the differentiation of the stromal cells into renal cell lineage.

Yet another objective of the present invention is to use thyroid hormone T4 for inducing the differentiation of the stromal cells into astroglial and neural cell lineages.

Yet another objective of the present invention is to use thyroid hormone T4 as an inducing agent for astroglial differentiation and re-myelination.

Yet another objective of the present invention is to use Insulin-Transferrin-Selenium, Taurine, Nicotinamide and Glucagon like peptide 1 as an inducing agent for pancreatic islet like clusters.

These and other objects and advantages of the embodiment herein will become readily apparent from the following detailed description taken in conjunction with the accompanying drawings.

### SUMMARY

The various embodiments herein provide a method for the regeneration and differentiation of human perinephric fat derived mesenchymal stromal cells into astroglial, pancreatic islet like clusters, renal and neuronal progenitor cells. The perinephric fat derived stem cells are advantageous over bone marrow or other stem cell source due to the abundance and ease of isolation. The perinephric fat derived stromal cells find their applications in the treatment of neurodegenerative disorder, renal disorder and pancreatic malfunction.

According to one embodiment herein, the method for isolating human perinephric fat derived stem/stromal cells (hPNF-MSCs), for propagation, regeneration and differentiation into astroglial cells, renal cells, neuronal cells and pancreatic islet like clusters comprises of the following steps. The perinephric fat tissue is collected from a volunteer or a kidney patient with a prior informed consent. The perinephric fat tissue is processed in a sterile environment. The sterile environment comprises Current Good Manufacturing Practices (cGMP) compliant clean room with a biosafety cabinet. The mesenchymal stem/stromal cells are isolated from the perinephric fat tissue using plurality of methods. The plurality of method includes mechanical method and enzymatic method. The tissue sample is washed in a IX Dulbecco's phosphate Buffered Saline (DPBS) solution comprising of antibiotics at a concentration of 2X concentration. The tissue sample is washed for a plurality of times. The antibiotic is Pens-Strep. The tissue sample is minced mechanically into plurality of small pieces with sterile scalpel blade. The tissue sample is treated in a Dulbecco's Modified Eagles medium (DMEM-KO) comprising enzyme and the Pen-Strep. The tissue sample is treated for 60 minutes at 37 °C on a laboratory rocker. The enzyme is a Collagenase type I. The Collagenase type I is mixed at predetermined concentration. The predetermined concentration of Collagenase type I is 0.2% v/v. The DMEM-KO medium is added with a fetal bovine serum (FBS) to neutralize an enzymatic action of Collagenase. The FBS is present in predetermined concentration. The predetermined concentration of FBS is 5% v/v. The DMEM-KO medium with the FBS neutralizes the enzymatic action of Collagense. Homogenate is obtained comprising mesenchymal stem/stromal cells from the perinephric fat tissues. The homogenate is filtered comprising mesenchymal stem/stromal cells from the perinephric fat tissues through a cell strainer to obtain filtrate. The cell strainer has pore size of 40µm. The filtrate is collected comprising mesenchymal stem/stromal cells from the perinephric fat tissues. The filtrate is centrifuged comprising mesenchymal stem/stromal cells at 1500 rpm for 2 minutes. The cell pellet is collected comprising mesenchymal stem/stromal cells after completion of centrifuging process. The pellet is re-suspended in a culture medium. The culture medium comprises DMEM-KO, fetal bovine serum (FBS) at a concentration of 10 %, L-glutamate at a concentration of IX, Pen-Strep, and basic fibroblast growth factor (bFGF) at predetermined concentration. The predetermined concentration of bFGF is 1 ng/ml. The cell suspension is obtained of mesenchymal stem/stromal cells. The mesenchymal stem/stromal cell suspension is seeded in a cell stack. The cell stack comprises 200 ml cell culture medium. The cell stack is rocked for even distribution of cells and culture medium. The cell stacks are incubated in humidified incubator at a temperature of 37°C. The humidified incubator consists of a carbon dioxide (CO₂). The concentration of CO₂ is 5%v/v. The culture medium is replenished after 48 hours. The culture medium is replenished to eliminate non adherent cells. The adherent fibroblast like populations of cells are allowed to expand in culture medium until cellular confluency of 80% is reached for harvesting. The culture medium is replenished 2-3 times every week. The hPNF-MSCs are trypsininzed and harvested from the cell stacks, after the cells attain a confluency of 80%. The mesenchymal stem/stromal cell population is counted. The hPNF-MSCs are cryopreserved in cryovial. The cryovial is labeled with sample ID, cell number, date of isolation. The cryovial is labeled as passage 0 (P0). The hPNF-MSCs are subjected for expansion. The hPNF-MSCs are subjected for characterization.

According to one embodiment herein, the step of cryopreserving comprises of the following steps. A freezing mixture is prepared. The freezing mixture comprises of fetal bovine serum at a concentration of 90% v/v, dimethyl sulfoxide 10%v/v and mesenchymal stem cells (MSC). The mixture is freezed at a temperature of 4°C. The cell suspension is centrifuged at 1400 rpm for 10 min at room temperature. The cell pellet is obtained and the supernatant is discarded. The freezing mixture is added dropwise to the cell pellet. The freezing mixture and cell pellet are mixed. The mixture is transferred in a cryovial. The cryovial is incubated at a temperature of -80°C. The cryovial is transferred into liquid nitrogen cryotank.

According to one embodiment herein, the isolated human perinephric fat derived stem/stromal cells (hPNF-MSCs) are subjected for characterization for confirming the cells to be the mesenchymal stem/stromal cells. The isolated human perinephric fat derived stem/stromal cells (hPNF-MSCs) are analyzed for seeding density analysis, morphological analysis, growth kinetic study, immunophenotypic analysis, differentiation potential analysis, karyotype analysis and secretome profile analysis.

According to one embodiment herein, the isolated human perinephric fat derived stem/stromal cells (hPNF-MSCs) are seeded at different densities in the seeding density analysis. The cells are seeded at low density and high density. The cells seeded at low density have 1000 cells/cm2. The cells seeded at high density have 2500 cells/cm2. The cells seeded at high density maintain growth kinetics and cellular morphology. The high density cell culture has cell senescence at passage 10 (P10).

According to one embodiment herein, the morphology of the isolated human perinephric fat derived stem/stromal cells (hPNF-MSCs) is similar to the morphology of a fibroblast. The hPNF-MSCs have the fibroblast morphology till a passage 7 (P7).

According to one embodiment herein, the cell population doubling time for the isolated human perinephric fat derived stem/stromal cells (hPNF-MSCs) is determined from passage 1 (P1) to passage 14 (P14). The population doubling time of the cells increases from 14.38±1.29 hours at passage 2 (P2) to 235.85±0.65 hours at passage 9 (P9).

According to one embodiment herein, the isolated human perinephric fat derived stem/stromal cells (hPNF-MSCs) are analyzed/characterized for cell surface antigen marker. The hPNF-MSCs express CD44 antigen and CD90 antigen at passage 0 (P0). The cells express HLA-DR antigen, CD 34 antigen and CD 45 antigen in less than 4% of the cell population at passage 5 (P5). 90% of the cell population expresses CD 44 antigen, CD 73 antigen, CD 90 antigen, CD 105 antigen and CD 166 antigen at the passage 5 (P5). The cells have a low expression of the cell surface antigen at passage 9 (P9) and passage 15 (P15).

According to one embodiment herein, the secretome profile analysis of the isolated human perinephric fat derived stem/stromal cells (hPNF-MSCs) comprises analysis of cytokines, chemokines and growth factors.

According to one embodiment herein, the cytokines analyzed are pro-inflammatory cytokine and anti-inflammatory cytokine. The pro-inflammatory cytokine secreted by the hPNF-MSCs secrete IL-6, IL-9, IL-12, INF-α2, and IL-1α. The IL-6 secretion is high in PNF-MSCs when compared to the other pro-inflammatory cytokine. The anti-inflammatory cytokine secreted by hPNF-MSCs are IL-4, IL-10 and IL-1Ra. The IL-13 secretion is high in hPNF-MSCs when compared to other anti-inflammatory cytokine.

According to one embodiment herein, the hPNF-MSC secretes plurality of chemokine including MDC, Fractalkine, IP-10, MIP-1β, eotaxin, PCP-1, MCP-3 and GRO.

According to one embodiment herein, the growth factors secreted by hPNF-MSC are G-CSF, GM-CSF, VEGF and PDGF-aa. The secretion of VEGF is at concentration of 2000-4000 pg/ml.

According to one embodiment herein, the method for differentiating the isolated human perinephric fat derived stem/stromal cells (hPNF-MSCs) into neural cells, comprises the following steps. The spheres are generated with a basic fibroblast growth factor (bFGF) at concentration of 1 ng/ml in DMEM-MO medium. The DMEM-KO medium comprises fetal bovine serum (FBS) at concentration of 1% v/v. The generated spheres are incubated in the medium for 24 hours. The growth medium is replaced with neural induction medium. The neural induction medium comprises DMEM-F12 culture medium. The DMEM-F12 medium comprises fetal bovine serum (FBS) at concentration of 1%v/v, N2 at concentration of 1% v/v, B27 at concentration of 2% and bFGF at concentration of 20 ng/mL. The FBS is nutritional supplement. The N2 is a cocktail of the growth factor. The N2 comprises insulin at concentration of 5µg/ml, transferrin at a concentration of 100 µg/ml, progesterone at concentration of 20 nM, putrescine at a concentration of 100 µM, selenium at concentration of 30 nM, B27 at a concentration of 2% v/v, and bFGF at concentration of 20ng/mL. The selenium is added for growth and maintenance of neurons in vitro. The B27 is growth supplement for neurons in vitro. The bFGF is cell differentiating agent. The bFGF is cell differentiating agent. The bFGF induces cell differentiation of the hPNF-MSCs into neuronal progenitor cells. The bFGF targets Wnt pathway. The neural induction medium is replenished after every 3-4 days. The cells are fixed with at a concentration of 4%v/v for characterization. The induction procedure is performed on a monolayer culture of the cells.

According to one embodiment herein, the isolated human perinephric fat derived stem/stromal cells (hPNF-MSCs) have positive expression for neural markers including nestin (78.12 ± 16%), NSE (75.68 ± 6.18%), β III tubulin (85.56 ± 10.91%). The neural marker expression confirms the differentiation of the hPNF-MSCs into neural cells.

According to one embodiment herein, the method for differentiating the isolated human perinephric fat derived stem/stromal cells (hPNF-MSCs) into astroglial cells, comprises the following steps. The spheres are generated with basic fibroblast growth factor (bFGF) at concentration of 1 nm/mL in DMEM-KO medium. The DMEM-KO medium is supplemented with fetal bovine serum (FBS) at concentration of 1% v/v. The generated spheres are incubated in the medium for 24 hours. The growth medium is replaced with an astroglial induction medium and L-thyroxine. The astroglial induction medium comprises FBS at concentration of 1% v/v, N2 at concentration of 1% v/v and T4 at concentration of 30 ng/mL. The FBS is a nutritional supplement. The N2 is a cocktail of the growth factors. The N2 comprises insulin at concentration of 5µg/ml, progesterone at concentration of 20nM, putrescine at concentration of 100 µM, selenium at concentration of 30nM, B27 at concentration of 2% v/v and bFGF at concentration of 20 ng/mL. The selenium is added for growth and maintenance of astroglial cells in vitro. The B27 is growth supplement for astroglial cells in vitro. The bFGF is cell differentiating agent. The bFGF induces cell differentiation of the hPNF-MSCs into astroglial progenitor cells. The T4 induces the differentiation of hPNF-MSCs into astroglial cells. The bFGF targets Wnt pathway. The astroglial induction medium is replenished after every 3-4 days. The cells are fixed with 4% PFA for characterization. The induction procedure is performed on monolayer culture of the cells.

According to one embodiment herein, the isolated human perinephric fat derived stem/stromal cells (hPNF-MSCs) show expression of GFAP (53.31 ± 14.11%) astroglial marker. The astroglial marker expression confirms the differentiation of the hPNF-MSCs into neural cells.

According to one embodiment herein, the method for differentiating the isolated human perinephric fat derived stem/stromal cells (hPNF-MSCs) into renal cells, comprises the following steps. The hPNF-MSCs are cultured on autoclaved coverslips in 35mm culture dishes comprising renal epithelial growth medium. The culture dishes are incubated at 37°C in CO₂ incubator for 17 days for identifying the differentiation of hPNF-MSC to metanephric mesenchyme. The CO₂ is at a concentration of 5%. The culture dishes are incubated at 37°C in a CO₂ incubator for 22 days for identifying the differentiation of hPNF-MSCs to podocyte and proximal convulated tubule. The CO₂ is at concentration of 5%. The culture medium is replenished after every 48 hours. The renal epithelium growth medium comprises BMP2 at concentration of 10 nm/ml, retinoic acid at concentration of 0.1µM/ml and Activin A at a concentration of 10ng/ml. The BMP2, retinoic acid, and Activin A are the growth factors which induce differentiation in the hPNF-MSC to form renal cells. The Activin A induces mesoderm specification. The retinoic acid induces the mesoderm patterning. The retinoic acid targets retinoid receptors RAR. The BMP2 induces nephrogenesis. The BMP2 is expressed in metanephric mesenchyme. The BMP2 induces epithelialization of metanephric mesenchyme. The syringe filtration is performed of the renal epithelium differentiation medium using a 0.22 micron filter. The hPNF-MSCs are incubated at a temperature of 37°C in CO₂ incubator for 17 days to identify the differentiation of hPNF-MSCs into metanephric mesenchyme cells. The concentration of CO₂ is 5%. The hPNF-MSCs are incubated at a temperature of 37°C in incubator for 22 days for a podocyte and a proximal convulated tubule differentiation, wherein the incubator has CO₂ at concentration of 5%. The renal epithelium differentiation medium is replenished after every 48 hours.

According to one embodiment herein, the Wnt pathway is targeted for differentiating the human perinephric fat derived stem/stromal cells (hPNF-MSCs) into renal cell. The Wnt pathway proteins expressed during renal development are Wnt 2b, Wnt 9. The BMP2, Activin A and Retinoic acid growth factors activate the Wnt signaling pathway enabling differentiation of hPNF-MSCs to renal progenitor cells.

According to one embodiment herein, the method for differentiating the isolated human perinephric fat derived stem/stromal cells (hPNF-MSCs) into islet like clusters comprises of the following steps. For differentiating hPNF-MSCs to islet like clusters, cells from passage 3 (P3) are seeded into three 35 mm non adherent culture dishes. One dish is control and the other two dishes are for differentiation. The control dish is un-induced dish. The control dish is supplemented with prepared medium without FBS and bFGF. The control dish comprises only Dulbecco's Modified Eagle's Medium-Knock Out (DMEM-KO) medium. The induction dish comprises Day 0 medium comprising DMEM-KO, 1.5% BSA, IX ITS. The control cell culture dish/plate (un-induced culture dish/plate) is supplemented with a cell culture medium comprising Dulbecco's Modified Eagle's Medium-Knock Out (DMEM-KO).The two cell differentiation plates/dishes are supplemented with a culture medium comprising Dulbecco's Modified Eagle's Medium-Knock Out (DMEM-KO), bovine serum albumin (BSA) in a concentration of 1.5%v/v, insulin-transferrin-selenium (ITS) in a concentration of 1X. The two cell differentiation culture dish/plate and one control cell culture dish/plate are incubated at 37°C for four days in CO2 incubator. The CO2 is at a concentration of 5%. The floating cells are collected with culture medium in a centrifuge tube after four days. The cells are allowed to settle down in the centrifuge tube for 15-20 minutes. The cells settle down to form pellet and the culture medium forms supernatant.

The supernatant is decanted, and the cell pellet is collected. The cells from the cell pellet are re-seeded in 4 day medium. The 4-day medium comprises a Dulbecco's Modified Eagle's Medium (DMEM-KO), bovine serum albumin (BSA) in a concentration of 1.5%, insulin-transferrin-selenium (ITS) in a concentration of IX and Taurine in a concentration of 0.3 mM. The cells are incubated at 37°C in CO2 incubator for further three days. The cells are incubated for a total of seven days. The CO2 is at a concentration of 5%. The cells are fed with a fresh 4 day medium after seven days. The cells are incubated at 37°C in a CO2 incubator for further three days. The cells are incubated for a total of ten days. The CO2 is at a concentration of 5%. The floating cell clusters are collected with a culture medium in a sterile centrifuge tube after 10 days. The cells are allowed to settle for 15-20 minutes at room temperature. The cells settle down to form the pellet and the culture medium forms the supernatant. The supernatant is decanted. The cell pellet comprises cell clusters. The cell clusters are re-seeded in a medium comprising DMEM-KO, BSA at a concentration of 1.5% v/v, ITS at a concentration of IX, 100nM nicotine amide at a concentration of 100 nM, taurine at a concentration of 3nM and a glucagon like peptide (GLP) at a concentration of 100 nM. The cells are subjected for dithiozone (DTZ) staining analysis and immunofluorescence analysis. The cell clusters are islet cell clusters.

According to one embodiment herein, the islet cell clusters are characterized by dithiozone (DTZ) staining. The islet cell clusters are positive for DTZ staining. The DTZ is a zinc chelating agent that selectively stains pancreatic beta cells.

According to one embodiment herein, the islet cell clusters are characterized by immunofluorescence staining for c-peptide. The cell clusters show a high expression of c-peptide, and the expression of c-peptide confirms the generation of functional islet cell clusters.

These and other aspects of the embodiments herein will be better appreciated and understood when considered in conjunction with the following description and the accompanying drawings. It should be understood, however, that the following descriptions, while indicating preferred embodiments and numerous specific details thereof, are given by way of illustration and not of limitation. Many changes and modifications may be made within the scope of the embodiments herein without departing from the spirit thereof, and the embodiments herein include all such modifications.

### BRIEF DESCRIPTION OF THE DRAWINGS

The other objects, features and advantages will occur to those skilled in the art from the following description of the preferred embodiment and the accompanying drawings in which:
**FIG.1A** and **FIG.1B** jointly illustrates a flow chart explaining a method for isolating the human perinephric fat-mesenchymal stem cells (hPNF-MSCs), according to an embodiment herein.
**FIG.2** illustrates a flow chart explaining a method for characterization and confirmation of cells derived and cultured from human perinephric fat mesenchymal stem cells (hPNF-MSC), according to an embodiment herein.
**FIG.3** illustrates a graph indicating the population doubling time of the high and low seeding density culture, according to an embodiment herein.
**FIG.4** illustrates a graph indicating the morphology of the human perinephric fat mesenchymal stem cells (hPNF-MSCs), according to an embodiment herein.
**FIG.5** illustrates a graph indicating the growth kinetics of the human perinephric fat mesenchymal stem cells (hPNF-MSCs), according to an embodiment herein.
**FIG.6** illustrates a flow cytometry histogram of surface marker expression by human perinephric fat mesenchymal stem cells (hPNF-MSCs) from early passage (P0 and P5), according to an embodiment herein.
**FIG.7** illustrates a flow cytometry histogram of surface marker expression by human perinephric fat mesenchymal stem cells (hPNF-MSCs) from late passage, according to an embodiment herein.
**FIG.8** illustrates a photograph indicating the multipotent differentiation human perinephric fat mesenchymal stem cells (hPNF-MSCs), according to an embodiment herein.
**FIG.9** illustrates the karyotype analysis of the human perinephric fat mesenchymal stem cells (hPNF-MSC), according to an embodiment herein.
**FIG.10** illustrates a graph indicating the comparison of the pro-inflammatory cytokines secreted by human perinephric fat mesenchymal stem cells (hPNF-MSCs) derived from perinephric fat (PNF) and sub-cutaneous fat (LA), according to an embodiment herein.
**FIG.11** illustrates a graph indicating the comparison of the anti-inflammatory cytokines secreted by human perinephric fat mesenchymal stem cells (hPNF-MSC) derived from perinephric fat (PNF) and sub-cutaneous fat (LA), according to an embodiment herein.
**FIG.12** illustrates a graph indicating the comparison of the chemokine secreted by human perinephric fat mesenchymal stem cells (hPNF-MSC) derived from perinephric fat (PNF) and sub-cutaneous fat (LA), according to an embodiment herein.
**FIG.13** illustrates a graph indicating the comparison of the growth factors secreted by human perinephric fat mesenchymal stem cells (hPNF-MSC) derived from perinephric fat (PNF) and sub-cutaneous fat (LA), according to an embodiment herein.
**FIG.14** illustrates a graph indicating the comparison of the other bioactive factors secreted by human perinephric fat mesenchymal stem cells (hPNF-MSC) derived from perinephric fat (PNF) and sub-cutaneous fat (LA), according to an embodiment herein.
**FIG.15** illustrates a photograph indicating the differentiation in neural and astroglial lineages, according to an embodiment herein.
**FIG.16** illustrates a photograph indicating the induction of neural and astroglial lineages, according to an embodiment herein.
**FIG.17A** illustrates a graph indicating the flow cytometry analysis of the neuro-glial markers in the human perinephric fat mesenchymal stem cells (hPNF-MSCs) derived by lipoaspiration (LA) control (early passage), according to an embodiment herein.
**FIG.17B** illustrates a graph indicating the flow cytometry analysis of the neuro-glial markers in the human perinephric fat mesenchymal stem cells (hPNF-MSCs) derived by perinephric fat (PNF) (early passage), according to an embodiment herein.
**FIG.18** illustrates a photograph indicating a generation of spheroids from human perinephric fat mesenchymal stem cells (hPNF-MSCs), according to an embodiment herein.
**FIG.19** illustrates a flow chart indicating the differentiation of human perinephric derived mesenchymal stem cells into the renal cells lineages, according to an embodiment herein.
**FIG.20A** illustrates a photograph indicating the phase contrast image of the adherent renal progenitor cells, according to an embodiment herein.
**FIG.20B** illustrates a photograph indicating the immune fluroscence image of the renal progenitor cells, according to an embodiment herein.
**FIG.21A** illustrates a photograph indicating the phase contrast image of the spheroid renal progenitor cells, according to an embodiment herein.
**FIG.21B** illustrates a photograph indicating the immune-fluroscence image of the spheroid renal progenitor cells, according to an embodiment herein.
**FIG.22A** and **FIG.22B** jointly illustrates a flow chart explaining a method for differentiating the human perinephric fat derived mesenchymal stem cells (hPNF-MSCs) into pancreatic progenitor cells, according to an embodiment herein.
**FIG.23** illustrates a photograph indicating the morphology of islet like cell clusters in the differentiation culture plates resembling pancreatic islets, according to an embodiment herein.
**FIG.24** illustrates a photograph indicating the morphology of uninduced human perinephric fat derived mesenchymal stem cells (hPNF-MSCs) (control for differentiation culture plate), according to an embodiment herein.
**FIG.25A** and **FIG.25B** illustrate photographs indicating the characterization of islet like cell clusters generated from human perinephric fat derived mesenchymal stem cells (hPNF-MSCs) by dithizone (DTZ) staining, according to an embodiment herein.
**FIG.26** illustrates a photograph indicating the characterization of islet like cell clusters generated from human perinephric fat derived mesenchymal stem cells (hPNF-MSCs) by immunofluorescence staining for C-peptide, according to an embodiment herein.

Although the specific features of the embodiments herein are shown in some drawings and not in others. This is done for convenience only as each feature may be combined with any or all of the other features in accordance with the embodiments herein.

### DETAILED DESCRIPTION OF THE EMBODIMENTS HEREIN

In the following detailed description, reference is made to the accompanying drawings that form a part hereof, and in which the specific embodiments that may be practiced is shown by way of illustration. These embodiments are described in sufficient detail to enable those skilled in the art to practice the embodiments and it is to be understood that the logical, mechanical and other changes may be made without departing from the scope of the embodiments. The following detailed description is therefore not to be taken in a limiting sense.

The various embodiments herein provide a method for the regeneration and differentiation of human perinephric fat derived mesenchymal stromal cells into renal progenitor cells, islet like clusters, neural progenitor cells and the astroglial progenitor cells. The adipose or perinephric fat derived stem cells are advantageous over bone marrow or other stem cell source due to the abundance and ease of isolation. The perinephric fat derived stromal cells find their application in the treatment of neurodegenerative disorder, renal disorder and pancreatic malfunction.

According to one embodiment herein, the method for isolating human perinephric fat derived stem/stromal cells (hPNF-MSCs), for propagation, regeneration and differentiation into astroglial cells, renal cells, neuronal cells and pancreatic islet like clusters, comprises of the following steps. The perinephric fat tissue is collected from a volunteer or a kidney patient with a prior informed consent. The perinephric fat tissue is processed in a sterile environment. The sterile environment comprises Current Good Manufacturing Practices (cGMP) compliant clean room with a biosafety cabinet. The mesenchymal stem/stromal cells are isolated from the perinephric fat tissue using plurality of methods. The plurality of method includes mechanical method and enzymatic method. The tissue sample is washed in a IX Dulbecco's phosphate Buffered Saline (DPBS) solution comprising of antibiotics at a concentration of 2X concentration. The tissue sample is washed for a plurality of times. The antibiotic is Pens-Strep. The tissue sample is minced mechanically into plurality of small pieces with sterile scalpel blade. The tissue sample is treated in a Dulbecco's Modified Eagles medium (DMEM-KO) comprising enzyme and the Pen-Strep. The tissue sample is treated for 60 minutes at 37°C on a laboratory rocker. The enzyme is a Collagenasetype I. The Collagenase type I is mixed at predetermined concentration. The predetermined concentration of Collagenase type I is 0.2% v/v. The DMEM-KO medium is added with a fetal bovine serum (FBS) to neutralize an enzymatic action of Collagenase. The FBS is present in predetermined concentration. The predetermined concentration of FBS is 5% v/v. The DMEM-KO medium with the FBS neutralizes the enzymatic action of Collagenase. Homogenate is obtained comprising mesenchymal stem/stromal cells from the perinephric fat tissues. The homogenate is filtered comprising mesenchymal stem/stromal cells from the perinephric fat tissues through a cell strainer to obtain filtrate. The cell strainer has pore size of 40µm. The filtrate is collected comprising mesenchymal stem/stromal cells from the perinephric fat tissues. The filtrate is centrifuged comprising mesenchymal stem/stromal cells at 1500 rpm for 2 minutes. The cell pellet is collected comprising mesenchymal stem/stromal cells after completion of centrifuging process. The pellet is re-suspended in a culture medium. The culture medium comprises DMEM-KO, fetal bovine serum (FBS) at a concentration of 10 %, L-glutamine at a concentration of 1X, Pen-Strep, and basic fibroblast growth factor (bFGF) at predetermined concentration. The predetermined concentration of bFGF is 1 ng/ml. The cell suspension is obtained of mesenchymal stem/stromal cells. The mesenchymal stem/stromal cell suspension is seeded in a cell stack. The cell stack comprises 200 ml cell culture medium. The cell stack is rocked for even distribution of cells and culture medium. The cell stacks are incubated in humidified incubator at a temperature of 37°C. The humidified incubator consists of a carbon dioxide (CO₂). The concentration of CO₂ is 5%v/v. The culture medium is replenished after 48 hours. The culture medium is replenished to eliminate non adherent cells. The adherent fibroblast like populations of cells are allowed to expand in culture medium until cellular confluency of 80% is reached for harvesting. The culture medium is replenished 2-3 times every week. The hPNF-MSCs are trypsinized and harvested from the cell stacks, after the cells attain a confluency of 80%. The mesenchymal stem/stromal cell population is counted. The hPNF-MSCs are cryopreserved in cryovial. The cryovial is labeled with sample ID, cell number, date of isolation. The cryovial is labeled as passage 0 (P0). The hPNF-MSCs are subjected for expansion. The hPNF-MSCs are subjected for characterization.

According to one embodiment herein, the step of cryopreserving comprises of the following steps. A freezing mixture is prepared. The freezing mixture comprises of fetal bovine serum at a concentration of 90% v/v, dimethyl sulfoxide 10%v/v and mesenchymal stem cells (MSC). The mixture is cooled at a temperature of 4°C. The cell suspension is centrifuged at 1400 rpm for 10 min at room temperature. The cell pellet is obtained and the supernatant is discarded. The freezing mixture is added dropwise to the cell pellet. The freezing mixture and cell pellet are mixed. The mixture is transferred in a cryovial. The cryovial is incubated at a temperature of -80°C. The cryovial is transferred into liquid nitrogen cryotank.

According to one embodiment herein, the isolated human perinephric fat derived stem/stromal cells (hPNF-MSCs) are subjected for characterization for confirming the cells to be the mesenchymal stem/stromal cells. The isolated human perinephric fat derived stem/stromal cells (hPNF-MSCs) are analyzed for seeding density analysis, morphological analysis, growth kinetic study, immunophenotypic analysis, differentiation potential analysis, karyotype analysis and secretome profile analysis.

According to one embodiment herein, the isolated human perinephric fat derived stem/stromal cells (hPNF-MSCs) are seeded at different densities in the seeding density analysis. The cells are seeded at low density and high density. The cells seeded at low density have 1000 cells/cm2. The cells seeded at high density have 2500 cells/cm2. The cells seeded at high density maintain growth kinetics and cellular morphology. The high density cell culture has cell senescence at passage 10 (P10).

According to one embodiment herein, the morphology of the isolated human perinephric fat derived stem/stromal cells (hPNF-MSCs) is similar to the morphology of a fibroblast. The hPNF-MSCs have the fibroblast morphology till a passage 7 (P7).

According to one embodiment herein, the cell population doubling time for the isolated human perinephric fat derived stem/stromal cells (hPNF-MSCs) is determined from passage 1 (P1) to passage 14 (P14). The population doubling time of the cells increases from 14.38±1.29 hours at passage 2 (P2) to 235.85±0.65 hours at passage 9 (P9).

According to one embodiment herein, the isolated human perinephric fat derived stem/stromal cells (hPNF-MSCs) are analyzed/characterized for cell surface antigen marker. The hPNF-MSCs express CD44 antigen and CD90 antigen at passage 0 (P0). The cells express HLA-DR antigen, CD 34 antigen and CD 45 antigen in less than 4% of the cell population at passage 5 (P5). 90% of the cell populations express CD 44 antigen, CD 73 antigen, CD 90 antigen, CD 105 antigen and CD 166 antigen at the passage 5 (P5). The cells have a low expression of the cell surface antigen at passage 9 (P9) and passage 15 (P15).

According to one embodiment herein, the secretome profile analysis of the isolated human perinephric fat derived stem/stromal cells (hPNF-MSCs) comprises analysis of cytokines, chemokines and growth factors.

According to one embodiment herein, the cytokines analyzed are pro-inflammatory cytokine and anti-inflammatory cytokine. The pro-inflammatory cytokine secreted by the hPNF-MSCs secrete IL-6, IL-9, IL-12, INF-α2, and IL-1α. The IL-6 secretion is high in PNF-MSCs when compared to the other pro-inflammatory cytokine. The anti-inflammatory cytokine secreted by hPNF-MSCs are IL-4, IL-10 and IL-1Ra. The IL-13 secretion is high in hPNF-MSCs when compared to other anti-inflammatory cytokine.

According to one embodiment herein, the hPNF-MSC secretes plurality of chemokine including MDC, Fractalkine, IP-10, MIP-1β, eotaxin, PCP-1, MCP-3 and GRO.

According to one embodiment herein, the growth factors secreted by hPNF-MSC are G-CSF, GM-CSF, VEGF and PDGF-aa. The secretion of VEGF is at concentration of 2000-4000 pg/ml.

According to one embodiment herein, the method for differentiating the isolated human perinephric fat derived stem/stromal cells (hPNF-MSCs) into neural cells, comprises the following steps. The spheres are generated with a basic fibroblast growth factor (bFGF) at concentration of 1 ng/ml in DMEM-MO medium. The DMEM-KO medium comprises fetal bovine serum (FBS) at concentration of 1% v/v. The generated spheres are incubated in the medium for 24 hours. The growth medium is replaced with neural induction medium. The neural induction medium comprises DMEM-F12 culture medium. The DMEM-F12 medium comprises fetal bovine serum (FBS) at concentration of 1%v/v, N2 at concentration of 1% v/v, B27 at concentration of 2% and bFGF at concentration of 20 ng/mL. The FBS is nutritional supplement. The N2 is a cocktail of the growth factors. The N2 comprises insulin at concentration of 5µg/ml, transferrin at concentration of 100 µg/ml, progesterone at concentration of 20 nM, putrescine at concentration of 100 µM, selenium at concentration of 30 nM, B27 at concentration of 2% v/v, and bFGF at concentration of 20ng/mL. The selenium is added for growth and maintenance of neurons in vitro. The B27 is growth supplement for neurons in vitro. The bFGF is cell differentiating agent. The bFGF is cell differentiating agent. The bFGF induces cell differentiation of the hPNF-MSCs into neuronal progenitor cells. The bFGF targets Wnt pathway. The neural induction medium is replenished after every 3-4 days. The cells are fixed with at a concentration of 4%v/v for characterization. The induction procedure is performed on a monolayer culture of the cells.

According to one embodiment herein, the isolated human perinephric fat derived stem/stromal cells (hPNF-MSCs) have positive expression for neural markers including nestin (78.12 ± 16%), NSE (75.68 ± 6.18%), β III tubulin (85.56 ± 10.91%). The neural marker expression confirms the differentiation of the hPNF-MSCs into neural cells.

According to one embodiment herein, the method for differentiating the isolated human perinephric fat derived stem/stromal cells (hPNF-MSCs) into astroglial cells, comprises the following steps. The spheres are generated with basic fibroblast growth factor (bFGF) at concentration of 1 nm/mL in DMEM-KO medium. The DMEM-KO medium is supplemented with fetal bovine serum (FBS) at concentration of 1% v/v. The generated spheres are incubated in the medium for 24 hours. The growth medium is replaced with an astroglial induction medium and L-thyroxine. The astroglial induction medium comprises FBS at concentration of 1% v/v, N2 at concentration of 1% v/v and T4 at concentration of 30 ng/mL. The FBS is a nutritional supplement. The N2 is a cocktail of the growth factors. The N2 comprises insulin at concentration of 5µg/ml, progesterone at concentration of 20nM, putrescine at concentration of 100 µM, selenium at concentration of 30nM, B27 at concentration of 2% v/v and bFGF at concentration of 20 ng/mL. The selenium is added for growth and maintenance of astroglial cells in vitro. The B27 is growth supplement for astroglial cells in vitro. The bFGF is cell differentiating agent. The bFGF induces cell differentiation of the hPNF-MSCs into astroglial progenitor cells. The T4 induces the differentiation of hPNF-MSCs into astroglial cells. The bFGF targets Wnt pathway. The astroglial induction medium is replenished after every 3-4 days. The cells are fixed with 4% PFA for characterization. The induction procedure is performed on monolayer culture of the cells.

According to one embodiment herein, the isolated human perinephric fat derived stem/stromal cells (hPNF-MSCs) show expression of GFAP (53.31 ± 14.11%) astroglial marker. The astroglial marker expression confirms the differentiation of the hPNF-MSCs into neural cells.

According to one embodiment herein, the method for differentiating the isolated human perinephric fat derived stem/stromal cells (hPNF-MSCs) into renal cells, comprises the following steps. The hPNF-MSCs are cultured on autoclaved coverslips in 35mm culture dishes comprising renal epithelial growth medium. The culture dishes are incubated at 37°C in CO₂ incubator for 17 days for identifying the differentiation of hPNF-MSC to metanephric mesenchyme. The CO₂ is at a concentration of 5%. The culture dishes are incubated at 37°C in a CO₂ incubator for 22 days for identifying the differentiation of hPNF-MSCs to podocyte and proximal convulated tubule.The CO₂ is at concentration of 5%. The culture medium is replenished after every 48 hours. The renal epithelium growth medium comprises BMP2 at concentration of 10 nm/ml, retinoic acid at concentration of 0.1µM/ml and Activin A at concentration of 10 ng/ml. The BMP2, retinoic acid, and Activin A are the growth factors which induce differentiation in the hPNF-MSC to form renal cells. The Activin A induces mesoderm specification. The retinoic acid induces the mesoderm patterning. The retinoic acid targets retinoid receptors RAR. The BMP2 induces nephrogenesis. The BMP2 is expressed in metanephric mesenchyme. The BMP2 induces epithelialization of metanephric mesenchyme. The syringe filtration is performed of the renal epithelium differentiation medium using a 0.22 micron filter. The hPNF-MSCs are incubated at a temperature of 37°C in CO₂ incubator for 17 days to identify the differentiation of hPNF-MSCs into metanephric mesenchyme cells. The concentration of CO₂ is 5%. The hPNF-MSCs are incubated at a temperature of 37°C in incubator for 22 days for a podocyte and a proximal convulated tubule differentiation, wherein the incubator has CO₂ at concentration of 5%. The renal epithelium differentiation medium is replenished after every 48 hours.

According to one embodiment herein, the Wnt pathway is targeted for differentiating the human perinephric fat derived stem/stromal cells (hPNF-MSCs) into renal cell. The Wnt pathway proteins expressed during renal development are Wnt 2b, Wnt 9. The BMP2, Activin A and Retinoic acid growth factors activate the Wnt signaling pathway enabling differentiation of hPNF-MSCs to renal progenitor cells.

According to one embodiment herein, the method for differentiating the isolated human perinephric fat derived stem/stromal cells (hPNF-MSCs) into islet like cell clusters comprises of the following steps. For differentiating hPNF-MSCs to islet like cell clusters, cells from passage 3 (P3) are seeded into three 35 mm non adherent culture dishes. One dish is control and the other two dishes are for differentiation. The control dish is uninduced dish. The control dish is supplemented with prepared medium without FBS and bFGF. The control dish comprises only Dulbecco's Modified Eagle's Medium-Knock Out (DMEM-KO) medium. The induction dish comprises Day 0 medium comprising DMEM-KO, 1.5% BSA, IX ITS. The control cell culture dish/plate (un-induced culture dish/plate) is supplemented with a cell culture medium comprising Dulbecco's Modified Eagle's Medium-Knock Out (DMEM-KO).The two cell differentiation plates/dishes are supplemented with a culture medium comprising Dulbecco's Modified Eagle's Medium-Knock Out (DMEM-KO), bovine serum albumin (BSA) in a concentration of 1.5%v/v, insulin-transferrin-selenium (ITS) in a concentration of 1X. The two cell differentiation culture dish/plate and one control cell culture dish/plate are incubated at 37°C for four days in CO2 incubator. The CO2 is at a concentration of 5%. The floating cells are collected with culture medium in a centrifuge tube after four days. The cells are allowed to settle down in the centrifuge tube for 15-20 minutes. The cells settle down to form pellet and the culture medium forms supernatant.

The supernatant is decanted, and the cell pellet is collected. The cells from the cell pellet are re-seeded in 4 day medium. The 4-day medium comprises a Dulbecco's Modified Eagle's Medium (DMEM-KO), bovine serum albumin (BSA) in a concentration of 1.5%, insulin-transferrin-selenium (ITS) in a concentration of IX and Taurine in a concentration of 0.3 mM. The cells are incubated at 37°C in CO2 incubator for further three days. The cells are incubated for a total of seven days. The CO2 is at a concentration of 5%. The cells are fed with a fresh 4 day medium after seven days. The cells are incubated at 37°C in a CO2 incubator for further three days. The cells are incubated for a total of ten days. The CO2 is at a concentration of 5%. The floating cell clusters are collected with a culture medium in a sterile centrifuge tube after 10 days. The cells are allowed to settle for 15-20 minutes at room temperature. The cells settle down to form the pellet and the culture medium forms the supernatant. The supernatant is decanted. The cell pellet comprises cell clusters. The cell clusters are re-seeded in a medium comprising DMEM-KO, BSA at a concentration of 1.5% v/v, ITS at a concentration of IX, 100nM nicotine amide at a concentration of 100 nM, taurine at a concentration of 3nM and a glucagon like peptide (GLP) at a concentration of 100 nM. The cells are subjected for dithiozone (DTZ) staining analysis and immunofluorescence analysis. The cell clusters are islet cell clusters.

According to one embodiment herein, the islet cell clusters are characterized by dithiozone (DTZ) staining. The islet cell clusters are positive for DTZ staining. The DTZ is a zinc chelating agent that selectively stains pancreatic beta cells.

According to one embodiment herein, the islet cell clusters are characterized by immunofluorescence staining for c-peptide. The cell clusters show a high expression of c-peptide, and the expression of c-peptide confirms the generation of functional islet cell clusters.

According to one embodiment herein, the following steps are involved in the isolation of the human perinephric fat derived stromal cells. The perinephric fat tissue samples are processed in a sterile environment comprising cGMP compliant clean room with class 100 biosafety cabinet. The mesenchymal stem cells or stromal cells (MSCs) are isolated from the sample adipose tissue using both mechanical and enzymatic methods. The sample is washed for several times in a IX Dulbecco's Phosphate Buffered Saline (DPBS) containing 2X concentration of antibiotics (Pen-Strep). After washing thoroughly, the sample tissues are mechanically minced into several small pieces with a sterile scalpel blade. After mincing the tissues, the tissue sample is treated enzymatically with 0.2% Collagenase type I in a Dulbecco's Modified Eagle's Medium (DMEM-KO) and Pen-Strep for 60 min at 37°C placed on rocker. After enzymatic treatment, the DMEM-KO medium supplemented with a 5% Fetal Bovine Serum (FBS) is added to neutralize the action of Collagenase and mixed thoroughly. The homogenate is filtered through 40µm cell strainer and the filtrate is collected for further processing. The cell pellet (SVF) is obtained by centrifugation of collected filtrate at 1500 rpm for 20 min and re-suspended in the culture medium consisting of the DMEM-KO supplemented with 10% FBS, IX L-Glutamine, Pen-Strep and 1 ng/ml bFGF. The cell suspension is then seeded in one cell stacks containing 200 ml culture medium. The cell stacks are gently rocked for even distribution of cells and maintained at 37°C in a humidified incubator with 5% CO₂. After 48 hrs, the medium is replenished to eliminate non-adherent cells and the adherent, fibroblast-like populations of cells are allowed to expand in culture medium until 80% confluency for harvesting. The culture medium is replenished two to three times every week. Once the cells attain a confluency of 80%, the cell stacks are harvested by trypsinization method, counted and cryopreserved in a cryovial. The cryovial is labeled as Passage 0 (P0), with sample ID number, cell number and the date of isolation.

According to one embodiment herein, after isolation of the stem cells, the expansion of the stem cells comprises the following steps; the culture medium is replenished based on floaters, change in the pH of media, background and if essential after every 48hrs. The representative microphotographs of cultures are clicked during each observation by inverted microscope. Once the cells become 90% confluent, the cells are harvested and sub-cultured. The spent medium is aspirated out and collected for later neutralization process. The cells are rinsed twice with appropriate volume of DPBS depending on the plate size for 2-3 minutes. Appropriate volume of 0.25 % warm Trypsin-EDTA is added, thereby ensuring that the Trypsin - EDTA covers the entire surface on which cells are adherent and incubated at 37°C for 2-3 minutes till the cells are detached from the plates and start floating, and which is confirmed under microscope. The cells are exposed to Trypsin - EDTA for longer time, if the cell detachment is low. The collected spent medium is added to neutralize the action of Trypsin - EDTA. Further, a forceful pipetting is done at the lower surface of the plates to dissociate the remaining cells. The cell suspension is transferred into the centrifuge tube and centrifuged at 1400 rpm for 10 minutes at room temperature. The supernatant is discarded carefully without disturbing the cell pellet and the cell pellet is re-suspended in 1 ml culture medium. The cell pellet is subjected to cell counting. The cell count is determined using hemocytometer and cells are re-seeded at a density of 1000 cells per square cm in the culture plates for further culturing. The culture plates are labeled appropriately, with sample ID number, the passage number and the date. The residual cells left behind after seeding are transferred into a cryovial. The cryovial is cryopreserved in liquid nitrogen as a backup after every two to three passages.

According to one embodiment herein, for cryopreservation the freezing mixture [90% Fetal Bovine Serum (FBS) + 10% dimethyl sulfoxide (DMSO)] is prepared as per requirement and allowed to obtain an ice-cold mixture at 4°C. The cell suspension to be frozen is centrifuged once again at 1400 rpm for 10 minutes at room temperature to obtain a cell pellet. The supernatant is discarded and the ice-cold freezing mixture is added drop wise to the cell pellet, mixed gently and transferred into a cryovial. The cryovial is frozen at -80°C overnight. After post-incubation, the cryovial is transferred into a liquid nitrogen cryotank.

According to one embodiment herein, the characterization of cells derived and cultured from human perinephric fat and confirmation of these cells as mesenchymal stem/stromal cells comprises the optimization of seeding density, morphological evaluation of hPNF-MSC, growth kinetics study, immunophenotypic evaluation, differentiation potential analysis, karyotyping and secretome profiling (cytokines, chemokines, and growth factors).

To determine the optimum seeding density for sub-culturing and expansion of MSCs, two different seeding densities are taken into consideration for one representative donor sample (PNF2). At each passage, the cells are seeded at 1000 cells/cm² [Low Density (LD)] and 2500 cells/cm² [High Density (HD)]. The expansion of mesenchymal stem cells after initial seeding is rapid in HD cultures, the cells failed to maintain similar growth kinetics and morphology as LD cultures over late passages. The HD culture exhibit cell senescence at passage 10 (P10), whereas LD cultures exhibits senescence at passage 15 (P15).

According to one embodiment herein, the morphological evaluation of the human perinephric fat-mesenchymal stem cells (hPNF-MSCs) reveals the fibroblast like morphology from all donor samples. All the three samples maintain the spindle shaped fibroblast like morphology till passage 7 (P7). The cell population doubling time is determined from passage 1 (P1) to passage 14 (P14). The population doubling time (PDT) of the cells from PNF2 tissue sample significantly increased from 14.38±1.29 hours at P2 to 235.85 ±0.65 hours at P9 (p< 0.05) in case of high density cultures, whereas in case of low density cultures, PDT is observed to be 75.065±0.36 hours at P14.A similar trend is observed in the tissue samples PNF 4 and PNF 7.

According to one embodiment herein, for the immune-phenotyping, a set of cell surface antigen markers are used for characterization of human perinephric fat-mesenchymal stem cells (hPNF-MSCs) at different passages to examine effect of prolonged sub-culturing on a population of MSCs derived from hPNF. The expression of surface antigens for cells from early and late passages detected using flow cytometry. The level of the surface marker expression is analyzed. The isolated population of MSCs from PNF tissue, namely P0 demonstrated negative expression for HLA-DR, CD 34 and CD45. The cells showed higher expression of CD44 and CD90. At passage P5, there is negative expression of HLA-DR, CD 34 and CD 45 in less than 4% of cells, whereas highly positive expression of CD 44, CD73, CD90, CD105 and CD 166 is observed in more than 90% of cells. The cells from late passages demonstrate similar range of surface antigen expression. The cells at P9 and P15 show lower expression of antigens when compared to other passages. There is no significant change in terms of antigens markers of PNF-MSC after successive subculture for long-term.

According to one embodiment herein, the international society for cellular therapy (ISCT) has published minimal criteria for defining or classifying multipotent mesenchymal stromal cells. The ISCT has defined what are the antigens present in the mesenchymal stem cells. If a cell is to be called or classified as mesenchymal stromal cell, then the cell should have CD73, CD 90 and CD 166 antigens in it. If the antigens (CD 73, CD 90 and CD 166) are present in the cells then the cells are called positive for these antigens. The cells must be negative for CD 34 and CD 48. These antigens are expressed by haematopoetic stem cells, which are not mesenchymal stem cells in nature. To classify a population of stem cells as mesenchymal stromal cells, more than 95% of the cells should be positive for CD 73, CD 90 and CD 166 antigens. Further the cells must be negative for CD 34 and CD 48 antigens; or the cells must not express CD 34 and CD48 more than 2%.

According to one embodiment herein, the multipotent differentiation potential of hPNF-MSCs is evaluated at passage 2 and passage 5. The cells are induced into osteocytes and adipocytes differentiation for all the three donor samples. The hPNF-MSCs from all three donors successfully differentiated into both osteocytes and adipocytes. The cells which are differentiated into osteocytes evidently showed production of extracellular matrix deposition, confirmed by Von Kossa staining. The lipid droplets in adipogenic differentiation are confirmed by Oil red O staining.

According to one embodiment herein, the karyotypic analysis is performed for all the three donors. The cells demonstrate normal karyotype from passage 2. A representative data is obtained which illustrates the normal karyotype with 46 chromosomes (XX, Female).

According to one embodiment herein, in general "secretome" refers to the number of factors secreted by the stem cells and it is now widely accepted that most of the regenerative actions of stem cells are attributed to these factors quantitatively ensure the regenerative potential of the stem cells. An analysis of the secretome of human perinephric fat derived mesenchymal stromal cells. For validation comparison of the data is done with secretome data of mesenchymal stem cells derived from subcutaneous fat (LA) which has been characterized. For the comparison the factors are divided into pro-inflammatory cytokines, anti-inflammatory cytokines, chemokines, growth factors and other bioactive factors. These factors individually or synergistically decide the regeneration potential of stem cells type.

According to one embodiment herein, further the range of cytokines, chemokines and growth factors, are evaluated using human cytokine panel kit (Milliplex®). Since mesenchymal stem cells (MSCs) derived from lipoaspirate (LA) are extensively studied and well characterized; the cytokine data set is obtained from LA-MSCs as the reference to examine differences in the cytokine secretion profiles obtained from MSCs populations of PNF. The comparison between the PNF-MSCs and LA-MSCs exhibited differences in levels of cytokines released. There are four bioactive factors (IL-4, IL-10, IL-Ra and IFN-γ) secreted at higher concentrations and four bioactive factors (IL-13, MCp-1, GRO and GM-CSF) secreted at significantly lower concentrations by hPNF-MSCs when compared to hLA-MSCs. Other bioactive factors do not exhibit any significant difference between hPNF-MSCs and hLA-MSCs. Other bioactive factors do not show any significant differences between hPNF-MSCs and hLA-MScs. To test whether cytokine profile of both samples follow any trend, the cytokines are categorized into five groups (pro-inflammatory, anti-inflammatory, chemokines, growth factors and others).

According to one embodiment herein, the secretion of the pro-inflammatory cytokines exhibits significant secretions of IFN-γ by hPNF-MSCs when compared to hLA-MSC. Adipose MSCs from both sources - visceral and sub-cutaneous- secrete pro-inflammatory cytokines, including IL-6, IL-9, IL-12, IFN-α2, and IL-1α between PNF and LA-MSCs. Both PNF and LA-MSC show very high level of IL-6 cytokine secretion. The IL-8 cytokine secretion is found to be higher in LA-MSCs when compared to PNF-MSCs.

According to one embodiment herein, the anti-inflammatory cytokines are analyzed including IL-3, IL-4 and IL-10. The IL-4, IL-10 and IL-1Ra show significant increase in hPNF-MSCs when compared to hLA-MSCs. The IL-13 secretion is found to be significantly higher in hLA-MSCs. The chemokines analyzed include MDC, Fractalkine, IP-10, MIP-1β, eotaxin, PCP-1, MCP-3 and GRO.

According to one embodiment herein, the growth factor levels of G-CSF, GM-CSF, VEGF and PDGF-aa are also analyzed. These growth factors are secreted considerably by both the sample types. The level of GM-CSF is reported to be significantly higher in hLA-MSCs when compared to hPNF-MSCs. The secretion of pro-angiogenic factor, VEGF is found to be higher, approximately 2000-4000 pg/mL. The EGF level are found to be negligent (0.6±1.25 pg/mL in PNF-MSCs and 0.00 pg/mL in LA-MSCs) in both sample types.

Other bioactive factors analyzed such as CD40L and FLT-3L are secreted at a similar concentration by both sample types.

According to one embodiment herein, the differentiation of the human perinephric fat derived/isolated mesenchymal stem cells or stromal cells into neural cells includes the generation of the spheres. For the neural cell differentiation, the spheres are generated using 1 ng/ml bFGF in DMEM-KO media supplemented with 1% Fetal bovine serum (FBS).The spheroid like bodies are allowed to be in same media for 24 hours. Next day the growth medium is replaced by neural induction medium including DMEM-F12 supplemented with 1% FBS, 1% N2, 2% B27 and 20 ng/mL bFGF (Life Technologies). The 1% FBS is a nutritional supplement, 1% N2 is a cocktail of the growth factor comprising 5µg/ml insulin or 100µg/ml transferrin, 20nM progesterone, 100 µM putrescine, 30nM selenium responsible for growth and maintenance of neurons in vitro, 2% B27 is a supplement for growth of neurons in vitro and 20 ng/mL bFGF are required for the differentiation of the MSC's into neuronal progenitor cells by targeting Wnt pathway. The medium is replenished after every 3-4 days. On the 15^{th} day, cells are fixed with 4% PFA for further characterization. A simultaneous induction procedure is performed on monolayer culture.

According to one embodiment herein, the differentiation of the human perinephric fat derived/isolated mesenchymal stem cells or stromal cells into astroglial cells includes the generation of the spheres. For the astroglial cell differentiation, the spheres are generated using a culture medium comprising 1ng/mL bFGF in DMEM-KO media supplemented with 1% FBS. The spheroids-like bodies are allowed to be in same media for 24 hours. Next day, the growth medium is replaced by astroglial induction medium including L-thyroxine (T4, Sigma) supplemented with 1% FBS, 1% N2, 30 ng/mL T4. The 1% FBS is a nutritional supplement, 1% N2 is a cocktail of growth factor comprises 5µg/ml -insulin, 20nM progesterone, 100µM putrescine, 30nM selenium is responsible for growth and maintenance of astroglial cells in vitro, 2% B27 is a supplement for the growth of astroglial cells in vitro, and 20 mg/ml bFGF is required for the differentiation of mesenchymal stem cells (MSCs) into neuronal progenitor cells using Wnt pathway. The 30ng/ml of T4 is required for the differentiation of MSCs into astroglial cells. The medium is replenished after every 3-4 days. On the 15^{th} day, cells are fixed with 4% PFA for further characterization. A simultaneous induction procedure is performed on monolayer culture.

According to one embodiment herein, for the determination of the confirmatory results, flow analysis of neural and astroglial markers for hPNF-MSCs from early passage. The hLA-MSCs are considered as a control. A similar trend in the expression of both neural and astroglial markers is observed as explicated by immunofluorescence. The flow analysis is performed on two donor samples and average expression percentage is taken into consideration. The cells from both the donor sample show highly positive expression for neural markers including nestin (78.12 ± 16%), NSE (75.68 ± 6.18%), β III tubulin (85.56 ± 10.91%). The flow analysis for astroglial markers show moderate expression of GFAP (53.31 ± 14.11%) and very low expression of A2B5 (19.07 ± 5.35%) similar to IF results. The expression level of GFAP is found to be contradictory to IF results. The adipose tissue derived-mesenchymal stem cells (LA-MSCs) are used as a control for comparative analysis. The results obtained from perinephric-mesenchymal stem cells (PNF-MSCs) are found to be concomitant to LA-MSCs, demonstrating its analogy to MSCs.

According to one embodiment herein, Small drops (20-30 µl) of MSCs cell suspension containing 5000-20000 cells/drop were placed on inner side of the 60 mm non-adherent Tarsons-petridish in hanging drop fashion. Briefly, 10-15 drops are created per plate and the bottom of plates is overlaid with DPBS to avoid loss of nutrients through desiccation. The plates are incubated for 48 hrs in hanging drop mode at 37°C with 5% CO₂. After 48 hrs incubation period, the hMSC spheroid aggregates are transferred to a 60 mm non-adherent dish with fresh culture media for further experiments. The spheroids are cultured for 18 days in culture medium, replenished after every 3days. A new approach is adopted to observe, whether PNF-MSCs can generate spheroids to represent 3D-model. The cells from all the groups are successfully generated spheroids by hanging drop method. The cells from early passage are used for the generation of the spheroids. The cells emerging out of the spheroids show fairly representative morphology of MSCs. A remarkable difference in morphology is observed when these spheroids are cultured in non-adherent dishes. A large number of cells budded out from the spheroids forming colonies of cells at distal ends. Further a tube like meshwork is observed projecting from the spheroids.

According to one embodiment herein, the pathway targeted for the renal lineage differentiation of the human perinephric mesenchymal stem cells is Wnt pathway. The Wnt pathway plays a critical role in the development of the kidney. The Wnt family members expressed during kidney development are Wnt 2b, Wnt 9 etc. The growth factors like BMP2, Activin A and Retinoic acid activate the Wnt signaling pathway enabling differentiation of hPNF-MSCs to renal progenitor cells.

According to one embodiment herein, the differentiation of the human perinephric fat derived/isolated mesenchymal stem cells or stromal cells into renal lineage includes the renal epithelial growth medium. The growth medium comprises the growth factors which induce the differentiation are BMP2, retinoic acid, and Activin A. The Activin A plays a major role in the mesoderm specification, the retinoic acid acts via the retinoid receptors RAR and aids in the mesoderm patterning, and the BMP2 is essential for nephrogenesis. The BMP2 is expressed in metanephric mesenchyme and cause epithelialization of metanephric mesenchyme. As the kidney responds to BMP2 stimulation, the response to injury and repair frequently recapitulates development having the same function of epithelization of metanephric mesenchyme.

Two different methods are used to carry out the differentiation of hPNF-MSC-Adherent monolayer and through the formation of spheroids. The Wnt pathway plays a critical role in the development of kidney. The Wnt family members expressed during kidney development are Wnt 2b; Wnt 9 etc. the growth factors like BMP2, Activin A and Retinoic acid activate the Wnt signaling pathway enabling differentiation of hPNF-MSCs to renal progenitor cells.

The differentiation of human perinephric-mesenchymal stem cells (hPNF-MSC's) into renal cells by adherent monolayer culture method comprises the following steps of culturing the hPNF-MSC on autoclaved coverslips in 35mm dishes. This includes one un-induced and two un-induced culture dishes. The un-induced dish contains renal epithelial growth medium (REGM) with REGM supplement mix and the induction dishes are supplemented with REGM medium, REGM supplement mix, BMP2 (10 nm/ml), Retinoic acid (0.1µM/ml) and Activin A (10ng/ml). This differentiation medium is then syringe filtered using a 0.22 micron filter. The cells are incubated at 37°C in 5% CO₂ incubator for 17 days to identify differentiation to metanephric mesenchyme and 22 days for podocyte and proximal convulated tubule. The medium is replenished after every 48 hours.

According to one embodiment herein, the differentiation of human perinephric-mesenchymal stem cells (hPNF-MSC's) into renal cells spheroid culture method comprises the following steps of plating 10,000 cells (approx) as drops on the lid of 60 mm TarsonsPetridish (30 drops per dish). The bottom of the petridish is overlaid with DPBS to avoid desiccation. The petridish is kept at 37°C in 5% CO₂ for 24 hours. The MSC spheroids thus formed are transferred onto autoclaved cover slips in 35 mm adherent dishes supplemented with renal differentiation medium. The spheroids are cultured for 17 days to identify metanephric mesenchyme and 22 days for podocyte and proximal convoluted tubule.

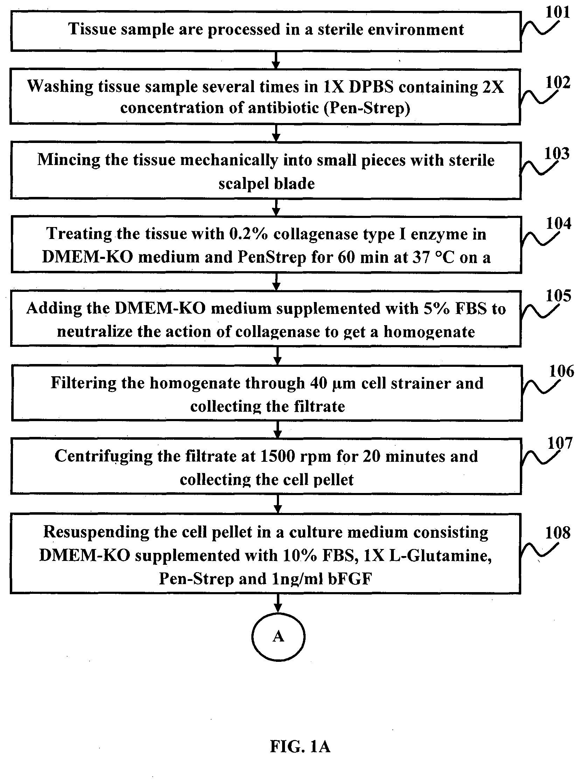

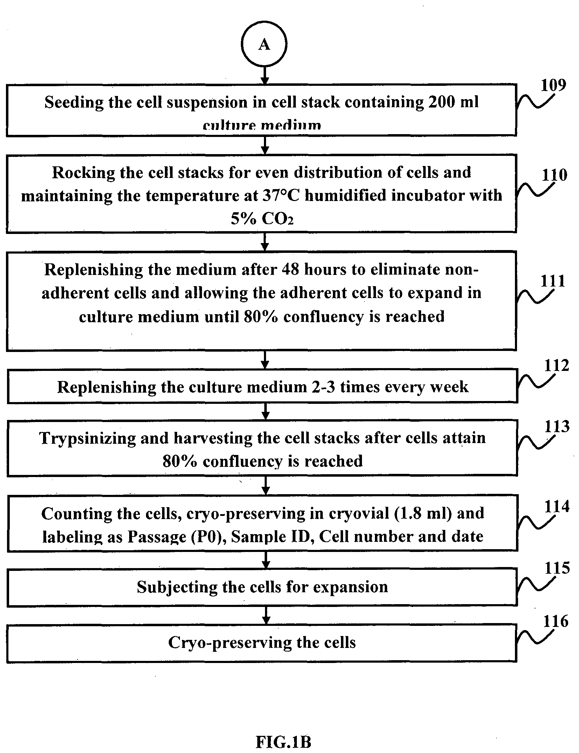

**FIG.1** illustrates a flow chart explaining a method for isolating the human perinephric fat-mesenchymal stem cells (hPNF-MSCs), according an embodiment herein.

According to one embodiment herein, the following steps are involved in the isolation of the human perinephric fat derived stromal cells; the perinephric fat tissue samples are processed in a sterile environment (101). The sterile environment comprises cGMP compliant clean room with class 100 biosafety cabinet. The mesenchymal stem cells or stromal cells (MSCs) are isolated from the sample adipose tissue using both mechanical and enzymatic method. The next step involves washing the tissue sample several times in a 1X Dulbecco's Phosphate Buffered Saline (DPBS) containing 2X concentration of antibiotics (Pen-Strep) (102). After washing thoroughly, the next step is mincing the tissue sample mechanically into several small pieces with a sterile scalpel blade (103). Further treating the tissue sample enzymatically with 0.2% Collagenase type I (Life Technologies) in a Dulbecco's Modified Eagle's Medium (DMEM-KO) medium and Pen-Strep for 60 min at 37°C placed on rocker (104). Adding the DMEM-KO medium supplemented with a 5% FBS to neutralize the action of Collagenase and to obtain a homogenate (105). Filtering the homogenate through 40µm cell strainer and collecting the filtrate (106). Centrifuging the collected filtrate at 1500 rpm for 20 min and collecting the cell pellet (107). Re-suspended in the culture medium consisting of the DMEM-KO supplemented with 10% FBS, IX L-Glutamax, Pen-Strep and 1 ng/ml bFGF (108). Seeding the cell suspension in cell stacks (Corning Incorporated Life Sciences, Tewksbury, MA) containing 200 ml culture medium (109). Rocking cell stacks gently for even distribution of cells and maintained at 37°C humidified incubator with 5% CO₂(110). Replenishing the medium after 48 hrs, to eliminate non-adherent cells and the adherent, fibroblast-like populations of cells are allowed to expand in culture medium until 80% confluency for harvesting is reached (111). Replenishing the culture medium 2-3 times every week (112). Trypsinizing and harvesting the cell stacks once the cells attain a confluency of 80% (113). Counting the cells and cryopreserving in a cryovial (1.8 ml, Nunc, Denmark), labeling cryovial with sample ID, cell number, date (114). The cryovial is labeled as Passage 0 (P0), with sample ID number, cell number and the date of isolation. Further the cells are subjected for expansion (115). The cells are cryopreserved for future use (116).

According to one embodiment herein, for expansion of the cells the culture medium is replenished based on floaters, change in the pH of the media, background and if essential after every 48hrs. The representative microphotographs of cultures are clicked during each observation by inverted microscope. Once the cells become 90% confluent, the cells are harvested and sub-cultured. The spent medium is aspirated out and collected for later neutralization process. The cells are rinsed twice with appropriate volume of Dulbecco's Phosphate Bufffered Saline (DPBS) depending on the plate size for 2-3 minutes. An appropriate volume of 0.25 % warm trypsin-EDTA is added, ensuring trypsin covers entire surface on which cells are adherent and incubated at 37°C for 2-3 minutes till the cells detached from the plates and start floating which is confirmed under microscope. The cells are exposed to trypsin for a longer time if cell detachment is low. The collected spent medium is added to neutralize the action of the trypsin. The pipetting is done forcefully downwards at the bottom surface of the culture plate to dissociate the remaining cells. The cell suspension is transferred into a centrifuge tube and centrifuged at 1400 rpm for 10 minutes at room temperature. The supernatant is discarded carefully without disturbing the cell pellet. The pellet is re-suspended in 1 ml culture medium. The cell count is determined using haemocytometer and the cells are re-seeded at a density of 1000 cells per square cm in the culture plates for further culture. The culture plates are labeled appropriately, with sample ID, passage number and date. The residual cells left behind after seeding are transferred into cryovial and cryopreserved in the liquid nitrogen as backup after every two to three passages.

According to one embodiment herein, for the cryopreservation, a freezing mixture of [90% fetal bovine serum (FBS) + 10% Dimethyl Sulfoxide (DMSO)] is prepared as per requirement and allowed to be ice-cold at 4°C. The cell suspension to be frozen is centrifuged once again at 1400 rpm for 10 minutes at room temperature to obtain a cell pellet. The supernatant is discarded and ice-cold freezing mixture is added drop wise to the cell pellet, mixed gently and transferred into a cryovial. The cryovial is incubated at a temperature of -80°C for overnight in Mr. Frosty. Post-incubation, the cryovial is transferred into liquid nitrogen cryotank.

### EXAMPLE-1 Characterization of Cells derived and Cultured from Human Perinephric fat and Confirmation of these Cells as Mesenchymal Stem/ Stromal Cells

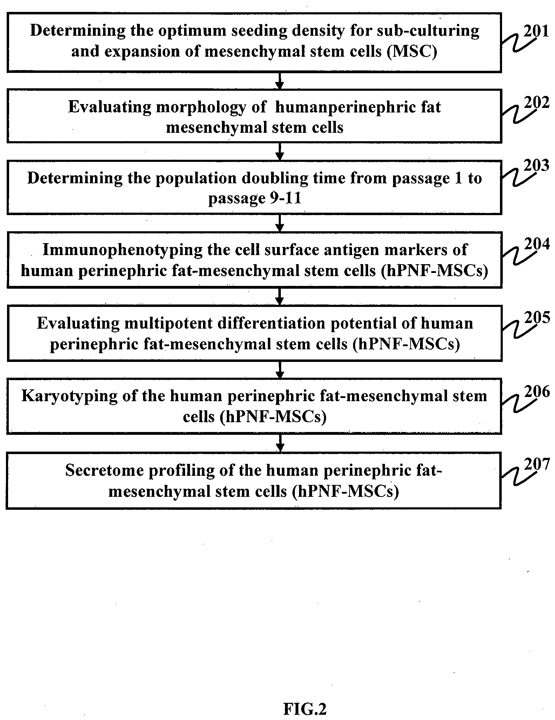

According to one embodiment herein, the characterization of cells derived and cultured from human perinephric fat and confirmation of these cells as mesenchymal stem/stromal cells comprises optimization of seeding density, morphological evaluation of hPNF-MSC, growth kinetics study, immunophenotypic evaluation, differentiation potential analysis, karyotyping and secretome profiling (cytokines, chemokines, and growth factors). **FIG.2** illustrates a flow chart explaining a method for characterization and confirmation of cells derived and cultured from human perinephric fat mesenchymal stem cells (hPNF-MSC), according to an embodiment herein. For the characterization of the cells the first step is determining the optimum seeding density for sub-culturing and expansion of mesenchymal stem cells MSCs (201). Next step is evaluating the morphology of human perinephric fat mesenchymal stem cells (202). Next step is determining the population doubling time from passage 1 to passage 9-11 (203). The next characterization method is immune-phenotyping the cell surface antigen markers of human perinephric fat-mesenchymal stem cells (hPNF-MSCs) (204). Further step involves evaluating the multipotent differentiation potential of human perinephric fat-mesenchymal stem cells (hPNF-MSCs) (205). Next step is karyotyping of the human perinephric fat-mesenchymal stem cells (hPNF-MSCs) (206). The last step in the characterization is secretome profiling of the human perinephric fat-mesenchymal stem cells (hPNF-MSCs) (207).

### EXAMPLE-1.1-Optimization of Seeding Density

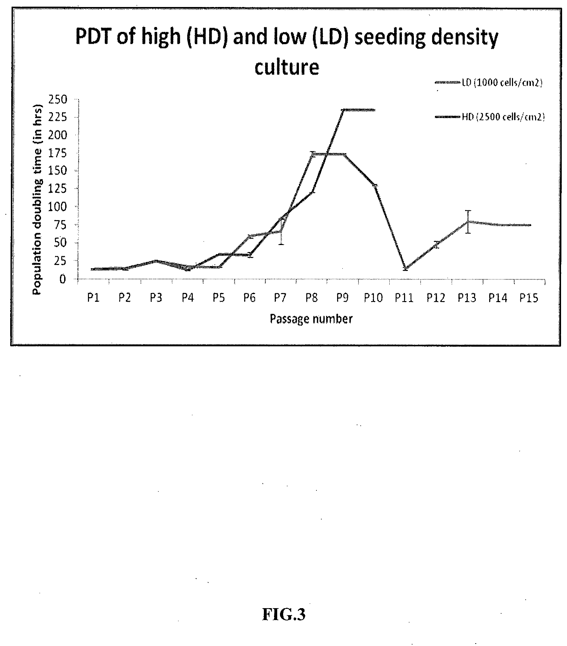

To determine the optimum seeding density for sub-culturing and expansion of MSCs, two different seeding densities were taken into consideration for one representative donor sample (PNF 2). At each passage cells were seeded at 1000 cells/cm²low density (LD) and 2500 cells/cm²high density (HD). Although expansion of MSCs after initial seeding was rapid in HD cultures, cells failed to maintain similar growth kinetics and morphology as LD cultures over late passages. Moreover, HD cultures showed cell senescence at P10, whereas LD cultures showed senescent at P15. Taken together, cells seeded at 1000 cells/cm² showed considerably better growth kinetics than compared to high density cultures. Following optimization data, 1000 cells/cm² seeding density was adopted for subsequent in vitro expansion of MSCs from donor samples (PNF 7 and PNF 4). **FIG.3** is a graph illustrating the population doubling time of the high and low seeding density culture, according to an embodiment of the present invention.**FIG.3** also illustrates that the cells seeded at 1000 cells/cm² show higher growth kinetics than compared to high density cultures. The optimization data is followed and 1000 cells/cm² seeding density is adopted for in vitro expansion of human perinephric fat-mesenchymal stem cells (hPNF-MSCs) from the donor samples (PNF4 and PNF7).

### EXAMPLE-1.2-Morphological Evaluation of the human perinephric fat-mesenchymal stem cells (hPNF-MSCs)

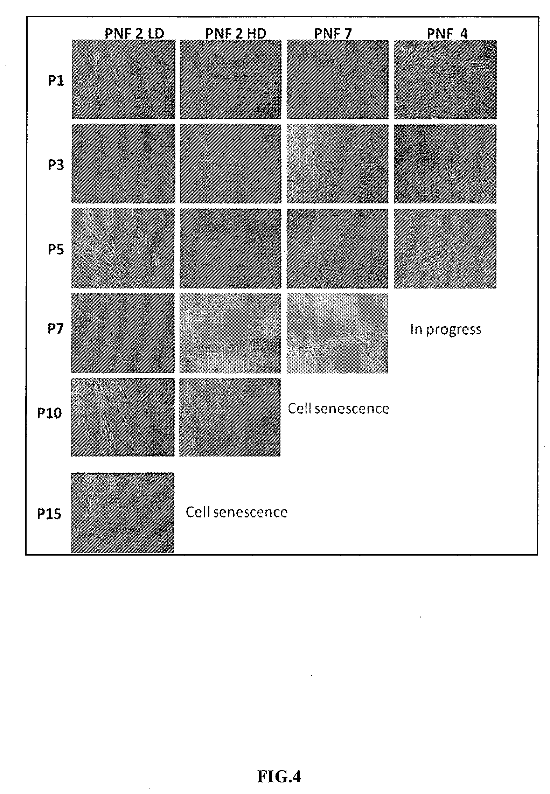

**FIG.4** is a photograph illustrating the morphology of the human perinephric fat mesenchymal stem cells (hPNF-MSCs), according to an embodiment herein. The human perinephric fat-mesenchymal stem cells (hPNF-MSCs) show a typical fibroblast like morphology from all donor samples (**FIG.4**). All the three samples maintained the typical-spindle shaped fibroblast-like morphology till P7. However, there was progressive change in morphology beyond passage 5. PNF-MSCs from early passages (P1-P5) showed rapidly growing (21.13 ± 0.54 hrs at P5) thin spindle-shaped, slender and fibroblast-like morphology, whereas PNF-MSCs from late passages (beyond P5) showed morphologically polygonal shape and consisted slower growth rate (66.31 ± 11.02 hrs at P7). At late passages, the cells appeared large, flattened, granulated with long processes. Cells from late passages showed vacuole formation within them. At passage 10, cells showed significantly minimal growth rate except one donor sample. Cells from donor sample PNF 2 showed progressive proliferation rate till passage 10.

### EXAMPLE-1.3-Determination of the Population Doubling Time of human perinephric fat-mesenchymal stem cells (hPNF-MSCs)

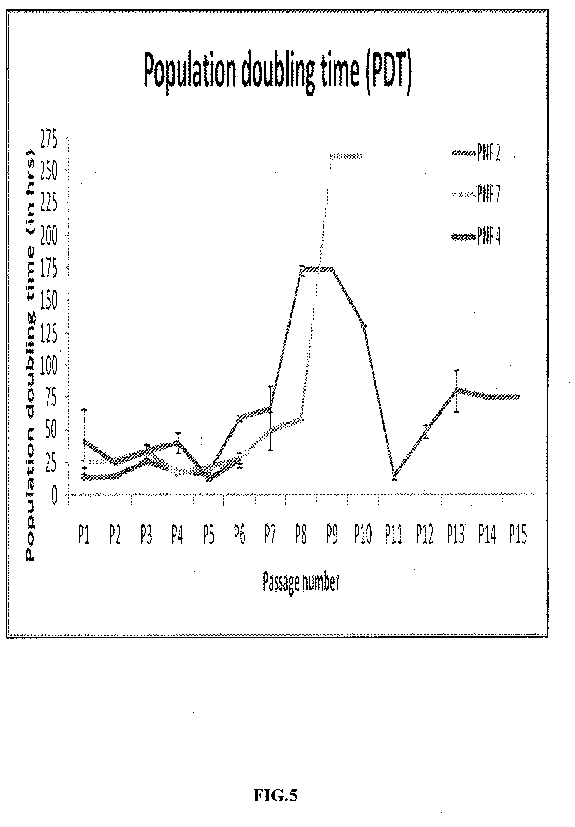

The population doubling time was determined from passage 1 to passage 9-14. As demonstrated in the Figure 1, the population doubling time (PDT) of cells from PNF 2 sample significantly increased from 14.38 ± 1.29 hours at P2 to 235.85 ± 0.65 hours at P9 (p<0.05) in case of high density cultures, whereas in case of low density cultures, PDT was observed to be 75.065 ± 0.36 hours at P14. **FIG.5** is a graph illustrating the growth kinetics of the human perinephric fat mesenchymal stem cells (hPNF-MSCs), according to an embodiment herein. **FIG.5** also illustrates population doubling time (PDT) for donor sample PNF 4 and PNF 7. The PDT for PNF 7 sample significantly increased from 26.69±0.19 hours at passage P2 to 260.95±0.85 hours at passage P9. The PDT of PNF 4 donor sample is observed to be 24.92±0.92 hours, which is significantly similar to PDT of passage 6 P6 (26.66±5.3).

### EXAMPLE-1.4-Immunophenotypic Evaluation

The mesenchymal stem cells are isolated from perinphric fat. The isolated cells are labeled as passage 0 (P0). The isolated mesenchymal stem cells are propagated toll passage 15 (P15). In each stage the cells are characterized immunophenotypically. Further the cells are analyzed if they maintain their immunophenotype till the last passage.

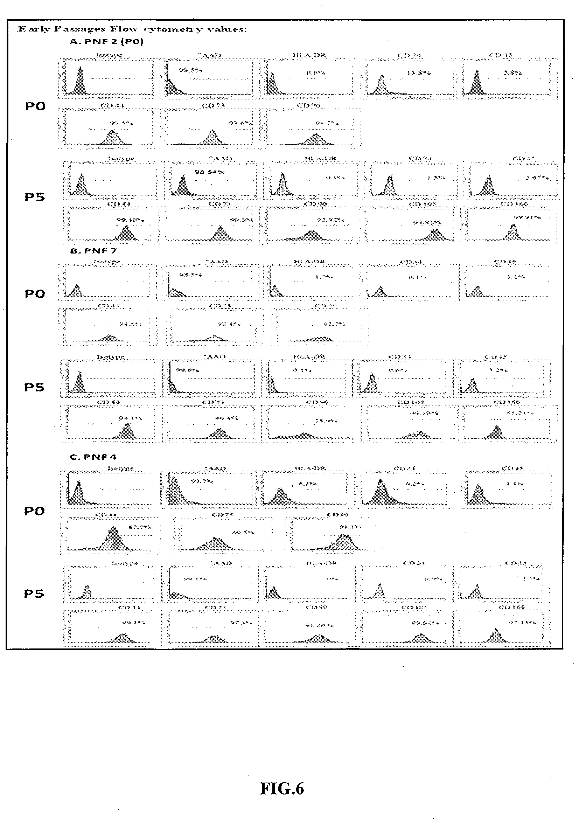

For immunophenotyping, a set of cell surface antigen markers were used for characterization of hPNF-MSCs at different passages to examine effect of prolonged sub-culturing on a population of MSCs derived from hPNF. The mesenchymal stem cells maintain their immunophenotype till last passage, indicating that the hPNF-MSCs, retain the expression of surface markers/surface antigens even after long term serial passaging.**FIG.6** illustrates expression of surface antigens for cells from early and late passages detected using flow cytometry. **FIG.6** illustrates a flow cytometry histogram of surface marker expression by human perinephric fat mesenchymal stem cells (hPNF-MSCs) from early passage (P0 and P5), according to an embodiment herein. The flow cytometry analysis is performed for detection of surface markers (HLA-DR, CD34, CD45, CD44, CD73, CD90, CD105 and CD166). The histograms show the level of marker expression with percentage expression. The 7AAD represents percentage of viable cells and isotype represents negative control cells.**FIG.6** illustrates that the hPNF-MSC expresses all the criteria proposed by ISCT guidelines and hence the perinephric fat derived mesenchymal stromal cells (hPNF-MSCs).

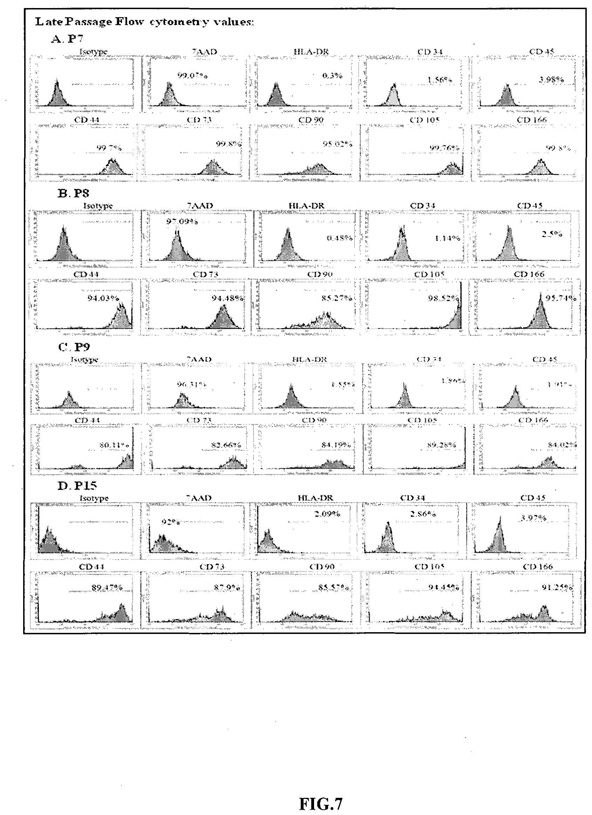

**FIG.7** illustrates a flow cytometry histogram of surface marker expression by human perinephric fat mesenchymal stem cells (hPNF-MSCs) from late passage, according an embodiment herein. The level of marker expression with percentage expression. The 7AAD represents percentage of viable cells and isotype represents negative control cells.

The isolated population of mesenchymal stem cells (MSCs) from perinephric fat (PNF) tissue, namely P0 demonstrated negative expression for HLA-DR, CD 34, and CD 45 (the mean expression detected were 2.83 ± 1.40%, 9.70 ± 1.83%, and 3.47 ± 0.39% respectively). The cells showed higher expression of CD 44 (93.90 ± 2.79%, CD 73 (82.17 ± 8.85%), and CD 90 (94.17 ± 1.89%). At passage P5, we found negative expression for HLA-DR, CD 34, and CD 45 in less than 4% of cells, whereas highly positive expression for CD 44(99.20 ± 0.10%), CD 73 (98.83 ± 0.78%), CD 90 (89.24 ± 6.89%), CD 105 (99.61 ± 0.13%) and CD 166 (94.09 ± 4.51%) was reported in more than 90% 0f cells. The cells from late passages demonstrated similar range of surface antigen expression (above 90%). A representative data for antigen expression of cells from late passages has been illustrated in the Table 1 below. Although cells at P9 and P15 showed to some extend lower expression of antigen expression when compared to other passages, there was no remarkable change in expression level detected. Therefore, there was no significant change in terms of antigen markers of PNF-MSCs after successive subculture for long-term.

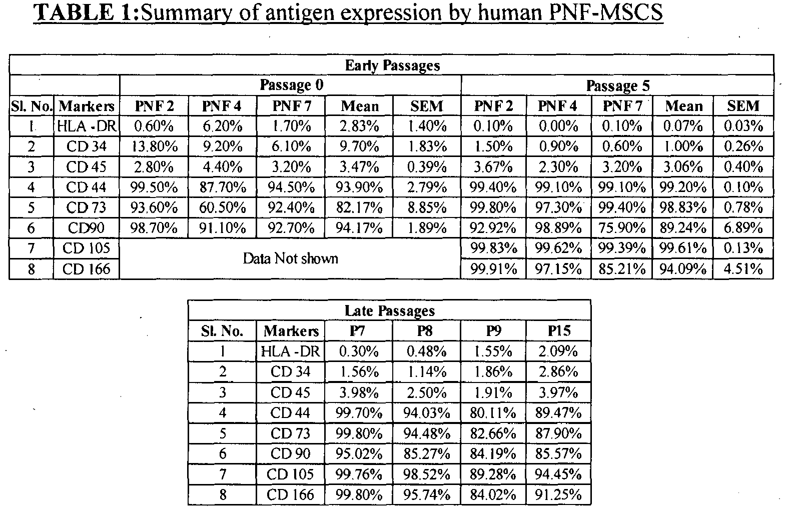

### EXAMPLE-1.5- Evaluation of Differentiation Potential

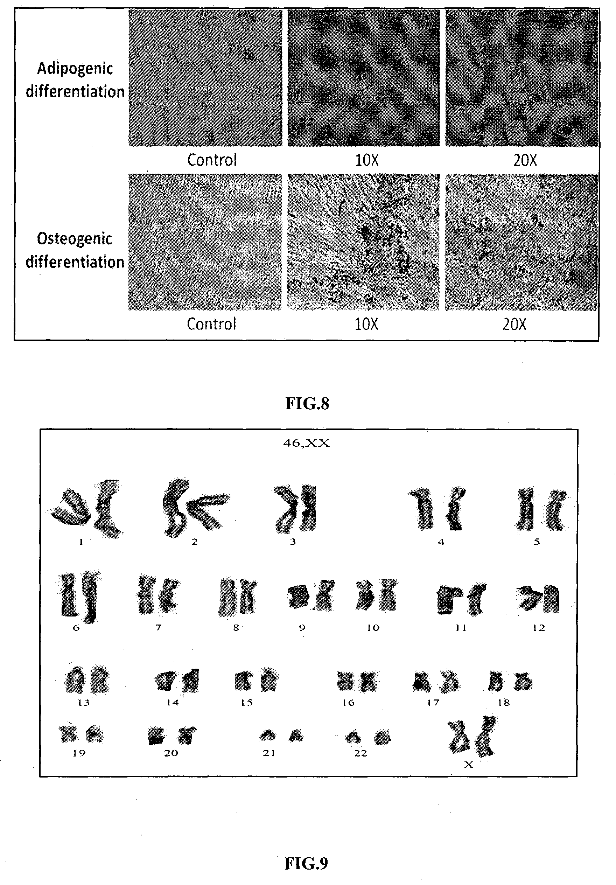

Multipotent differentiation potential of human perinephric-mesenchymal stem cells (hPNF-MSCs) was evaluated at passage 2 and passage 5. The cells were induced for osteocytes and adipocytes differentiation for all the three donor samples. The hPNF-MSCs from all three donors successfully differentiated into both osteocytes and adipocytes. The staining was done for the cells. The cells which were differentiated into osteocytes evidently showed production of extracellular matrix deposition, confirmed by Von Kossa staining. The lipid droplets in adipogenic differentiation were confirmed by Oil red O staining. **FIG.8** is a photograph illustrating the multipotent differentiation human perinephric fat mesenchymal stem cells (hPNF-MSCs), according to an embodiment herein. The adipogenic and osteogenic differentiation are performed at passage 2.

### EXAMPLE-1.6-Karyotyping Analysis

The Karyotype analysis was performed for all the three donors. **FIG.9** illustrates the karyotype analysis of the human perinephric fat mesenchymal stem cells (hPNF-MSC), according to an embodiment herein. The cells demonstrated normal karyotype from passage 2. A representative data of one of the donor sample has been illustrated in **FIG.9** showing normal karyotype with 46 chromosomes, XX (female).

### EXAMPLE- 1.7-Secretome Profiling

The "secretome" refers to the number of factors secreted by the stem cells. The regenerative properties of the stem cells are attributed to the factors secreted by stem cells in the micro-environment. An evaluation of these factors quantitatively ensures the regenerative potential of the stem cells. An analysis of the secretome of the human perinephric fat derived mesenchymal stromal cells (hPF-MSCs). The validation and comparison of the data is done with secretome data of mesenchymal stem cells derived from subcutaneous fat or lipoaspirate (LA) which has been already characterized. For the comparison, the factors are divided into pro-inflammatory cytokines, anti-inflammatory cytokines, chemokines, growth factors and other bioactive factors. These factors individually or synergistically decide the regeneration potential of the stem cells type.

In this study, range of cytokines, chemokines and growth factors were evaluated using human cytokine panel kit (Milliplex). Since MSCs derived from lipoaspirate (LA) is extensively studied and well characterized, we used cytokine dataset obtained from LA-MSCs as the reference to examine differences in the cytokine secretion profiles obtained from the MSCs populations of PNF. The results are illustrated in Figures below. The comparison between PNF-MSCS and LA-MSCS results exhibited differences in levels of cytokines released. There were 4 bioactive factors (IL-4, IL-10, IL-Ra, and IFN-γ) secreted at higher concentrations and 4 bioactive factors (IL-13, MCP-1, GRO, and GM-CSF) secreted at significantly lower concentrations by hPNF-MSCs when compared to hLA-MSCs. Other bioactive factors did not show any significant difference between hPNF-MSCs and hLA-MSCs. To demonstrate whether cytokine profile of both samples follow any trend, the cytokines are categorized into five groups (pro-inflammatory, anti-inflammatory, chemokines, growth factors and others). A two-tailed t-test was performed to determine significant difference in cytokine secretions between both samples. Overall, there was no significant change in secretion of analyzed bioactive factors between both sample types (p<0.05).

The secretome analysis between hPNF-MSCs and the hLA-MSCs, shows that there is no significant difference (p>0.05). Hence it is concluded that mesenchymal stem cells (MSCs) derived from hPNF are analogous to hLA-MSCs in terms of cytokines secretions. Further it is inferred that hPNF-MSCs secrete pro-inflammatory, anti-inflammatory, growth factor and the other bioactive factors of great importance as a therapeutic tool in regenerative medicine.

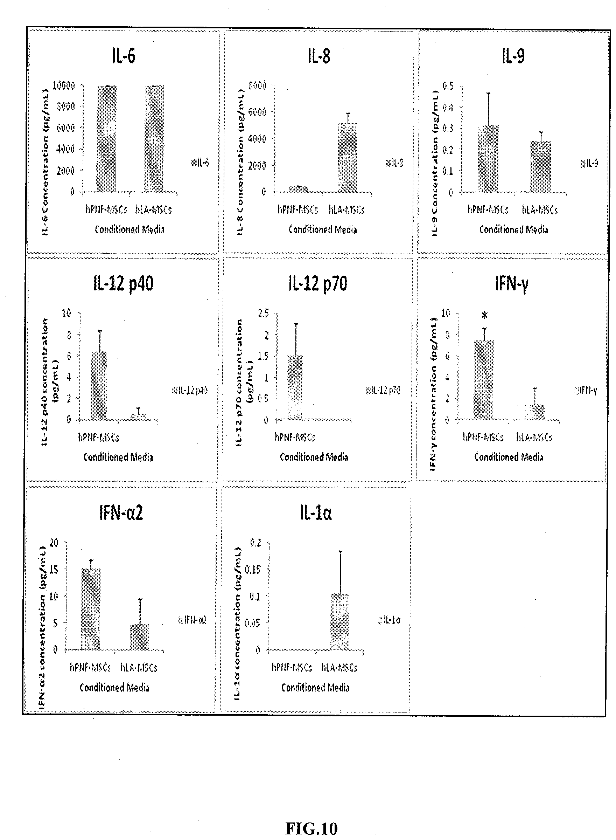

**FIG.10** is a graph illustrating the comparison of the pro-inflammatory cytokines secreted by human perinephric fat mesenchymal stem cells (hPNF-MSCs) derived from perinephric fat (PNF) and lipo-aspiration (LA), according to an embodiment herein. The average concentrations of the pro-inflammatory cytokines, IL-6, IL-8, IL-9, IL-12 p40, IL-12 p 70, IFN-γ, IFN-α2, and IL-1α are measured in the conditioned medium obtained from hPNF-MSCs and hLA-MSCs from passage 5. **FIG.10** illustrates that there is significant increase in the secretions of IFN-γ by hPNF-MSCs when compared to hLA-MSCs. There is no significant difference in secretion of other pro-inflammatory cytokines, including IL-6, IL-8, IL-9, IL-12 (p40), IL-12 (p70), IFN-α2, and IL-1α between PNF and LA MSCs. Both PNF and LA MSCs showed very high level of IL-6 cytokine secretion. IL-8 cytokine secretion is found to be higher in LA-MSCS when compared to PNF-MSCS.

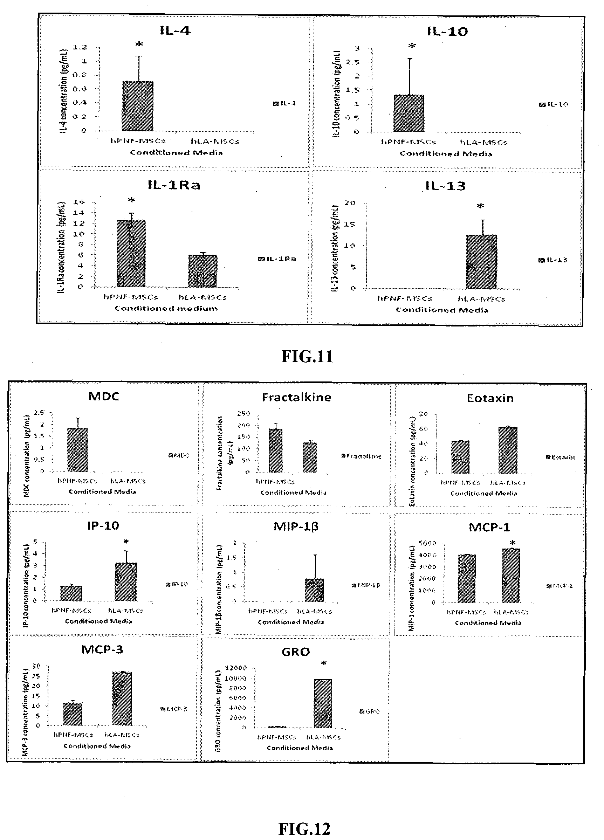

The anti inflammatory cytokines are analyzed including IL-3, IL-4, and IL-10. IL-4, IL-10, and IL-1Ra. The anti inflammatory cytokines showed significant increased in hPNF-MSCs when compared to hLA-MSCs. The IL-13 secretion is found to be significantly higher in hLA-MSCs.**FIG.11** is a graph illustrating the comparison of the anti-inflammatory cytokines secreted by human perinephric fat mesenchymal stem cells (hPNF-MSC) derived from perinephric fat (PNF) and lipoaspiration (LA), according to an embodiment herein. The average concentrations of the anti-inflammatory cytokines, IL-4, IL-10, IL-1Ra and IL-13 were measured in the conditioned medium of the PNF-MSCs and LA-MSCs from passage 5.

**FIG.12** is a graph illustrating the comparison of the chemokine secreted by human perinephric fat mesenchymal stem cells (hPNF-MSC) derived from perinephric fat (PNF) and lipoaspiration (LA), according to an embodiment herein. The MDC, Fractalkine, IP-10, MIP-1β, eotaxin, MCP-1, MCP-3, and GRO are the chemokines analyzed. Most of the investigated chemokines except MCP-1, IP-10, and GRO, reported no significant change in concentrations between hPNF-MSCs and hLA-MSCS. There was highly significant difference in levels of GRO between both samples. The average concentrations of the chemokines, MDC, Fractalkine, IP-10, MIP-1β, Eotaxin, MCP-3, MCP-1, and GRO were measured in conditioned medium of hPNF-MSCs and hLA-MSCs from Passage 5.

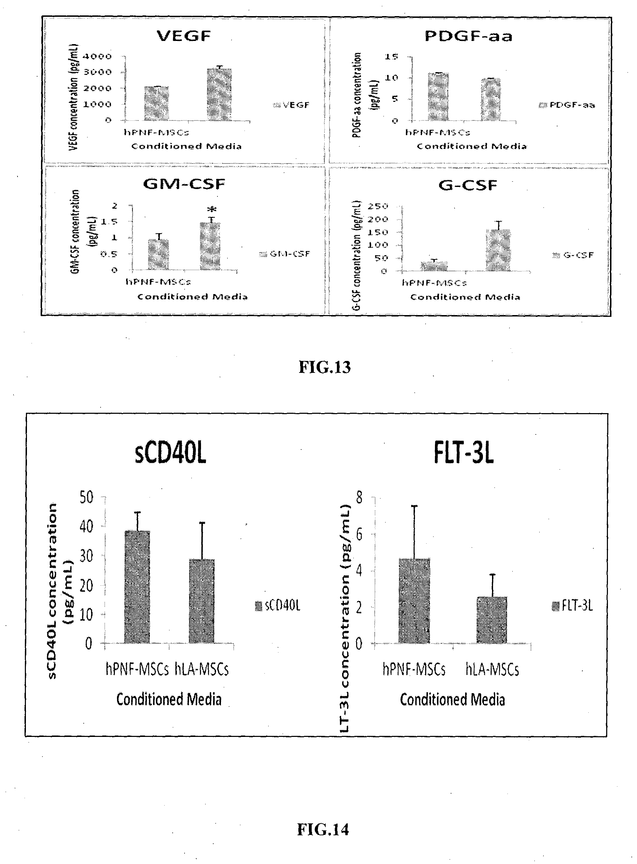

The levels of G-CSF, GM-CSF, VEGF and PDGF-aa were analyzed. The concentrations of the growth factors, G-CSF, GM-CSF, VEGF, and PDGF-aa were measured in conditioned medium of hPNF-MSCs and hLA-MSCs from Passage 5. **FIG.13** is a graph illustrating the comparison of the growth factors secreted by human perinephric fat mesenchymal stem cells (hPNF-MSC) derived from perinephric fat (PNF) and lipoaspiration (LA), according to an embodiment herein. As shown in the **FIG. 13****,** G-CSF, GM-CSF, VEGF and PDGF-aa are secreted in a considerable concentration by both sample types. However, the level of GM-CSF is reported to be significantly higher in hLA-MSCS when compared to hPNF-MSCs. The other growth factors, including G-CSF, PDGF-aa, and VEGF show no significant differences between two sample types. The secretion of pro-angiogenic factor, VEGF was found to be higher, approximately 2000-4000 pg/mL. The EGF level is found to be negligent (0.6 ± 1.25 pg/mL in PNF-MSCS and 0.00 pg/mL in LA-MSCS) in both sample types.

**FIG.14** is a graph illustrating the comparison of the other bioactive factors secreted by human perinephric fat mesenchymal stem cells (hPNF-MSC) derived from perinephric fat (PNF) and lipoaspiration (LA), according to an embodiment herein. The concentrations of the cytokines, sCD40L, and FLT-3L were measured in conditioned medium of PNF-MSCs and LA-MSCs from Passage 5. Both sCD40L and FLT-3L are found to be secreted at a similar concentration by both sample types.

### EXAMPLE 2 -Differentiation in Neural and Astroglial Lineages

For neural differentiation, spheres are generated using Ing/mL bFGF in DMEM-KO media supplemented with 1% FBS. These spheroids-like bodies were allowed to be in same media for 24hrs. Next day, the growth medium was replaced by neural induction medium including DMEM-F12 supplemented with 1% FBS, 1% N2, 2% B27, and 20 ng/mL bFGF (Life technologies). Medium was replenished after every 3-4 days. On 15th day, cells were fixed with 4% PFA for further characterization. A simultaneous induction procedure was performed on monolayer culture. Pictures were clicked during 15 days differentiation protocol.

For the astroglial differentiation the spheres were generated using 1 ng/mL in DMEM-KO media supplemented with 1% FBS. These spheroids like bodies were allowed to be in same media for 24 hours. Next day, the growth medium was replaced by astroglial induction medium including DMEM-F12 supplemented with L-thyroxine (T4-Sigma), 1% FBS, 1% N2, and 30 ng/mL T4. The 1% FBS is a nutritional supplement, 1% N2 is a cocktail of growth factor comprises 5µg/ml insulin, 100 µg/ml transferrin, 20nM progesterone, 100µM putrescine, 30nM selenium is responsible for growth and maintenance of astroglial cells in vitro, 2% B27 is a supplement for the growth of astroglial cells in vitro, and 20 mg/ml bFGF is required for the differentiation of mesenchymal stem cells (MSCs) into neuronal progenitor cells using Wnt pathway. The 30ng/ml of T4 is required for the differentiation of MSCs into astroglial cells. The medium is replenished after every 3-4 days. On the 15^{th} day, cells are fixed with 4% PFA for further characterization. A simultaneous induction procedure is performed on monolayer culture.

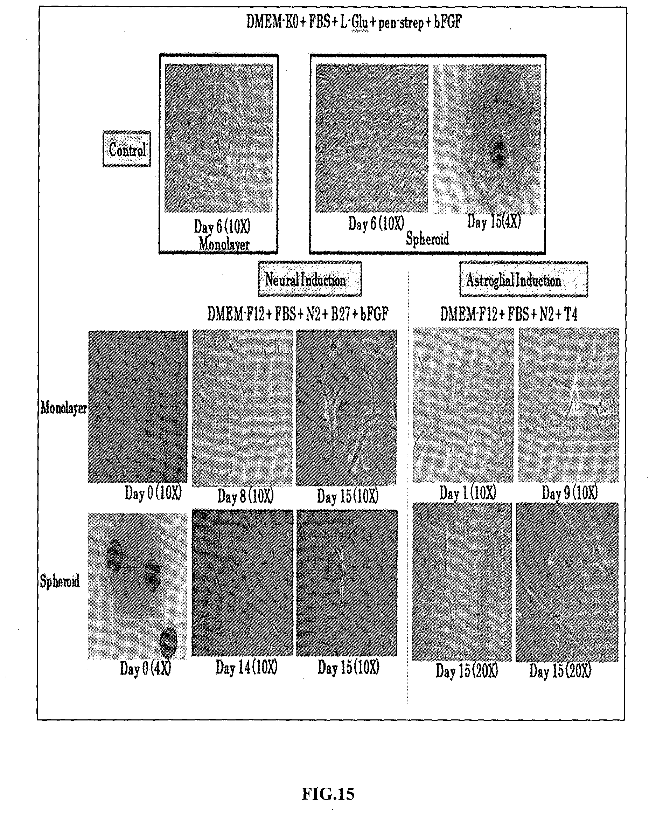

**FIG.15** is a photograph illustrating the differentiation in neural and astroglial lineages, according to an embodiment herein. The **FIG.15** shows the development of the monolayer and spheroid type culture of the hPNF-MSC. The **FIG.15** also illustrates the development of the neural and astroglial cell lineages on different days. The **FIG.15** further shows the formation of neurons and astroglial cells from human perinephric fat derived mesenchymal stromal cells. Hence it is inferred that the hPNF-MSC have high affinity to differentiate into neuroglial lineage and are primary source of stem cells for cellular therapy in neuro degenerative disorder.

### EXAMPLE 3 - Neural and Astroglial Induction

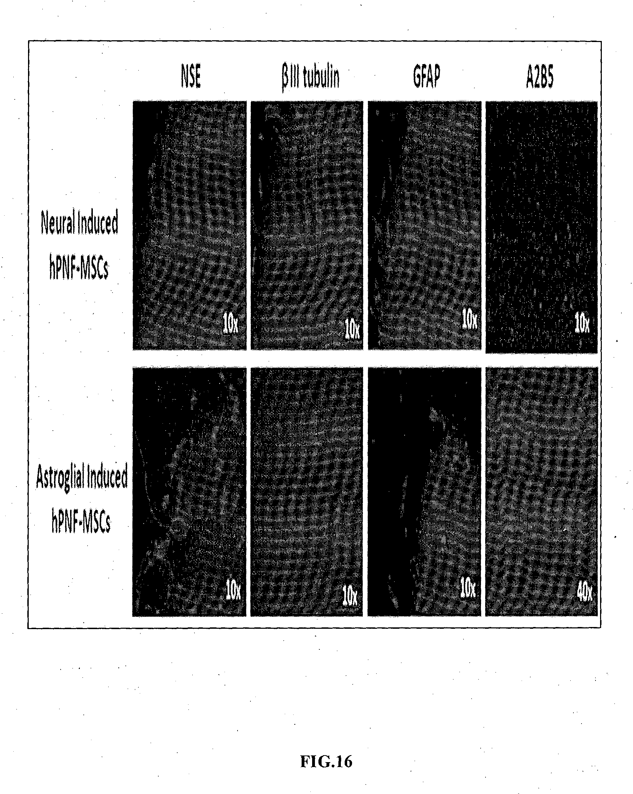

**FIG. 16** is a photograph illustrating the induction of neural and astroglial lineages, according to an embodiment herein. **FIG. 16** illustrates different day point during neural induction period. The cells adapted long and slender morphology with processes. The cells show connections as depicted in **FIG.16****.** The cells are characterized for neuronal markers by immuno-fluorescence staining. The cells are maintained at the expression level of NSE and β III tubulin. The negative expression of A2B5 is observed.

**FIG.16** also illustrates the different day point during astroglial induction period. The cells are adapted flat and star-shaped morphology with projections as depicted in **FIG.16****.** The cells are characterized for astroglial markers by immunofluorescence staining. A similar level of GFAP expression is found when compared to un-induced cells. The expression of late astroglial marker A2B5 is explicit after induction. Furthermore, down regulation in expression of neural-related markers (NSE and β III tubulin) are observed. **FIG. 16** shows a confirmatory result that the differentiated cells are neuronal and astroglial progenitors only by using the techniques of immunofluorescence. This is made possible by showing expressions of neuroglial related proteins in hPNF-MSC's. Hence it is inferred that the hPNF-MSC have high affinity to differentiate into neuroglial lineage and are primary source of stem cells for cellular therapy in neuro degenerative disorder.

### EXAMPLE 4 - Flow cytometry analysis of neuro-glial markers in undifferentiated hPNF-MSCs

To determine confirmatory results, flow cytometry analysis of neural and astroglial markers for hPNF-MSCs from early passage was performed. The human adipose tissue derived mesenchymal stem cells (hLA-MSCs) were considered as control in this study. A similar trend in expression of both neural and astroglial markers were observed as explicated by Immunofluorescence results. The flow analysis was performed on two donor samples and average expression percentage was taken into consideration. The cells from both the donor sample showed highly positive expression for neural markers including nestin (78.12 ± 16%), NSE (75.68 ± 6.18%), β III tubulin (85.56 ± 10.91%). The flow cytometry analysis for astroglial markers showed moderate expression of GFAP (53.31 ± 14.11%) and very low expression of A2B5 (19.07 ± 5.35%) similar to IF results. However, the expression level of GFAP was found to be contradictory to IF results. LA-MSCs were used as a control for comparative analysis. The results obtained from PNF-MSCs were found to be concomitant to LA-MSCs, demonstrating its analogy to MSCs.

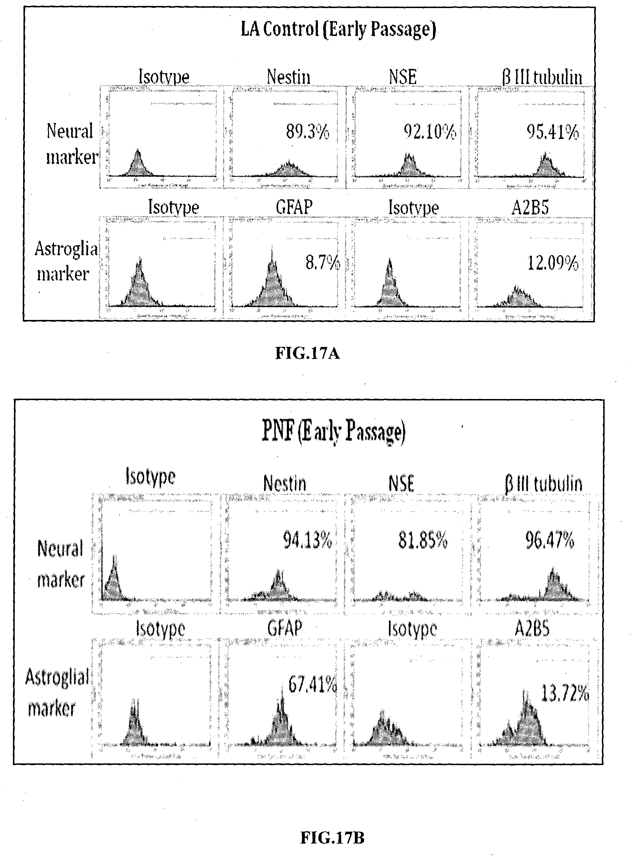

**FIG.17A** is a graph illustrating the flow cytometry analysis of the neuro-glial markers in the human perinephric fat mesenchymal stem cells (hPNF-MSCs) derived by lipoaspiration (LA) control (early passage), according to an embodiment herein. In the neural markers Nestin, NSE and β-III tubulin showed the expression level of 89.3%, 92.10% and 95.41% respectively. In the astroglial markers the GFAP and A2B5 showed the expression level of 8.7% and 12.09% respectively.

**FIG.17B** is a graph illustrating the flow cytometry analysis of the neuro-glial markers in the human perinephric fat mesenchymal stem cells (hPNF-MSCs) derived by perinephric fat (PNF) (early passage), according to an embodiment herein. In the neural markers Nestin, NSE and βIII tubulin showed the expression level of 94.13%, 81.85% and 96.47% respectively. In the astroglial markers the GFAP and A2B5 showed the expression level of 67.41% and 13.72% respectively.

Table 2 below summarizes the results of the neural and astroglial related marker expression in un-induced human perinephric fat mesenchymal stem cells (hPNF-MSCs):

| **LA (Control)** | **Marker** | **PNF 2** | **PNF 4** | **Mean** | **SEM** |
|---|---|---|---|---|---|
| 89.30% | **NESTIN** | 94.13% | 62.12% | 78.12% | 16.00% |
| 92.10% | **NSE** | 81.85% | 69.50% | 75.68% | 6.18% |
| 95.41% | **β III tubulin** | 96.47% | 74.64% | 85.56% | 10.91% |
| 8.68% | **GFAP** | 67.41% | 39.20% | 53.31% | 14.11% |
| 12.10% | **A2B5** | 13.72% | 24.42% | 19.07% | 5.35% |

### EXAMPLE 5 -Generation of 3D Spheroid

For the generation of 3D spheroid small drops (20-30 µl) of MSCs cell suspension containing 5000-20000 cells/drop were plated on inner side of the 60 mm non-adherent TarsonsPetridish (Tarsons Products, Kolkata, WB) in hanging drop fashion. Approximately 10-15 drops were created per plate and the bottom of plates was overlaid with DPBS to avoid loss of nutrients through desiccation. The Plates were incubated for 48 hrs in hanging drop mode at 37°C with 5% CO₂. After 48hrs incubation period, the hMSC spheroid aggregates were transferred to a 60 mm non-adherent dish with fresh culture media for further experiments. The spheroids were cultured for 18 days in culture medium, replenished after every 3days. A new approach to observe, whether perinephric fat-mesenchymal stem cells (PNF-MSCs) can generate spheroids to represent 3D-model was adopted. The cells from all the groups successfully generated spheroids by hanging drop method. The cells from early passage were used for generation of spheroids. Cells emerging out of spheroids showed fairly representative morphology of MSCs. A remarkable difference in morphology was observed when the spheroids were cultured in non-adherent dishes.

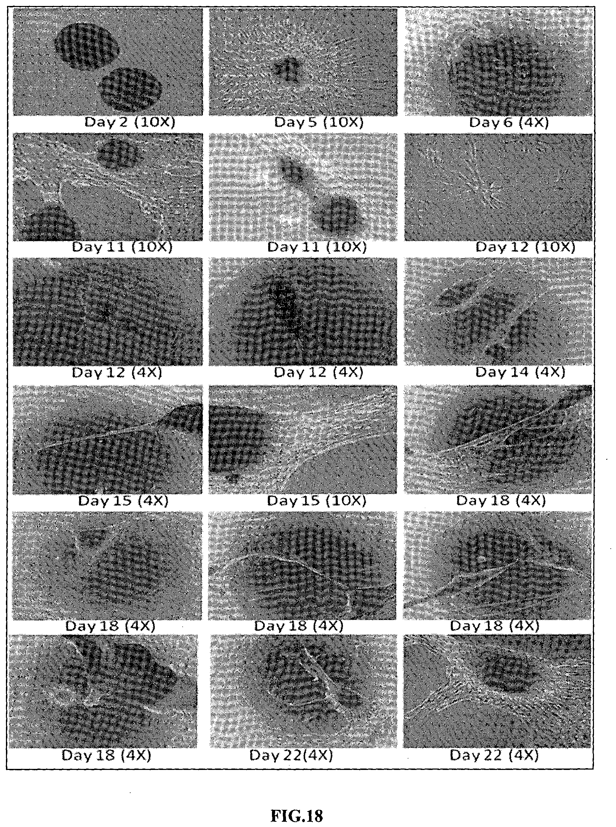

**FIG.18** is a photograph illustrating generation of spheroids from human perinephric fat mesenchymal stem cells (hPNF-MSCs), according to an embodiment herein. **FIG.18** illustrates a large number of cells budding out from the spheroids forming colonies of cells at distal ends are seen. Further a tube like meshwork is observed projected from spheroids.

### EXAMPLE 6 -Renal Cell Lineage Differentiation

To differentiate the hPNF- MSCs to renal lineage Renal Epithelial Growth Medium was used. The growth factors that induce differentiation are BMP2, Retinoic acid and Activin A. The Activin A plays a major role in mesoderm specification. The Retinoic acid acts via the retinoid receptors RAR and aids in mesoderm patterning. In kidney the BMP plays a role in nephrogenesis. BMP's are expressed in metanephric mesenchyme and cause epithelialization of metanephricmesenchyme. As kidney responds to BMP stimulation, response to injury and repair frequently recapitulates development having the same function of epithelialization of metanephric mesenchyme.

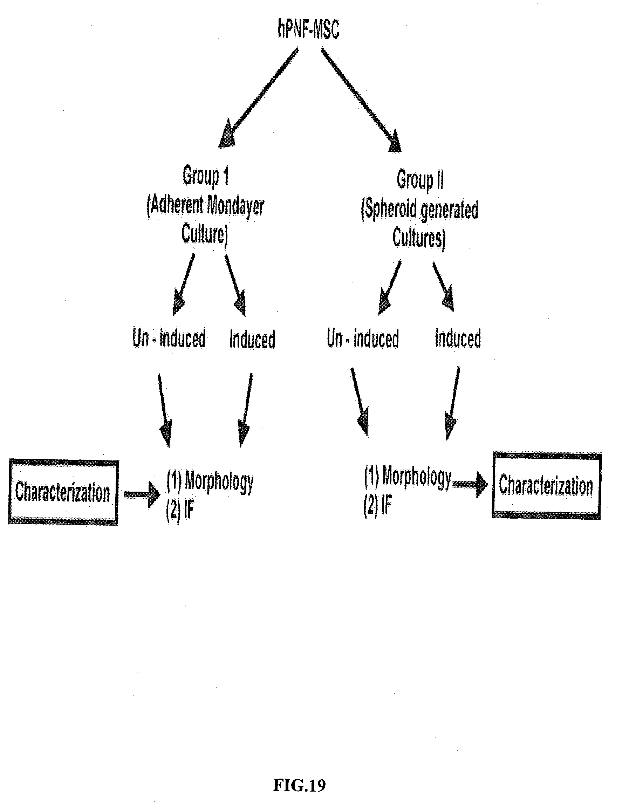

Two different methods were used to carry out the differentiation of hPNF-MSCs - Adherent monolayer and through formation of spheroids. **FIG. 19** is a flow chart illustrating the differentiation of human perinephric derived mesenchymal stem cells (hPNF-MSCs) into the renal cells lineages, according to an embodiment herein. The flow chart illustrates the two groups of the cultures of hPNF-MSC i.e. Group 1-Adherent Monolayer Culture and Group 2- Spheroid generated Culture. The Adherent Monolayer cultures of hPNF-MSC are subjected to induced and un-induced culture. The Spheroid generated cultures of hPNF-MSC are subjected to induced and un-induced culture. After the culture the induced and un-induced cultures are subjected to characterization.

### Adherent monolayer culture:-

The hPNF- MSCs (P3) were cultured on autoclaved coverslips in 35 mm dishes, this included one un-induced and two induced culture dishes. The un-induced dish contained Renal Epithelial Growth Medium (REGM) with REGM supplement mix and the induction dishes were supplemented with REGM medium, REGM supplement mix, BMP2 (10ng/ml), Retinoic acid (0.1µM/ml) and Activin A (10ng/ml). This differentiation medium was then syringe filtered using a 0.22 micron filter. The cell were incubated at 37°C in 5% CO2 incubator for 17 days to identify differentiation to metanephric mesenchyme and 22 days for podocyte and proximal convoluted tubule. The culture medium was replenished after 48 hrs.

### Formation of Spheroids:-

To generate spheroids from hPNF-MSCs (P3), approximately 10,000 cells were plated as drops on the lid of 60mm Tarsons Petridish (30 drops per dish). The bottom of the petridish was overlaid with DPBS to avoid desiccation. The petridish was kept at 37°C in 5% CO2 for 24 hrs. The MSC spheroids thus formed were transferred onto autoclaved cover slips in 35 mm adherent dishes supplemented with renal differentiation medium. These were cultured for 17 days to identify metanephric mesenchyme and 22 days for podocyte and proximal convoluted tubule.

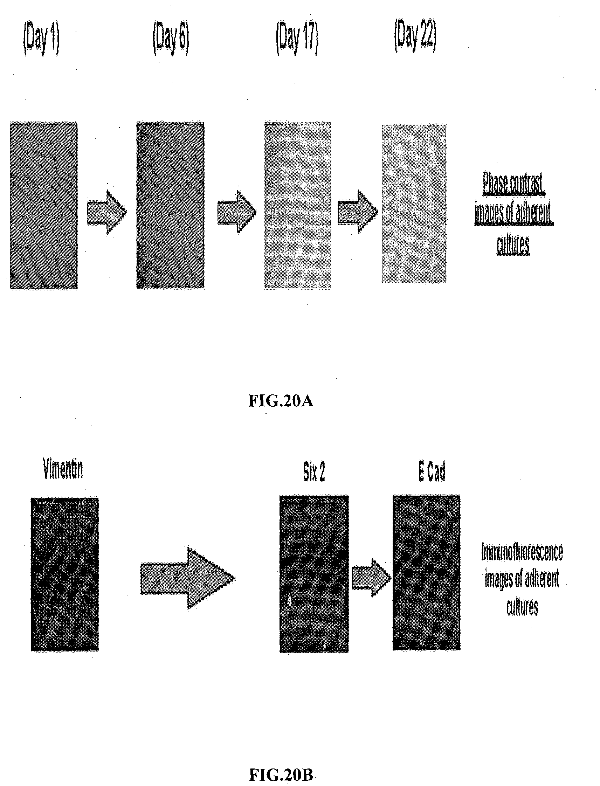

**FIG.20A** is a photograph illustrating the phase contrast image of the adherent renal progenitor cells, according to an embodiment herein. The **FIG.20A** illustrates the 10X magnification view of adherent cultures at day 1, day 6, day 17 and day 22 using phase contrast microscope.

**FIG.20B** is a photograph illustrating the Immunoflurescence image of the renal progenitor cells, according to an embodiment herein. The **FIG.20B** illustrates the Immunoflurescence image of adherent cultures showing positive for Vimentin at day 1, six2 at day 17 and E Cad at day 22. After day 17 the adherent monolayer culture and spheroid cultures show positive result for six2 i.e. a metanephric mesenchyme marker. After 22 days the proximal convoluted tubule marker E Cad is observed.

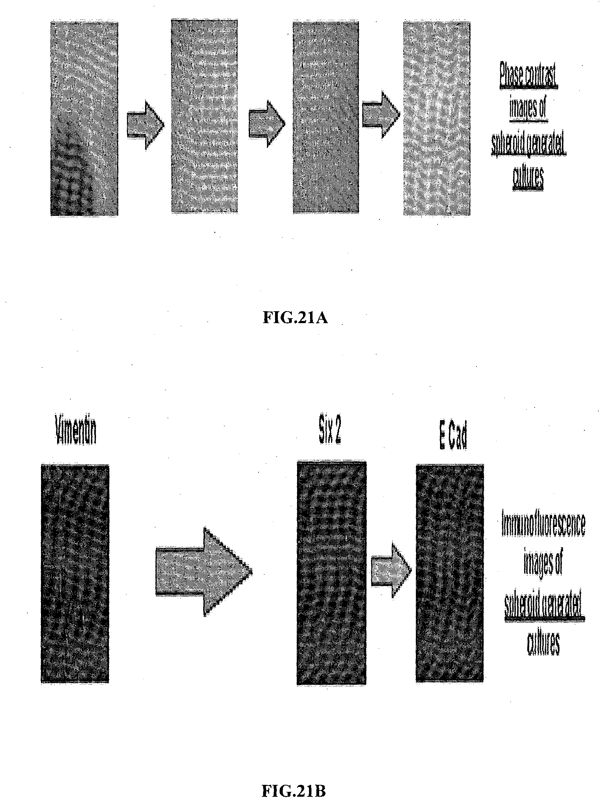

**FIG.21A** is a photograph illustrating the phase contrast image of the spheroid renal progenitor cells, according to an embodiment herein. The **FIG. 21A** illustrates the 10X magnification view of adherent cultures at day 1, day 6, day 17 and day 22 using phase contrast microscope.

**FIG. 21B** is a photograph illustrating the immuno-fluorescence image of the spheroid renal progenitor cells, according to an embodiment herein. The **FIG. 21B** illustrates the immunofluorescence image of adherent cultures showing positive for vimentin at day 1, six2 at day 17 and E Cad at day 22.

After 17 days adherent monolayer cultures and spheroid generated cultures shows a positive for Six 2 - Metanephric mesenchyme marker and 22 days for proximal convoluted tubule marker E CAD. The pathway involved in the renal lineage differentiation is the Wnt pathway plays a critical role in the development of kidney. The Wnt family members expressed during kidney development are Wnt 2b, Wnt 9 etc. The growth factors like BMP2, Actvin A and Retinoic acid activates the Wnt signaling pathway enabling differentiation of hPNF-MSCs to Renal Progenitor cells.

### EXAMPLE 7 -Differentiation into Islet like Cell Lineages

For differentiating human perinephric mesenchymal stem cells (hPNF-MSCs) to islet like cell clusters, the cells from passage 3 (P3) were seeded into three different 35mm non adherent culture dishes or plates. One dish /plate was labeled as control and the two dishes/plates were for cell differentiation. The un-induced/control culture dish/plate was supplemented with culture medium comprising only Dulbecco's Modified Eagle's Medium-Knock Out (DMEM-KO) without fetal bovine serum (FBS) and basic fibroblast growth factor (bFGF).The induction dish or cell differentiation dishes/plates were having culture medium comprising of 1.5% BSA, 1X ITS in DMEM-KO medium. The control culture dish/plate and the cell differentiation dish/plates were incubated in 5 % CO₂ incubator at 37°C. After incubation on day 4, all the floating cell clusters with medium were collected in a sterile centrifuge tube with a conical bottom. The tubes were allowed to stand for about 15- 20 minutes, thus allowing the cells to settle at the bottom. The supernatant was carefully decanted and the cells from bottom of the centrifuge tube were re-seeded in the day 4 medium. The day 4 medium comprised of the following:- DMEM-KO medium, 1.5% BSA, 1X ITS and 0.3mM Taurine was added. On day 7 the cells were fed with fresh day 4 medium. On Day 10 the floating cell clusters with medium were collected in a sterile centrifuge tube with a conical bottom. The tubes were allowed to stand for about 15- 20 minutes, thus allowing the cells to settle at the bottom. The supernatant was decanted. The cell pellet comprised of cell clusters. The cell clusters were re-seeded into day 10 medium comprising BSA at a concentration of 1.5%, 1X ITS and 0.3 mM Taurine in DMEM-KO medium and additionally 100mM nicotine amide. The cell clusters are subjected for dithizone (DTZ) staining and immunofluorescence analysis.

### Dithiazone (DTZ) Staining Analysis:-

For dithiazone (DTZ) analysis 10 islet cell clusters were handpicked and were inoculated in a fresh petridish/culture dish. The islet cell clusters were incubated in 10µl DTZ stock suspended in 1ml of phosphate buffer saline (PBS) consisting of calcium and magnesium salts at 37C for one hour. The brick red colour stained islets were observed under inverted microscope.

### Immunofluorescence Analysis:-

For immunofluorescence analysis 10 islet cell clusters were handpicked and were inoculated in a sterile Eppendorf tube. The islet cell clusters were then fixed using 4% paraformaldehyde and were blocked using 3% bovine serum albumin (BSA). After blocking, the islet cell clusters were washed thrice with washing solution phosphate buffer saline tween 20 (PBST). The islet cell clusters were incubated with primary antibody (C- Peptide) diluted in blocking solution and kept overnight at 4°C. Following day, the islet cell clusters were washed thrice in washing solution (PBST) and incubated in dark for 60 minutes at room temperature with appropriate FITC conjugated secondary antibodies (1:50 Goat Anti Mouse IgG-Fitc) diluted in PBS. The islet cell clusters were washed in PBST followed by counterstaining with a nuclear marker 4',6'-diamidino-2-phenylindole (DAPI) solution for 10 minutes. The islet cell clusters were washed with PBST and mounted with 1,4-diazabicyclo(2.2.2) octane (DABCO) onto a slide. They were examined under Nikon Eclipse 80i digital microscope and images were captured by Nikon's Digital Sight DS-Fil (5M2) camera using NIS- Elements F3.0 imaging software provided by Nikon.

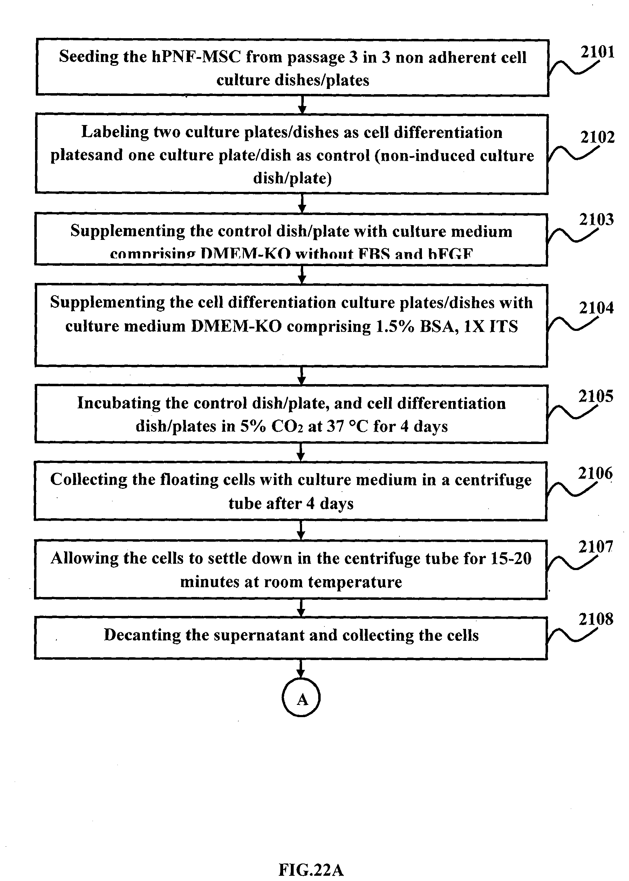

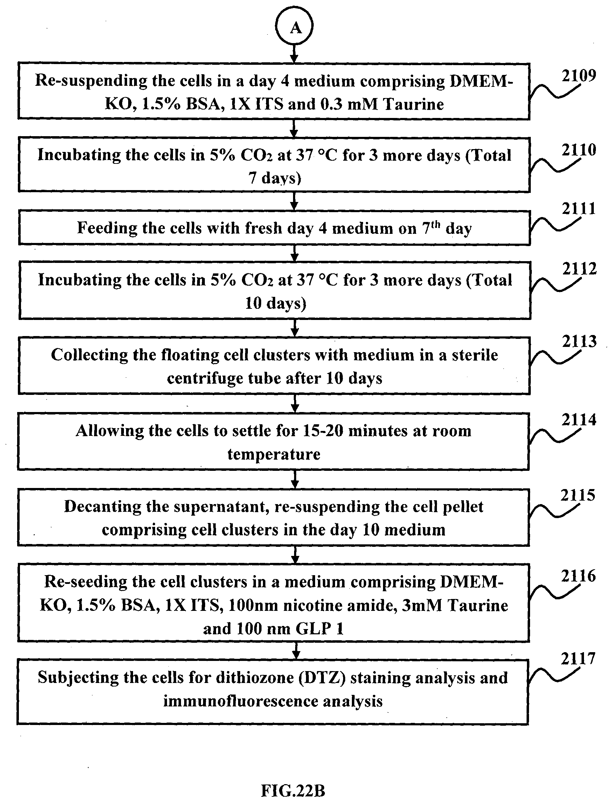

**FIG. 22A** **and** **22B** jointly illustrates a flow chart explaining a method for differentiating the human perinephric fat derived mesenchymal stem cells (hPNF-MSCs) into pancreatic progenitor cells, according to an embodiment herein. The human perinephric mesenchymal stem cells (hPNF-MSCs) are seeded from passage 3 (P3) in three non-adherent cell culture dishes /plates (2101). Two cell culture dishes/plates are labeled for cell differentiation (hPNF-MSCs differentiation into pancreatic progenitor cell clusters) and one cell culture dish/plate is labeled as control (un-induced culture dish/plate) (2102). The control cell culture dish/plate (un-induced culture dish/plate) is supplemented with only DMEM-KO culture medium without FBS and bFGF (2103). The cell differentiation/induction medium comprises Day 0 medium comprising Dulbecco's Modified Eagle's Medium (DMEM-KO), 1.5% bovine serum albumin (BSA) and IX insulin-transferrin-selenium (ITS) (2104). The two cell differentiation (hPNF-MSCs differentiation into pancreatic progenitor cell clusters) plates/dishes are supplemented with induction medium i.e. culture medium comprising Dulbecco's Modified Eagle's Medium (DMEM-KO), 1.5% bovine serum albumin (BSA) and 1X insulin-transferrin-selenium (ITS). The two cell differentiation (hPNF-MSCs differentiation into pancreatic progenitor cell clusters) and one cell culture dish/plate control (un-induced culture dish/plate) are incubated in 5% CO₂ at 37°C for four days (2105). The floating cell culture with medium is collected in a centrifuge tube after four days (2106). The cells are allowed to settle down in the centrifuge tube for 15-20 minutes (2107). The cells will settle down to form pellet. The supernatant is decanted and the cell pellet is collected (2108).The cells from cell pellet are re-seeded in a 4 day medium. The 4-day medium comprises Dulbecco's Modified Eagle's Medium (DMEM-KO), 1.5% bovine serum albumin (BSA), 1X insulin-transferrin-selenium (ITS), and 0.3 mM Taurine (2109). The cells are incubated in 5% CO₂ at 37°C for 3 more days (Total 7 days) (2110). The cells are fed with fresh 4-day medium on 7^{th} day (2111). The cells are incubated in 5% CO₂ at 37°C for 3 more days (Total 10 days) (2112). The floating cell clusters are collected with medium in a sterile centrifuge tube after 10 days (2113). The cells are allowed to settle for 15-20 minutes at room temperature (2114). The cells will settle down to form the pellet. The supernatant is decanted (2115). The cell pellet comprises cell clusters. The cell clusters are re-seeded in a day 10 medium comprising DMEM-KO, 1.5% BSA, 1X ITS, 100nm nicotine amide, 3mM Taurine and 100 nm GLP1 (2016). The cells are subjected for dithiozone (DTZ) staining analysis and immunofluorescence analysis (2017).

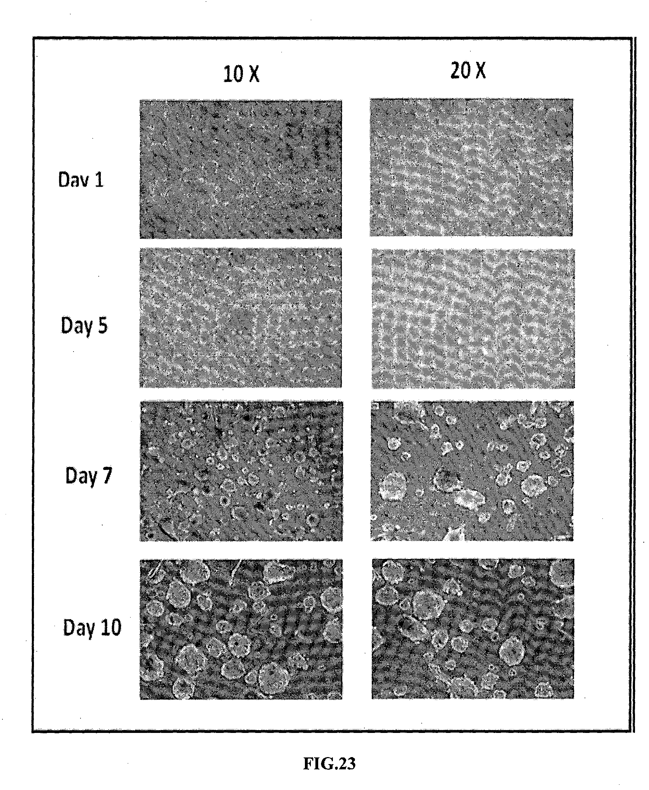

**FIG. 23** is a photograph illustrating the morphology of islet like cell clusters in the differentiation culture plates, resembling pancreatic islets, according to an embodiment herein. Within 24 hours cell clusters were observed in the induced dish resembling pancreatic islets.

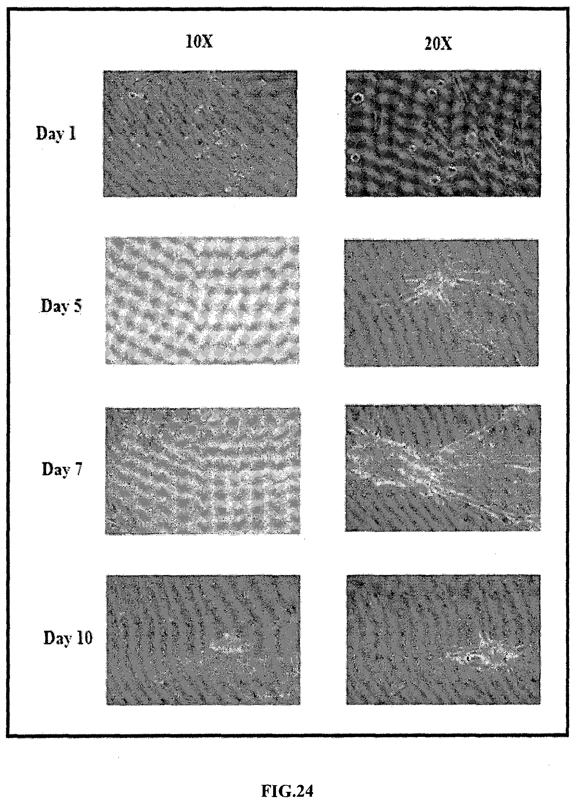

**FIG. 24** is a photograph illustrating the morphology of un-induced human perinephric fat derived mesenchymal stem cells (hPNF-MSCs) (control for differentiation culture plate), according to an embodiment herein. The cells in the un-induced dish formed aggregates initially but after a certain period cells were found to attach.

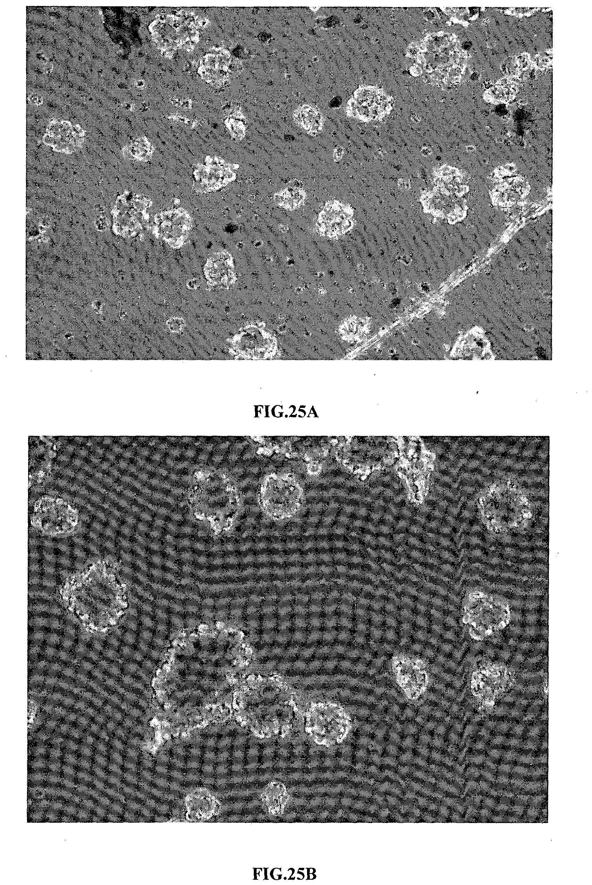

**FIG.25A and 25B** are photographs illustrating the characterization of islet like cell clusters generated from human perinephric fat derived mesenchymal stem cells (hPNF-MSCs) by dithizone (DTZ) staining, according to an embodiment herein. Following ten days of incubation period the islet cell-clusters thus formed are characterized by DTZ staining. Islet cell clusters are positive for DTZ staining (a zinc chelating agent that selectively stains pancreatic beta cells).

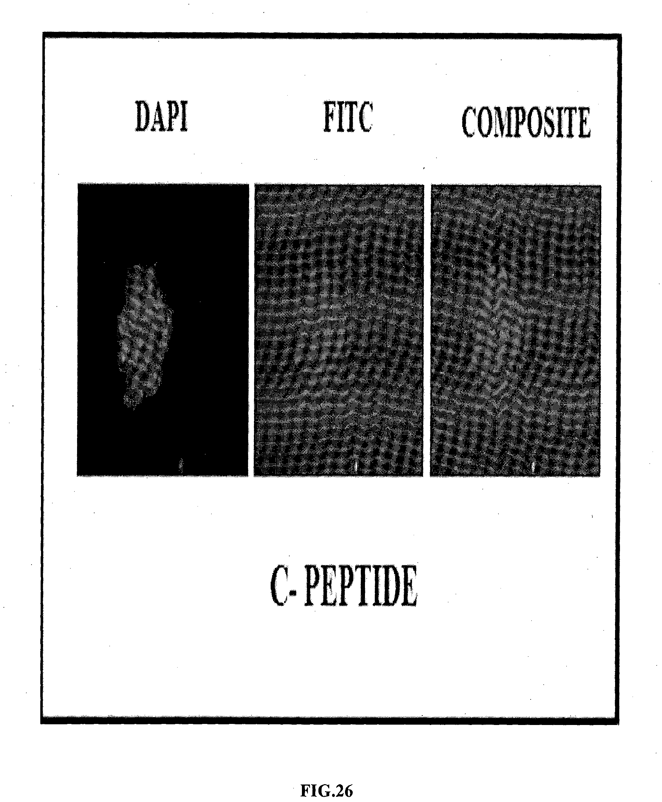

**FIG. 26** is a photograph illustrating the characterization of islet like cell clusters generated from human perinephric fat derived mesenchymal stem cells (hPNF-MSCs) by immunofluorescence staining for C-peptide, according to an embodiment herein. Following ten days of incubation period the islet cell-clusters thus formed are characterized by immunofluorescence. Further islet cell clusters were characterized by immunofluorescence staining for c-peptide (Late marker). The cell clusters showed a high expression of C-peptide, thus confirming the generation of functional islet cell clusters.

The foregoing description of the specific embodiments will so fully reveal the general nature of the embodiments herein that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments.

It is to be understood that the phraseology or terminology employed herein is for the purpose of description and not of limitation. Therefore, while the embodiments herein have been described in terms of preferred embodiments, those skilled in the art will recognize that the embodiments herein can be practiced with modification within the spirit and scope of the claims submitted below. The scope of the invention will be ascertained by the following claims.

## Claims

1. A method for isolating and characterizing human perinephric fat derived stem/stromal cells (hPNF-MSCs), for propagation, regeneration and differentiation into astroglial cells, renal cells, neuronal cells and islet cells, the method comprises the steps of:
in a perinephric fat tissue collected from a volunteer or a kidney patient with a prior informed consent;
processing the perinephric fat tissue in a sterile environment, and wherein the sterile environment comprises Current Good Manufacturing Practices(cGMP) compliant clean room with a biosafety cabinet;
isolating the mesenchymal stem/stromal cells from the perinephric fat tissues using a plurality of methods, and wherein the plurality of methods are a mechanical method and an enzymatic method;
washing the tissue sample in a 1X Dulbecco's phosphate Buffered Saline (DPBS) solution comprising of antibiotics at a concentration of 2X concentration, and wherein the tissue sample is washed for a plurality of times, and wherein the antibiotic is Pen -Strep;
mincing the tissue sample mechanically into a plurality of small pieces with a sterile scalpel blade;
treating the tissue sample in a Dulbecco's Modified Eagles medium (DMEM-KO) comprising an enzyme and the Pen-Strep, and wherein the tissue sample is treated for 60 minutes at 37 °C on a laboratory rocker, and wherein the enzyme is a Collagenase type I, and wherein the Collagenase type I is mixed at a predetermined concentration, and wherein the predetermined concentration of Collagenase type I is 0.2% v/v;
adding the DMEM-KO medium with a fetal bovine serum (FBS) to neutralize an enzymatic action of Collagenase, and wherein the FBS is present in a predetermined concentration, and wherein the predetermined concentration of FBS is 5% v/v, and wherein DMEM-KO medium with the FBS neutralizes the enzymatic action of Collagenase;
obtaining a homogenate comprising mesenchymal stem/stromal cells from the perinephric fat tissues;
filtering the homogenate comprising mesenchymal stem/stromal cells from the perinephric fat tissues through a cell strainer to obtain a filtrate, and wherein the cell strainer has a pore size of 40µm;
collecting the filtrate comprising mesenchymal stem/stromal cells from the perinephric fat tissues;
centrifuging the filtrate comprising mesenchymal stem/stromal cells at 1500 rpm for 2 minutes;
collecting a cell pellet comprising mesenchymal stem/stromal cells after a completion of centrifuging process;
re-suspending the pellet in a culture medium, and wherein the culture medium comprises a DMEM-KO, a fetal bovine serum (FBS) at a concentration of 10 %, a L-glutamate at a concentration of 1X, a Pen-Strep, and a basic fibroblast growth factor (bFGF) at a predetermined concentration, and wherein the predetermined concentration of the bFGF is 1 ng/ml;
obtaining a cell suspension of mesenchymal stem/stromal cells;
seeding the mesenchymal stem/stromal cell suspension in a cell stack, and wherein the cell stack comprises 200 ml cell culture medium;
rocking the cell stack for even distribution of cells and culture medium, and wherein the cell stacks are incubated in a humidified incubator at a temperature of 37°C, and wherein the humidified incubator consist of a carbon dioxide (CO₂), and wherein the concentration of CO₂ is 5%v/v;
replenishing the culture medium after 48 hours, and wherein the culture medium is replenished to eliminate non adherent cells, and wherein adherent fibroblast like populations of cells are allowed to expand in culture medium until a cellular confluency of 80% is reached for harvesting;
replenishing the culture medium 2-3 times every week;
trypsininzing and harvesting the hPNF-MSCs from the cell stacks, after the cells attain a confluency of 80%;
counting the mesenchymal stem/stromal cell population;
cryopreserving the hPNF-MSCs in a cryovial, and wherein the cryovial is labelled with sample ID, cell number, date of isolation, and wherein the cryovial is labelled as passage 0 (P₀), and subjecting the hPNF-MSCs to expansion; and
wherein the hPNF-MSCs are subjected to characterization, and wherein the isolated and characterized hPNF-MSCs are subjected to differentiation into neural cells, and wherein the isolated and characterized hPNF-MSCs are subjected to differentiation into astroglial cells, and wherein the isolated and characterized hPNF-MSCs are subjected to differentiation into renal cells, and wherein the isolated and characterized hPNF-MSCs are subjected to differentiation into islet cells.

2. The method according to claim 1, wherein the step of cryopreserving comprises the steps of:
preparing a freezing mixture, and wherein the freezing mixture comprises of fetal bovine serum at a concentration of 90% v/v, dimethyl sulfoxide 10%v/v and mesenchymal stem cells (MSC);
cooling the mixture at a temperature of 4°C;
centrifuging the cell suspension at 1400 rpm for 10 min at room temperature;
obtaining a cell pellet and discarding the supernatant;
adding the freezing mixture dropwise to the cell pellet;
mixing the freezing mixture and cell pellet;
transferring the mixture in a cryovial;
incubating the cryovial at a temperature of -80°C; and
transferring the cryovial into liquid nitrogen cryotank.

3. The method according to claim 1, wherein the isolated human perinephric fat derived stem/stromal cells (hPNF-MSCs) are subjected to characterization for confirming the cells to be the mesenchymal stem/stromal cells, and wherein the isolated human perinephric fat derived stem/stromal cells (hPNF-MSCs) are analyzed for a seeding density analysis, a morphological analysis, a growth kinetic study, an immunophenotypic analysis, a differentiation potential analysis, a karyotype analysis and a secretome profile analysis.

4. The method according to claim 3, wherein the isolated human perinephric fat derived stem/stromal cells (hPNF-MSCs) are seeded at a different densities in the seeding density analysis, and wherein the cells are seeded at a low density and a high density, and wherein the cells seeded at a low density have 1000 cells/cm², and wherein the cells seeded at high density have 2500 cells/cm², and wherein the cells seeded at high density maintain a growth kinetics and a cellular morphology, and wherein the high density cell culture have a cell senescence at passage 10 (P10).

5. The method according to claim 3, wherein the morphology of the isolated human perinephric fat derived stem/stromal cells (hPNF-MSCs) is similar to a morphology of a fibroblast, and wherein the hPNF-MSCs have the fibroblast morphology till a passage 7 (P7).

6. The method according to claim 3, wherein a cell population doubling time for the isolated human perinephric fat derived stem/stromal cells (hPNF-MSCs) is determined from passage 1 (P1) to passage 14 (P14), and wherein the population doubling time of the cells increases from 14. 38±1.29 hours at passage 2 (P2) to 235.85±0.65 hours at passage 9 (P9).

7. The method according to claim 3, wherein the isolated human perinephric fat derived stem/stromal cells (hPNF-MSCs) are analyzed/characterized for cell surface antigen marker, and wherein the hPNF-MSCs express a CD44 antigen and a CD90 antigen at a passage 0 (P0), and wherein the cells express a HLA-DR antigen, a CD 34 antigen and a CD 45 antigen in less than 4% of the cell population at a passage 5 (P5), and wherein 90% of the cell population express a CD 44 antigen, a CD 73 antigen, a CD 90 antigen, a CD 105 antigen and a CD 166 antigen at the passage 5 (P5), and wherein the cells have a low expression of the cell surface antigen at a passage 9 (P9) and a passage 15 (P15).

8. The method according to claim 3, wherein the secretome profile analysis of the isolated human perinephric fat derived stem/stromal cells (hPNF-MSCs) comprises analysis of cytokines, chemokines and growth factors.

9. The method according to claim 8, wherein the cytokines analyzed are a pro-inflammatory cytokine and an anti-inflammatory cytokine, and wherein the pro-inflammatory cytokine secreted by the hPNF-MSCs secrete IL-6, IL-9, IL-12, INF-a2, and IL-1α, and wherein IL-6 secretion is high in PNF-MSCs when compared to the other pro-inflammatory cytokine, and wherein anti-inflammatory cytokine secreted by hPNF-MSCs are a IL-4, a IL-10 and a IL-1Ra, and wherein IL-13 secretion is high in hPNF-MSCs when compared to other anti-inflammatory cytokine.

10. The method according to claim 8, wherein the hPNF-MSC secrete a plurality of chemokine including a MDC, a Fractalkine, a IP-10, a MIP-1β, an eotaxin, a PCP-1, a MCP-3 and a GRO.

11. The method according to claim 8, wherein the growth factors secreted by hPNF-MSC are a G-CSF, a GM-CSF, a VEGF and a PDGF-aa, and wherein the secretion of VEGF is at a concentration of 2000-4000 pg/ml.

12. The method according to claim 1, wherein the step of differentiating the isolated human perinephric fat derived stem/stromal cells (hPNF-MSCs) into neural cells comprises the steps of:
generating spheres with a basic fibroblast growth factor (bFGF) at a concentration of 1 ng/ml in a DMEM-MO medium, and wherein the DMEM-KO medium comprises a fetal bovine serum (FBS) at a concentration of 1% v/v;
incubating the generated spheres in the medium for 24 hours;
replacing the growth medium with a neural induction medium, and wherein the neural induction medium comprises a DMEM-F12 culture medium, and wherein the DMEM-F12 medium comprises fetal bovine serum (FBS) at a concentration of 1%v/v, a N2 at a concentration of 1% v/v, a B27 at a concentration of 2% and a bFGF at a concentration of 20 ng/ml, and wherein the FBS is a nutritional supplement, and wherein the N2 is a cocktail of the growth factor, and wherein the N2 comprises an insulin at a concentration of 5µg/ml, a transferrin at a concentration of 100 µg/ml, a progesterone at a concentration of 20 nM, a putrescine at a concentration of 100 µM, a selenium at a concentration of 30 nM, a B27 at a concentration of 2% v/v, and bFGF at a concentration of 20ng/ml, and wherein the selenium is added for growth and maintenance of neurons in vitro, and wherein the B27 is a growth supplement for neurons in vitro, and wherein the bFGF is a cell differentiating agent, and wherein the bFGF induces cell differentiation of the hPNF-MSCs into neuronal progenitor cells, and wherein the bFGF targets Wnt pathway;
replenishing the neural induction medium after every 3-4 days;
fixing the cells with at a concentration of 4%v/v for characterization; and
performing induction procedure on a monolayer culture of the cells.

13. The method according to claim 12, wherein the isolated human perinephric fat derived stem/stromal cells (hPNF-MSCs) have a positive expression for neural markers including nestin (78.12 ± 16%), NSE (75.68 ± 6.18%), β III tubulin (85.56 ± 10.91%), and wherein the neural marker expression confirms the differentiation of the hPNF-MSCs into neural cells.

14. The method according to claim 1, wherein the step of differentiating the isolated human perinephric fat derived stem/stromal cells (hPNF-MSCs) into astroglial cells comprises the steps of:
generating spheres with a basic fibroblast growth factor (bFGF) at a concentration of 1 nm/ml in a DMEM-KO medium, and wherein the DMEM-KO medium is supplemented with a fetal bovine serum (FBS) at a concentration of 1 % v/v;
incubating the generated spheres in the medium for 24 hours;
replacing the growth medium with an astroglial induction medium and a L-thyroxine, and wherein the astroglial induction medium comprises a FBS at a concentration of 1% v/v, a N2 at a concentration of 1% v/v and a T4 at a concentration of 30 ng/ml, and wherein the FBS is a nutritional supplement, and wherein the N2 is a cocktail of the growth factor, and wherein the N2 comprises an insulin at a concentration of 5µg/ml, a progesterone at a concentration of 20nM, a putrescine at a concentration of 100 µM, a selenium at a concentration of 30nM, a B27 at a concentration of 2% v/v and a bFGF at a concentration of 20 ng/ml, and wherein the selenium is added for growth and maintenance of astroglial cells in vitro, and wherein the B27 is a growth supplement for astroglial cells in vitro, and wherein the bFGF is a cell differentiating agent, and wherein the bFGF induces cell differentiation of the hPNF-MSCs into astroglial progenitor cells, and wherein the T4 induces the differentiation of hPNF-MSCs into astroglial cells, and wherein the bFGF targets Wnt pathway;
replenishing the astroglial induction medium after every 3-4 days;
fixing the cells with a 4% PFA for characterization; and
performing induction procedure on a monolayer culture of the cells.

15. The method according to claim 14, wherein the isolated human perinephric fat derived stem/stromal cells (hPNF-MSCs) show expression of GFAP (53.31 ± 14.11%) astroglial marker, and wherein the astroglial marker expression confirms the differentiation of the hPNF-MSCs into neural cells.

16. The method according to claim 1, wherein the step of differentiating isolated human perinephric fat derived stem/stromal cells (hPNF-MSCs) into renal cells comprises the steps of:
culturing the hPNF-MSC on autoclaved coverslips in 35mm culture dishes comprising a renal epithelial growth medium;
incubating the culture dishes at 37°C in a CO₂ incubator for 17 days for identifying the differentiation of hPNF-MSC to metanephric mesenchyme, and wherein the CO₂ is at a concentration of 5%;
incubating the culture dishes at 37°C in a CO₂ incubator for 22 days for identifying the differentiation of hPNF-MSC to podocyte and proximal convulated tubule, and wherein the CO₂ is at a concentration of 5%;
replenishing the culture medium after every 48 hours;
wherein the renal epithelium growth medium comprises a BMP2 at a concentration of 10 nm/ml, a retinoic acid at a concentration of 0.1µM/ml and an Activin A at a concentration of 10ng/ml, and wherein the BMP2, the retinoic acid, and the Activin A are the growth factors which induce differentiation in the hPNF-MSC to form renal cells, and wherein the Activin A induces a mesoderm specification, and wherein the retinoic acid induces the mesoderm patterning, and wherein the retinoic acid targets retinoid receptors RAR, and wherein the BMP2 induces nephrogenesis, and wherein the BMP2 is expressed in a metanephric mesenchyme, and wherein the BMP2 induces epithelialization of a metanephric mesenchyme;
performing syringe filtration of the renal epithelium differentiation medium using a 0.22 micron filter;
incubating the hPNF-MSCs at a temperature of 37°C in a CO₂ incubator for 17 days to identify the differentiation of hPNF-MSCs into metanephric mesenchyme cells, and wherein the concentration of CO₂ is 5%;
incubating the hPNF-MSCs at a temperature of 37°C in an incubator for 22 days for a podocyte and a proximal convulated tubule differentiation, wherein the incubator has CO₂ at a concentration of 5%; and
replenishing the renal epithelium differentiation medium after every 48 hours.

17. The method according to claim 16, wherein the Wnt pathway is targeted for differentiating the human perinephric fat derived stem/stromal cells (hPNF-MSCs) into renal cell, and wherein the Wnt pathway proteins expressed during a renal development are a Wnt 2b and a Wnt 9, and wherein the BMP2, the Activin A and the Retinoic acid growth factors activate the Wnt signaling pathway enabling differentiation of hPNF-MSCs to renal progenitor cells.

18. The method according to claim 1, wherein the step of differentiating isolated human perinephric fat derived stem/stromal cells (hPNF-MSCs) into islet cells comprises the steps of:
seeding the human perinephric mesenchymal stem cells (hPNF-MSCs) from a passage 3 (P3) in three non-adherent cell culture dishes/plates;
labeling two cell culture dishes/plates as cell differentiation culture dish/plate, and wherein the hPNF-MSCs are differentiated into pancreatic progenitor cell clusters in the cell differentiation culture dish/plates;
labeling one cell culture dish/plate as control dish/plate, and wherein the control dish/plate is an un-induced culture dish/plate;
supplementing the control cell culture dish/plate (un-induced culture dish/plate) with a cell culture medium, and wherein the cell culture medium comprises Dulbecco's Modified Eagle's Medium-Knock Out (DMEM-KO);
supplementing the two cell differentiation plates/dishes with a culture medium and wherein the cell culture medium comprises Dulbecco's Modified Eagle's Medium-Knock Out (DMEM-KO), a bovine serum albumin (BSA) in a concentration of 1.5%v/v, insulin-transferrin-selenium (ITS) in a concentration of 1X;
incubating the two cell differentiation culture dish/plate and one control cell culture dish/plate at 37°C for four days in a CO₂ incubator, and wherein the CO₂ incubator is filled with CO₂ at a concentration of 5%;
collecting a plurality of floating cells in a culture medium in a centrifuge tube after four days;
allowing the cells to settle down in the centrifuge tube for 15-20 minutes, and wherein cells settle down to form a pellet and the culture medium forms a supernatant and wherein the cell pellet is collected;
decanting the supernatant;
re-seeding the cells from the cell pellet in a day 4 medium and wherein the 4-days old medium comprises a Dulbecco's Modified Eagle's Medium (DMEM-KO), bovine serum albumin (BSA) in a concentration of 1.5%, insulin-transferrin-selenium (ITS) in a concentration of 1X, a bovine serum albumin (BSA) and Taurine at a concentration of 0.3 mM;
incubating the cells at preset temperature in the CO₂ incubator for further three days, and wherein the preset temperature is 37°C and wherein the cells are incubated for a total period of seven days, and wherein the CO₂ incubator is filled with CO₂ at a concentration of 5%;
feeding the cells with a fresh day 4 medium on day 7;
incubating the cells at preset temperature in a CO₂ incubator for further three days, and wherein the preset temperature is 37°C and wherein the cells are incubated for a total period of ten days, and wherein the CO₂ incubator is filled with CO₂ at a concentration of 5%;
collecting floating cell clusters with culture medium in a sterile centrifuge tube after 10 days;
allowing the cells to settle for 15-20 minutes at a room temperature, and wherein the cells settle down to form the pellet, and wherein the culture medium forms the supernatant;
decanting the supernatant;
wherein the cell pellet comprises cell clusters, and wherein the cell clusters are re-seeded in a medium comprising a DMEM-KO, BSA at a concentration of 1.5% v/v, ITS at a concentration of 1X, 100nM nicotine amide at a concentration of 100 nM, taurine at a concentration of 3nM and a glucagon like peptide 1 (GLP 1) at a concentration of 100 nM;
subjecting the cells for a dithiozone (DTZ) staining analysis and an immunofluorescence analysis; and
wherein the cell clusters are islet cell clusters.

19. The method according to claim 18, wherein the islet cell clusters are **characterized by** dithiozone (DTZ) staining, and wherein the islet cell clusters are positive for DTZ staining, and wherein the DTZ is a zinc chelating agent that selectively stains pancreatic beta cells.

20. The method according to claim 18, wherein the islet cell clusters are **characterized by** an immunofluorescence staining for c-peptide, and wherein the cell clusters show expression of c-peptide, and wherein the expression of c-peptide confirms the generation of functional islet cell clusters.

## Patentansprüche

1. Verfahren zur Isolierung und Charakterisierung von Stamm-/Stromazellen aus menschlichem perinephrischem Fett (hPNF-MSCs) zur Vermehrung, Regeneration und Differenzierung in Astrogliazellen, Nierenzellen, Nervenzellen und Inselzellen, wobei das Verfahren die folgenden Schritte umfasst:
in einem perinephrischen Fettgewebe, das von einem Freiwilligen oder einem Nierenpatienten mit vorheriger Einwilligung nach Aufklärung entnommen wurde;
Verarbeiten des perinephrischen Fettgewebes in einer sterilen Umgebung, wobei die sterile Umgebung einen mit Current Good Manufacturing Practices (cGMP) kompatiblen Reinraum mit einem Biosicherheitsschrank umfasst;
Isolieren der mesenchymalen Stamm-/Stromazellen aus den perinephrischen Fettgeweben unter Verwendung mehrerer Verfahren, wobei die mehreren Verfahren ein mechanisches Verfahren und ein enzymatisches Verfahren sind;
Waschen der Gewebeprobe in einer 1X Dulbecco Phosphat-gepufferten Kochsalzlösung (DPBS), die Antibiotika in einer Konzentration von 2X enthält, wobei die Gewebeprobe mehrere Male gewaschen wird und wobei das Antibiotikum Pen-Strep ist;
mechanische Zerkleinerung der Gewebeprobe in mehrere kleine Stücke mit einer sterilen Skalpellklinge;
Behandeln der Gewebeprobe in einem Dulbecco-modifizierten Eagles-Medium (DMEM-KO), das ein Enzym und das Pen-Strep umfasst, wobei die Gewebeprobe 60 Minuten bei 37 °C auf einer Laborwippe behandelt wird und wobei das Enzym eine Kollagenase vom Typ I ist und wobei die Kollagenase vom Typ I mit einer vorbestimmten Konzentration gemischt ist und wobei die vorbestimmte Konzentration der Kollagenase vom Typ I 0,2 Vol.-% beträgt;
Zugabe des DMEM-KO-Mediums mit einem fötalen Rinderserum (FBS), um eine enzymatische Wirkung von Kollagenase zu neutralisieren, wobei das FBS in einer vorbestimmten Konzentration vorliegt und wobei die vorbestimmte Konzentration von FBS 5 Vol.-% beträgt, und wobei DMEM-KO-Medium mit dem FBS die enzymatische Wirkung von Kollagenase neutralisiert;
Erhalten eines Homogenats, das mesenchymale Stamm-/Stromazellen, aus den perinephrischen Fettgeweben umfasst;
Filtrieren des Homogenats, das mesenchymale Stamm-/Stromazellen umfasst, aus den perinephrischen Fettgeweben durch ein Zellsieb, um ein Filtrat zu erhalten, wobei das Zellsieb eine Porengröße von 40 µm aufweist;
Sammeln des Filtrats, das mesenchymale Stamm-/Stromazellen umfasst, aus den perinephrischen Fettgeweben;
2 Minuten lang Zentrifugieren des Filtrats, das mesenchymale Stamm-/Stromazellen umfasst, bei 1500 U/min;
Sammeln eines Zellpellets, das mesenchymale Stamm-/Stromazellen umfasst, nach Abschluss des Zentrifugationsprozesses;
Resuspendieren des Pellets in einem Kulturmedium, wobei das Kulturmedium ein DMEM-KO, ein fötales Rinderserum (FBS) in einer Konzentration von 10%, ein L-Glutamat in einer Konzentration von 1X, ein Pen-Strep und einen basischen Fibroblasten-Wachstumsfaktor (bFGF) in einer vorbestimmten Konzentration umfasst und wobei die vorbestimmte Konzentration des bFGF 1 ng/ml beträgt;
Erhalten einer Zellsuspension von mesenchymalen Stamm-/Stromazellen;
Impfen der Suspension von mesenchymalen Stammzellen/Stromazellen in einem Zellstapel, wobei der Zellstapel 200 ml Zellkulturmedium umfasst;
Schütteln des Zellstapels zur gleichmäßigen Verteilung von Zellen und Kulturmedium, wobei die Zellstapel in einem befeuchteten Inkubator bei einer Temperatur von 37 °C bebrütet werden und wobei der befeuchtete Inkubator aus einem Kohlendioxid (CO₂) besteht und wobei die Konzentration CO₂ 5 Vol.-% beträgt;
Auffüllen des Kulturmediums nach 48 Stunden, wobei das Kulturmedium aufgefüllt wird, um nicht anhaftende Zellen zu entfernen und wobei es anhaftenden fibroblastenähnlichen Zellpopulationen erlaubt wird, sich im Kulturmedium zu vermehren, bis eine Zellkonfluenz von 80% zum Ernten erreicht ist;
Auffüllen des Kulturmediums 2-3 mal pro Woche;
Trypsinieren und Ernten der hPNF-MSCs aus den Zellstapeln, nachdem die Zellen eine Konfluenz von 80% erreicht haben;
Zählen der mesenchymalen Stamm-/Stromazellpopulation;
Kryokonservierung der hPNF-MSCs in einem Kryovial, wobei das Kryovial mit Proben-ID, Zellnummer und Isolationsdatum markiert ist und wobei das Kryovial als Passage 0 (P0) markiert ist und die hPNF-MSCs einer Expansion unterzogen werden; und
wobei die hPNF-MSCs einer Charakterisierung unterzogen werden und wobei die isolierten und charakterisierten hPNF-MSCs einer Differenzierung in Nervenzellenunterzogen werden und wobei die isolierten und charakterisierten hPNF-MSCs einer Differenzierung in astrogliale Zellen unterzogen werden und wobei die isolierten und charakterisierten hPNF-MSCs einer Differenzierung in astrogliale Zellen unterzogen werden, wobei die hPNF-MSCs einer Differenzierung in Nierenzellen unterzogen werden und wobei die isolierten und charakterisierten hPNF-MSCs einer Differenzierung in Inselzellen unterzogen werden.

2. Verfahren gemäß Anspruch 1, wobei der Schritt des Kryokonservierens die Schritte umfasst:
Vorbereiten einer Gefriermischung, wobei die Gefriermischung fötales Rinderserum in einer Konzentration von 90 Vol.-%, Dimethylsulfoxid 10 Vol.-% und mesenchymale Stammzellen (MSC) umfasst;
Abkühlen der Mischung auf eine Temperatur von 4 °C;
10 min lang Zentrifugieren der Zellsuspension bei 1400 U/min bei Raumtemperatur;
Erhalten eines Zellpellets und Verwerfen des Überstands;
tropfenweise Zugabe der Gefriermischung zum Zellpellet;
Mischen der Gefriermischung und des Zellpellets;
Übertragen der Mischung in ein Kryovial;
Bebrüten des Kryovials bei einer Temperatur von -80 °C; und
Überführen des Kryovials in einen Flüssigstickstoff-Kryotank.

3. Verfahren gemäß Anspruch 1, wobei die aus menschlichem perinephrischen Fett stammenden Stamm/Stromazellen (hPNF-MSCs) einer Charakterisierung unterzogen werden, um zu bestätigen, dass sie mesenchymale Stamm-/Stromazellen sind und wobei die aus isolierten, von menschlichem perinephrischen Fett stammenden Stamm/Stromazellen (hPNF-MSCs) für eine Saatdichteanalyse; eine morphologische Analyse, eine wachstumskinetische Untersuchung, eine immunphänotypische Analyse, eine Differenzierungspotentialanalyse, eine Karyotypanalyse und eine Sekretomprofilanalyse analysiert werden.

4. Verfahren gemäß Anspruch 3, wobei die isolierten, aus menschlichem perinephrischen Fett stammenden Stamm-/Stromazellen (hPNF-MSCs) bei der Keimdichteanalyse mit unterschiedlichen Dichten gesät werden, und wobei die Zellen mit einer geringen Dichte und einer hohen Dichte gesät werden, und wobei die mit einer geringen Dichte gesäten Zellen 1000 Zellen/cm² aufweisen und wobei die mit einer hohen Dichte gesäten Zellen 2500 Zellen/cm² aufweisen und wobei die mit einer hohen Dichte gesäten Zellen eine Wachstumskinetik und eine Zellmorphologie aufrechterhalten und wobei die Zellkultur mit der hohen Dichte eine Zellalterung bei Passage 10 (P10) aufweisen.

5. Verfahren gemäß Anspruch 3, wobei die Morphologie der isolierten, aus menschlichem perinephrischen Fett stammenden Stamm-/Stromazellen (hPNF-MSCs) ähnlich einer Morphologie eines Fibroblasten ist, und wobei die hPNF-MSCs die Fibroblastenmorphologie bis zu einer Passage 7 aufweisen (P7).

6. Verfahren gemäß Anspruch 3, wobei eine Zellpopulationsverdopplungszeit für die isolierten aus menschlichem perinephrischen Fett stammenden Stamm-/Stromazellen (hPNF-MSCs) von Passage 1 (P1) bis Passage 14 (P14) bestimmt wird und wobei die Populationsverdopplungszeit der Zellen von 14,38 ± 1,29 Stunden bei Passage 2 (P2) auf 235,85 ± 0,65 Stunden bei Passage 9 (P9) steigt.

7. Verfahren gemäß Anspruch 3, wobei die isolierten, aus menschlichem perinephrischen Fett stammenden Stamm-/Stromazellen (hPNF-MSCs) auf Zelloberflächenantigenmarker analysiert/charakterisiert werden und wobei die hPNF-MSCs an einer Passage 0 (P0) ein CD44-Antigen und ein CD90-Antigen präsentieren, und wobei die Zellen ein HLA-DR-Antigen, ein CD 34-Antigen und ein CD 45-Antigen in weniger als 4% der Zellpopulation an einer Passage 5 (P5) präsentieren und wobei 90% der Zellpopulation ein CD44-Antigen, ein CD73-Antigen, ein CD90-Antigen, ein CD105-Antigen und ein CD166-Antigen an der Passage 5 (P5) präsentieren und wobei die Zellen an einer Passage 9 (P9) und einer Passage 15 (P15) eine geringe Präsentation des Zelloberflächenantigens aufweisen.

8. Verfahren gemäß Anspruch 3, wobei die Sekretomprofilanalyse der isolierten, aus menschlichem perinephrischen Fett stammenden Stamm-/Stromazellen (hPNF-MSCs) die Analyse von Zytokinen, Chemokinen und Wachstumsfaktoren umfasst.

9. Verfahren gemäß Anspruch 8, wobei die analysierten Zytokine, ein entzündungsförderndes Zytokin und ein entzündungshemmendes Zytokin sind, wobei das entzündungsfördernde von den hPNF-MSCs abgesonderte Zytokin durch die IL-6, IL-9, IL-12, INF-a2 und IL-1α absondert und wobei die IL-6-Sekretion durch PNF-MSCs im Vergleich zu dem anderen entzündungsfördernden Zytokin hoch ist und wobei durch hPNF-MSCs abgesondertes entzündungshemmendes Zytokin ein IL-4, ein IL-10 und ein IL-1Ra ist und wobei die IL-13-Sekretion im Vergleich zu anderen entzündungshemmenden Zytokinen durch hPNF-MSCs hoch ist.

10. Verfahren gemäß Anspruch 8, wobei die hPNF-MSC eine Vielzahl von Chemokinen absondert, einschließlich eines MDC, eines Fractalkine, eines IP-10, eines MIP-1β, eines Eotaxins, eines PCP-1, eines MCP-3 und eines GRO.

11. Verfahren gemäß Anspruch 8, wobei die durch hPNF-MSC sekretierten Wachstumsfaktoren ein G-CSF, ein GM-CSF, ein VEGF und ein PDGF-aa sind und wobei die Sekretion von VEGF bei einer Konzentration von 2000 bis 4000 pg/ml liegt.

12. Verfahren gemäß Anspruch 1, wobei der Schritt des Differenzierens der isolierten, aus menschlichem perinephrischen Fett stammenden Stamm-/Stromazellen (hPNF-MSCs) in Nervenzellen die Schritte umfasst:
Erzeugen von Kugeln mit einem basischen Fibroblastenwachstumsfaktor (bFGF) in einer Konzentration von 1 ng/ml in einem DMEM-MO-Medium, und wobei das DMEM-KO-Medium ein fötales Rinderserum (FBS) in einer Konzentration von 1 Vol.-% umfasst;
24 Stunden lang bebrüten der erzeugten Kugeln in dem Medium;
Ersetzen des Wachstumsmediums durch ein Nerveneinleitungsmedium, wobei das Nerveneinleitungsmedium ein DMEM-F12-Kulturmedium umfasst und wobei das DMEM-F12-Medium fötales Rinderserum (FBS) in einer Konzentration von 1 Vol.-%, ein N2 mit einer Konzentration von 1 Vol.-%, ein B27 mit einer Konzentration von 2% und ein bFGF mit einer Konzentration von 20 ng/ml umfasst, wobei das FBS ein Nahrungsergänzungsmittel ist und wobei das N2 ein Cocktail des Wachstumsfaktors ist und wobei das N2 ein Insulin in einer Konzentration von 5 µg/ml, ein Transferrin in einer Konzentration von 100 µg/ml, ein Progesteron in einer Konzentration von 20 nM, ein Putrescin in einer Konzentration von 100 µM und Selen in einer Konzentration von 30 nM, ein B27 in einer Konzentration von 2 Vol.-% und bFGF in einer Konzentration von 20 ng/ml und wobei das Selen zum Wachstum und zur Aufrechterhaltung von Neuronen in vitro zugesetzt wird und wobei das B27 ein Wachstumszusatz für Neuronen in vitro ist, und wobei der bFGF ein zelldifferenzierendes Mittel ist und wobei der bFGF die Zelldifferenzierung der hPNF-MSCs in Nervenvorläuferzellen einleitet und wobei der bFGF auf den Wnt-Weg abzielt;
Auffüllen des Nerveneinleitungsmediums nach jeweils 3-4 Tagen;
Fixieren der Zellen mit einer Konzentration von 4 Vol.-% zur Charakterisierung; und
Durchführen eines Einleitungsverfahrens an einer Monoschichtkultur der Zellen.

13. Verfahren gemäß Anspruch 12, wobei die isolierten, aus menschlichem perinephrischen Fett stammenden Stamm-/Stromazellen (hPNF-MSCs) eine positive Präsentation für neurale Marker aufweisen, einschließlich Nestin (78,12 ± 16%), NSE (75,68 ± 6,18%), β III-Tubulin (85,56 ± 10,91%) und wobei die Präsentation des neuralen Markers die Differenzierung der hPNF-MSCs in Nervenzellen bestätigt.

14. Verfahren gemäß Anspruch 1, wobei der Schritt des Differenzierens der isolierten, aus menschlichem perinephrischen Fett stammenden Stamm-/Stromazellen (hPNF-MSCs) in Astrogliazellen die Schritte umfasst:
Erzeugen von Kugeln mit einem basischen Fibroblastenwachstumsfaktor (bFGF) bei einer Konzentration von 1 nm/ml in einem DMEM-KO-Medium, wobei das DMEM-KO-Medium mit einem fötalen Rinderserum (FBS) bei einer Konzentration von 1 Vol.-% ergänzt wird;
24 Stunden lang Bebrüten der erzeugten Kugeln in dem Medium;
Ersetzen des Wachstumsmediums durch ein astrogliales Einleitungsmedium und ein L-Thyroxin, wobei das astrogliale Einleitungsmedium ein FBS in einer Konzentration von 1 Vol.-%, ein N2 in einer Konzentration von 1 Vol.-% und ein T4 in einer Konzentration von 30 ng/ml umfasst und wobei das FBS ein Nahrungsergänzungsmittel ist und wobei das N2 ein Cocktail des Wachstumsfaktors ist und wobei das N2 ein Insulin in einer Konzentration von 5 µg/ml, ein Progesteron in einer Konzentration von 20 nM ein Putrescin in einer Konzentration von 100 µM, Selen in einer Konzentration von 30 nM, ein B27 in einer Konzentration von 2 Vol.-% und ein bFGF in einer Konzentration von 20 ng/ml umfasst, und wobei das Selen für das Wachstum und die Aufrechterhaltung von Astrogliazellen in vitro zugesetzt wird, wobei das B27 ein Wachstumszusatz für Astrogliazellen in vitro ist und wobei das bFGF ein Zelldifferenzierungsmittel ist und wobei das bFGF die Zelldifferenzierung der hPNF-MSCs in astrogliale Vorläuferzellen einleitet und wobei der T4 die Differenzierung von hPNF-MSCs in Astrogliazellen einleitet und wobei der bFGF den Wnt-Weg abzielt;
Nachfüllen des astroglialen Einleitungsmediums nach jeweils 3-4 Tagen;
Fixieren der Zellen mit 4% PFA zur Charakterisierung; und
Durchführen eines Einleitungsverfahrens an einer Monoschichtkultur der Zellen.

15. Verfahren nach Anspruch 14, wobei die isolierten aus menschlichem perinephrischem Fett stammenden Stamm-/Stromazellen (hPNF-MSCs) die Präsentation von GFAP (53,31 ± 14,11%) Astroglial-Marker zeigen und wobei die Präsentation des Astroglialmarkers die Differenzierung der hPNF-MSCs in Nervenzellen bestätigt.

16. Verfahren nach Anspruch 1, wobei der Schritt des Differenzierens der isolierten, aus menschlichem perinephrischen Fett stammenden Stamm-/Stromazellen (hPNF-MSCs) in Nierenzellen die Schritte umfasst:
Kultivieren des hPNF-MSC auf autoklavierten Deckgläsern in 35-mm-Kulturschalen, die ein Nierenepithelwachstumsmedium enthalten;
Bebrüten der Kulturschalen bei 37 °C in einem CO₂-Inkubator 17 Tage lang zur Identifizierung der Differenzierung von hPNF-MSC zu metanephrischem Mesenchym, wobei das CO₂ in einer Konzentration von 5% vorliegt;
Bebrüten der Kulturschalen bei 37 °C in einem CO₂-Inkubator 22 Tage lang zur Identifizierung der Differenzierung von hPNF-MSC zu Podozyten und proximalem konvulierten Tubulus, wobei das CO₂ in einer Konzentration von 5% vorliegt;
Nachfüllen des Kulturmediums nach jeweils 48 Stunden;
wobei das Nierenepithel-Wachstumsmedium ein BMP2 in einer Konzentration von 10 nm/ml, eine Retinsäure in einer Konzentration von 0,1µM/ml und ein Activin A in einer Konzentration von 10 ng/ml umfasst und wobei das BMP2 die Retinsäure und das Activin A die Wachstumsfaktoren sind, die eine Differenzierung in der hPNF-MSC induzieren, um Nierenzellen zu bilden, und wobei das Activin A eine Mesodermspezifikation einleitet und wobei die Retinsäure die Mesodermmusterbildung einleitet, wobei die Retinsäure auf Retinoidrezeptoren RAR abzielt, wobei das BMP2 die Nephrogenese einleitet und wobei das BMP2 in einem metanephrischen Mesenchym präsentiert wird und wobei das BMP2 die Epithelisierung eines metanephrischen Mesenchyms einleitet;
Durchführen einer Spritzenfiltration des Nierenepitheldifferenzierungsmediums unter Verwendung eines 0,22-Mikrometer-Filters;
Bebrüten der hPNF-MSCs bei einer Temperatur von 37 °C in einem CO₂-Inkubator 17 Tage lang, um die Differenzierung von hPNF-MSCs in metanephrische Mesenchymzellen zu identifizieren, wobei die CO₂-Konzentration 5% beträgt;
Bebrüten der hPNF-MSCs bei einer Temperatur von 37 °C in einem Inkubator 22 Tage lang für eine Podozyten- und eine proximale konvulierte Tubulusdifferenzierung, wobei der Inkubator CO₂ in einer Konzentration von 5% aufweist; und
Auffüllen des Nierenepitheldifferenzierungsmediums nach jeweils 48 Stunden.

17. Verfahren gemäß Anspruch 16, wobei der Wnt-Weg darauf abzielt, die aus menschlichem perinephrischem Fett stammenden Stamm-/Stromazellen (hPNF-MSCs) in Nierenzellen zu differenzieren und wobei die Wnt-Weg-Proteine, die während einer Nierenentwicklung präsentiert werden, ein Wnt 2b, ein Wnt 9 sind und wobei der BMP2-, der Activin-A- und der Retinsäurewachstumsfaktor den Wnt-Signalweg aktivieren, der die Differenzierung von hPNF-MSCs zu Nierenvorläuferzellen ermöglicht.

18. Verfahren gemäß Anspruch 1, wobei der Schritt des Differenzierens der isolierten, aus menschlichem perinephrischen Fett stammenden Stamm-/Stromazellen (hPNF-MSCs) in Inselzellen die Schritte umfasst:
Impfen der menschlichen perinephrischen mesenchymalen Stammzellen (hPNF-MSCs) aus einer Passage 3 (P3) in drei unverbundenen Zellkulturschalen/-platten;
Markieren von zwei Zellkulturschalen/-platten als Zelldifferenzierungskulturschale/-platte, wobei die hPNF-MSCs in Bauchspeicheldrüsenvorläuferzellhaufen in den Zelldifferenzierungskulturschalen/-platten differenziert werden;
Markieren einer Zellkulturschale/-platte als Kontrollschale/-platte, wobei die Kontrollschale/-platte eine nicht eingeleitete Kulturschale/-platte ist;
Ergänzen der Kontrollzellkulturschale/-platte (nicht eingeleitete Kulturschale/- platte) mit einem Zellkulturmedium, wobei das Zellkulturmedium Dulbeccos modifiziertes Eagle-Medium-Knock-Out (DMEM-KO) umfasst;
Ergänzen der zwei Zelldifferenzierungsplatten/-schalen mit einem Kulturmedium, wobei das Zellkulturmedium Dulbeccos modifiziertes Eagle-Medium-Knock-Out (DMEM-KO), ein Rinderserumalbumin (BSA) in einer Konzentration von 1,5 Vol.-% Insulin-Transferrin-Selen (ITS) in einer Konzentration von 1X umfasst;
Bebrüten der beiden Zelldifferenzierungskulturschalen/-platte und einer Kontrollzellkulturschale/-platte bei 37 °C vier Tage lang in einem CO₂-Inkubator, wobei der CO₂-Inkubator mit CO₂ in einer Konzentration von 5% gefüllt ist;
Sammeln einer Vielzahl von schwimmenden Zellen in einem Kulturmedium in einem Zentrifugenröhrchen nach vier Tagen;
Absetzenlassen der Zellen in dem Zentrifugenröhrchen 15 bis 20 Minuten lang, wobei sich die Zellen absetzen, um ein Pellet zu bilden und das Kulturmedium einen Überstand bildet und wobei das Zellpellet gesammelt wird;
Abgießen des Überstands;
Erneutes Impfen der Zellen aus dem Zellpellet in ein Tag-4-Medium, wobei das 4 Tage alte Medium ein Dulbecco-modifiziertes Eagle-Medium (DMEM-KO), Rinderserumalbumin (BSA) in einer Konzentration von 1,5%, Insulin-Transferrin-Selen (ITS) in einer Konzentration von 1X, ein Rinderserumalbumin (BSA) und Taurin in einer Konzentration von 0,3 mM umfasst;
Bebrüten der Zellen bei voreingestellter Temperatur in dem CO₂-Inkubator weitere drei Tage lang, wobei die voreingestellte Temperatur 37 °C beträgt und wobei die Zellen für einen Gesamtzeitraum von sieben Tagen bebrütet werden und wobei der CO₂-Inkubator mit CO₂ in einer Konzentration von 5% gefüllt ist;
Füttern der Zellen mit einem frischen Tag-4-Medium an Tag 7;
Bebrüten der Zellen bei voreingestellter Temperatur in einem CO₂-Inkubator weitere drei Tage lang, wobei die voreingestellte Temperatur 37 °C beträgt und wobei die Zellen für einen Gesamtzeitraum von zehn Tagen bebrütet werden und wobei der CO₂-Inkubator mit CO₂ in einer Konzentration von 5% gefüllt ist,
Sammeln von schwimmenden Zellhaufen mit Kulturmedium in einem sterilen Zentrifugenröhrchen nach 10 Tagen;
Absetzenlassen der Zellen für 15 bis 20 Minuten bei Raumtemperatur, wobei sich die Zellen absetzen, um das Pellet zu bilden und wobei das Kulturmedium den Überstand bildet;
Abgießen des Überstands;
wobei das Zellpellet Zellhaufen umfasst und wobei die Zellhaufen in ein Medium, das ein DMEM-KO, BSA in einer Konzentration von 1,5 Vol .-%, ITS in einer Konzentration von 1X, 100 nM, Nikotinamid in einer Konzentration von 100 nM, Taurin in einer Konzentration von 3 nM und ein glucagonartiges Peptid 1 (GLP 1) in einer Konzentration von 100 nM umfasst, erneut geimpft werden;
Unterziehen der Zellen einer Dithiozone (DTZ)-Färbungsanalyse und einer Immunfluoreszenzanalyse; und
wobei die Zellhaufen Inselzellhaufen sind.

19. Verfahren gemäß Anspruch 18, wobei die Inselzellhaufen durch Dithiozone (DTZ)-Färbung gekennzeichnet sind und wobei die Inselzellhaufen für DTZ-Färbung positiv sind und wobei das DTZ ein Zink-Chelatbildner ist, der selektiv Bauchspeicheldrüsen-Beta-Zellen färbt.

20. Verfahren nach Anspruch 18, wobei die Inselzellhaufen durch eine Immunfluoreszenzfärbung für c-Peptid gekennzeichnet sind und wobei die Zellhaufen die Präsentation von c-Peptid zeigen und wobei die Präsentation von c-Peptid die Erzeugung von funktionellen Inselzellhaufen bestätigt.

## Revendications

1. Procédé pour isoler et caractériser les cellules souches/stromales humaines dérivées du tissu adipeux périnéphrique (hPNF-MSC), pour la propagation, la régénération et la différentiation en cellules astrogliales, cellules rénales, cellules neuronales et îlots, le procédé comprenant les étapes suivantes :
dans un tissu adipeux périnéphrique prélevé sur un volontaire ou un patient présentant une pathologie du rein ayant donné son consentement préalable ;
traitement du tissu adipeux périnéphrique dans un environnement stérile, l'environnement stérile comprenant une salle blanche conforme aux bonnes pratiques de fabrication actuelles (cGMP, Current Good Manufacturing Practices en anglais) avec une enceinte de sécurité biologique ;
isolement de cellules souches/stromales mésenchymateuses à partir de tissus adipeux périnéphriques à l'aide d'une pluralité de procédés, la pluralité de procédés étant un procédé mécanique et un procédé enzymatique ;
lavage de l'échantillon de tissu dans un 1X la solution de tampon phosphate salin de Dulbecco (DPBS, Dulbecco's Phosphate Buffered Saline en anglais) comprenant des antibiotiques à une concentration de 2X la concentration, l'échantillon de tissu étant lavé une pluralité de fois et l'antibiotique étant le Pen-Strep ;
hachage de l'échantillon de tissu mécaniquement en une pluralité de petits morceaux avec une lame de scalpel stérile ;
traitement de l'échantillon de tissu dans un milieu Eagle modifié de Dulbecco (DMEM-KO, Dulbecco's Modified Eagles Medium en anglais) comprenant une enzyme et le Pen-Strep, l'échantillon de tissu étant traité pendant 60 minutes à 37 °C sur un culbuteur de laboratoire et l'enzyme étant une Collagénase de type I et la Collagénase de type I étant mélangée à une concentration prédéterminée et la concentration prédéterminée de la Collagénase de type I étant de 0,2 %vol;
ajout du milieu DMEM-KO avec un sérum fœtal bovin (FBS, Fetal Bovine Serum en anglais) pour neutraliser une action enzymatique de la Collagénase, le sérum FBS étant présent dans une concentration prédéterminée et la concentration prédéterminée de sérum FBS étant de 5 %vol et le milieu DMEM-KO avec le sérum FBS neutralisant l'action enzymatique de la Collagénase ;
obtention d'un broyat comprenant des cellules souches/stromales mésenchymateuses à partir de tissus adipeux périnéphriques ;
filtrage du broyat comprenant des cellules souches/stromales mésenchymateuses à partir de tissus adipeux périnéphriques à travers un conteneur de cellule pour obtenir un filtrat, le conteneur de cellule ayant une taille de pore de 40 µm ;
prélèvement du filtrat comprenant des cellules souches/stromales mésenchymateuses à partir de tissus adipeux périnéphriques ;
centrifugeage du filtrat comprenant des cellules souches/stromales mésenchymateuses à 1500 tr/min pendant 2 minutes ;
prélèvement d'un culot de cellules comprenant des cellules souches/stromales mésenchymateuses à la fin du processus de centrifugeage ;
nouvelle mise en suspension du culot dans un milieu de culture, le milieu de culture comprenant un milieu DMEM-KO, un sérum fœtal bovin (FBS) à une concentration de 10 %, un L-glutamate à une concentration de 1X, un Pen-Strep et un facteur de croissance des fibroblastes basique (bFGF, basic Fibroblast Growth Factor en anglais) à une concentration prédéterminée, la concentration prédéterminée du facteur bFGF étant de 1 ng/ml ;
obtention d'une suspension cellulaire de cellules souches/stromales mésenchymateuses ;
ensemencement de la suspension de cellules souches/stromales mésenchymateuses dans un empilement de cellules, l'empilement de cellules comprenant 200 ml de milieu de culture de cellules ;
basculement de l'empilement de cellules pour une répartition régulière des cellules et du milieu de culture, les empilements de cellules étant incubés dans un incubateur humidifié à une température de 37 °C, l'incubateur humidifié consistant en un dioxyde de carbone (CO₂) et la concentration de CO₂ étant de 5 %vol;
remplissage du milieu de culture après 48 heures, le milieu de culture étant rempli pour éliminer les cellules non adhérentes, les populations de cellules analogues à des fibroblastes adhérents pouvant ainsi croître dans le milieu de culture jusqu'à obtention d'une confluence cellulaire de 80 % à prélever ; le remplissage du milieu de culture s'effectue 2-3 fois par semaine ;
trypsinisation et le prélèvement des cellules hPNF-MSC à partir des empilements de cellules, après obtention d'une confluence de 80 % ;
comptage de la population de cellules souches/stromales mésenchymateuses ;
cryoconservation des cellules hPNF-MSC dans un système Cryovial, le système Cryovial étant étiqueté avec l'identifiant de l'échantillon, le nombre de cellules, la date d'isolement, le système Cryovial étant étiqueté comme passage 0 (P₀) et soumettant les cellules hPNF-MSC à expansion ; et
dans lequel les cellules hPNF-MSC sont soumises à caractérisation et dans lequel les cellules hPNF-MSC isolées et caractérisées sont soumises à différentiation dans des cellules neuronales et dans lequel les cellules hPNF-MSC isolées et caractérisées sont soumises à différentiation dans des cellules astrogliales et dans lequel les cellules hPNF-MSC isolées et caractérisées sont soumises à différentiation dans des cellules rénales ; et
dans lequel les cellules hPNF-MSC isolées et caractérisées sont soumises à différentiation dans des îlots.

2. Procédé selon la revendication 1, dans lequel l'étape de cryoconservation comprend les étapes suivantes :
préparation d'un mélange congelé, le mélange congelé comprenant du sérum fœtal bovin à une concentration de 90 %vol du diméthylsulfoxyde 10 %vol et des cellules souches mésenchymateuses (MSC) ;
refroidissement du mélange à une température de 4 °C ;
centrifugeage de la suspension cellulaire à 1400 tr/min pendant 10 min à température ambiante ;
obtention d'un culot de cellules et élimination du surnageant ;
ajout au compte-gouttes du mélange congelé au culot de cellules ;
mélange du mélange congelé et du culot de cellules ;
transfert du mélange dans un système Cryovial ;
incubation du système Cryovial à une température de -80 °C ; et
transfert du système Cryovial dans un cryoréservoir d'azote liquide.

3. Procédé selon la revendication 1, dans lequel les cellules souches/stromales humaines dérivées du tissu adipeux périnéphrique (hPNF-MSC) isolées sont soumises à caractérisation pour confirmer que les cellules sont des cellules souches/stromales mésenchymateuses et dans lequel les cellules souches/stromales humaines dérivées du tissu adipeux périnéphrique (hPNF-MSC) isolées sont analysées pour une analyse de densité d'ensemencement, une analyse morphologique, une étude cinétique de croissance, une analyse immunophénotypique, une analyse de potentiel de différentiation, une analyse de caryotype et une analyse de profil de sécrétome.

4. Procédé selon la revendication 3, dans lequel les cellules souches/stromales humaines dérivées du tissu adipeux périnéphrique (hPNF-MSC) isolées sont ensemencées à différentes densités dans l'analyse de densité d'ensemencement et dans lequel les cellules sont ensemencées à une faible densité et à une forte densité, et dans lequel les cellules ensemencées à faible densité ont 1000 cellules/cm² et dans lequel les cellules ensemencées à forte densité ont 2500 cellules/ cm² et dans lequel les cellules ensemencées à forte densité conservent des cinétiques de croissance et une morphologie cellulaire, et dans lequel la culture cellulaire à forte densité a une sénescence cellulaire au passage 10 (P10).

5. Procédé selon la revendication 3, dans lequel la morphologie des cellules souches/stromales humaines dérivées du tissu adipeux périnéphrique (hPNF-MSC) isolées est similaire à la morphologie d'un fibroblaste et dans lequel les cellules hPNF-MSC ont une morphologie de fibroblaste jusqu'à un passage 7 (P7).

6. Procédé selon la revendication 3, dans lequel une temps de doublement de population de cellule pour les cellules souches/stromales humaines dérivées du tissu adipeux périnéphrique (hPNF-MSC) isolées est déterminé du passage 1 (P1) au passage 14 (P14) et dans lequel le temps de doublement de population des cellules augmente de 14,38±1,29 heures au passage 2 (P2) à 235,85±0,65 heure au passage 9 (P9).

7. Procédé selon la revendication 3, dans lequel les cellules souches/stromales humaines dérivées du tissu adipeux périnéphrique (hPNF-MSC) isolées sont analysées/caractérisées pour le marqueur d'antigène de surface de cellule et dans lequel les cellules hPNF-MSC expriment un antigène CD44 et un antigène CD90 à un passage 0 (P0) et dans lequel les cellules expriment un antigène HLA-DR, un antigène CD 34 et un antigène CD 45 dans moins de 4 % de la population de cellules à un passage 5 (P5) et dans lequel 90 % de la population de cellules expriment un antigène CD 44, un antigène CD 73, un antigène CD 90, un antigène CD 105 et un antigène CD 166 au passage 5 (P5) et dans lequel les cellules ont une faible expression de l'antigène de surface de cellule à un passage 9 (P9) et à un passage 15 (P15).

8. Procédé selon la revendication 3, dans lequel l'analyse de profil du sécrétome des cellules souches/stromales humaines dérivées du tissu adipeux périnéphrique (hPNF-MSC) isolées comprend l'analyse des cytokines, des chimiokines et des facteurs de croissance.

9. Procédé selon la revendication 8, dans lequel les cytokines analysées sont une cytokine pro-inflammatoire et une cytokine anti-inflammatoire et dans lequel la cytokine pro-inflammatoire secrétée par les cellules hPNF-MSC secrète IL-6, IL-9, IL-12, INF-α2, et IL-1α et dans lequel la sécrétion de IL-6 est élevée dans les cellules PNF-MSC en comparaison à l'autre cytokine pro-inflammatoire et dans lequel la cytokine anti-inflammatoire secrétée par les cellules hPNF-MSC sont une IL-4, une IL-10 et une IL-1Ra et dans lequel la sécrétion de IL-13 est élevée dans les cellules hPNF-MSC en comparaison à l'autre cytokine anti-inflammatoire.

10. Procédé selon la revendication 8, dans lequel les cellules hPNF-MSC secrètent une pluralité de chimiokines comprenant une MDC, une Fractalkine, une IP-10, une MIP-1β, une eotaxine, une PCP-1, une MCP-3 et une GRO.

11. Procédé selon la revendication 8, dans lequel les facteurs de croissance secrétés par les cellules hPNF-MSC sont un facteur G-CSF, un facteur GM-CSF, un facteur VEGF et un facteur PDGF-aa et dans lequel la sécrétion de VEGF est à une concentration de 2000-4000 pg/ml.

12. Procédé selon la revendication 1, dans lequel l'étape de différentiation des cellules souches/stromales humaines dérivées du tissu adipeux périnéphrique (hPNF-MSC) isolées en cellules neuronales comprend les étapes suivantes :
génération de sphères avec un facteur de croissance des fibroblastes basique (bFGF) à une concentration de 1 ng/ml dans un milieu DMEM-MO, le milieu DMEM-KO comprenant un sérum fœtal bovin (FBS) à une concentration de 1 %vol;
incubation des sphères générées dans le milieu pendant 24 heures ;
remplacement du milieu de croissance par un milieu d'induction neutre, le milieu d'induction neutre comprenant un milieu de culture DMEM-F12, le milieu DMEM-F12 comprenant un sérum fœtal bovin (FBS) à une concentration de 1 %vol un N2 à une concentration de 1 %vol un B27 à une concentration de 2 % et un bFGF à une concentration de 20 ng/ml, le FBS étant un complément alimentaire, le N2 étant un cocktail de facteur de croissance et le N2 comprenant une insuline à une concentration de 5 µg/ml, une transferrine à une concentration de 100 µg/ml, une progestérone à une concentration de 20 nM, une putrescine à une concentration de 100 µm, un sélénium à une concentration de 30 nM, un B27 à une concentration de 2 %vol et un bFGF à une concentration de 20 ng/ml, le sélénium étant ajouté pour la croissance et le maintien des neurones in vitro, le B27 étant un supplément de croissance pour les neurones in vitro, le bFGF étant un agent de différentiation cellulaire, le bFGF induisant une différentiation cellulaire des cellules hPNF-MSC en cellules neuronales progénitrices et le bFGF ciblant une voie Wnt ;
remplissage du milieu d'induction neutre tous les 3-4 jours ;
fixation des cellules à une concentration de 4 %vol pour caractérisation ; et
mise en œuvre d'une procédure d'induction sur une culture monocouche des cellules.

13. Procédé selon la revendication 12, dans lequel les cellules souches/stromales humaines dérivées du tissu adipeux périnéphrique (hPNF-MSC) isolées ont une expression positive pour les marqueurs neuronaux comprenant la nestine (78,12 ± 16 %), la NSE (75,68 ± 6,18 %), la β III tubuline (85,56 ± 10,91 %) et dans lequel l'expression du marqueur neuronal confirme la différentiation des cellules hPNF-MSC en cellules neuronales.

14. Procédé selon la revendication 1, dans lequel l'étape de différentiation des cellules souches/stromales humaines dérivées du tissu adipeux périnéphrique (hPNF-MSC) isolées en cellules astrogliales comprend les étapes suivantes :
génération de sphères avec un facteur de croissance des fibroblastes basique (bFGF) à une concentration de 1 nm/ml dans un milieu DMEM-KO, le milieu DMEM-KO étant supplémenté avec un sérum fœtal bovin (FBS) à une concentration de 1 %vol;
incubation des sphères générées dans le milieu pendant 24 heures ;
remplacement du milieu de croissance par un milieu d'induction astrogliale et une L-thyroxine, le milieu d'induction astrogliale comprenant un FBS à une concentration de 1 %vol un N2 à une concentration de 1 %vol et un T4 à une concentration de 30 ng/ml, le FBS étant un complément alimentaire, le N2 étant un cocktail du facteur de croissance, le N2 comprenant une insuline à une concentration de 5 µg/ml, une progestérone à une concentration de 20 nM, une putrescine à une concentration de 100 µm, un sélénium à une concentration de 30 nM, un B27 à une concentration de 2 %vol et un bFGF à une concentration de 20 ng/ml, le sélénium étant ajouté pour la croissance et le maintien des cellules astrogliales in vitro, le B27 étant un supplément de croissance pour les cellules astrogliales in vitro, le bFGF étant un agent de différentiation cellulaire, le bFGF induisant une différentiation cellulaire des cellules hPNF-MSC en cellules astrogliales progénitrices, le T4 induisant la différentiation des cellules hPNF-MSC en cellules astrogliales et le bFGF ciblant la voie Wnt ;
remplissage du milieu d'induction astrogliale tous les 3-4 jours ;
fixation des cellules avec un PFA à 4 % pour caractérisation ; et
mise en œuvre d'une procédure d'induction sur une culture monocouche des cellules.

15. Procédé selon la revendication 14, dans lequel les cellules souches/stromales humaines dérivées du tissu adipeux périnéphrique (hPNF-MSC) isolées montrent l'expression du marqueur astroglial GFAP (53,31 ± 14.11 %) et dans lequel l'expression du marqueur astroglial confirme la différentiation des cellules hPNF-MSC en cellules neuronales.

16. Procédé selon la revendication 1, dans lequel l'étape de différentiation des cellules souches/stromales humaines dérivées du tissu adipeux périnéphrique (hPNF-MSC) isolées en cellules rénales comprend les étapes suivantes :
mise en culture des cellules hPNF-MSC sur des lamelles couvre-objet autoclaves dans des boîtes de culture de 35 mm comprenant un milieu de croissance épithélial rénal ;
incubation des boîtes de culture à 37 °C dans un incubateur de CO₂ pendant 17 jours pour identifier la différentiation des cellules hPNF-MSC en cellules de mésenchyme métanéphrique, le CO₂ étant à une concentration de 5 % ;
incubation des boîtes de culture à 37°C dans un incubateur de CO₂ pendant 22 jours pour identifier la différentiation des cellules hPNF-MSC en podocyte et en tubule convoluté proximal, le CO₂ étant à une concentration de 5 % ;
remplissage du milieu de culture après 48 heures ;
dans lequel le milieu de croissance épithélial rénal comprend une BMP2 à une concentration de 10 nm/ml, un acide rétinoïque à une concentration de 0,1 µm/ml et une Activine A à une concentration de 10 ng/ml, la BMP2, l'acide rétinoïque et l'Activine A étant les facteurs de croissance induisant la différentiation dans les cellules hPNF-MSC pour former les cellules rénales, l'Activine A induisant une spécification mésodermique, l'acide rétinoïque induisant la structuration mésodermique, l'acide rétinoïque ciblant les récepteurs rétinoïdes RAR, la BMP2 induisant une néphrogénèse, la BMP2 étant exprimée dans un mésenchyme métanéphrique, la BMP2 induisant une épithélialisation d'un mésenchyme métanéphrique ;
réalisation d'un filtrage par seringue du milieu de différentiation épithélial rénal à l'aide d'un filtre de 0,22 micron ;
incubation des cellules hPNF-MSC à une température de 37°C dans un incubateur de CO₂ pendant 17 jours pour identifier la différentiation des cellules hPNF-MSC en cellules de mésenchyme métanéphrique, la concentration de CO₂ étant de 5 % ;
incubation des cellules hPNF-MSC à une température de 37°C dans un incubateur pendant 22 jours pour une différentiation en podocyte et en tubule convoluté proximal, l'incubateur ayant du CO₂ à une concentration de 5 % ; et
remplissage du milieu de différentiation épithélial rénal toutes les 48 heures.

17. Procédé selon la revendication 16, dans lequel la voie Wnt est ciblée pour la différentiation des cellules souches/stromales humaines dérivées du tissu adipeux périnéphrique (hPNF-MSC) en cellule rénale et dans lequel les protéines de la voie Wnt exprimées pendant un développement rénal sont une Wnt 2b et une Wnt 9 et dans lequel les facteurs de croissance de BMP2, de l'Activine A et de l'Acide rétinoïque activent la voie de signalisation Wnt permettant la différentiation des cellules hPNF-MSC en cellules rénales progénitrices.

18. Procédé selon la revendication 1, dans lequel l'étape de différentiation des cellules souches/stromales humaines dérivées du tissu adipeux périnéphrique (hPNF-MSC) isolées en îlots comprend les étapes suivantes :
ensemencement des cellules souches mésenchymateuses périnéphriques humaines (hPNF-MSC) à partir d'un passage 3 (P3) dans trois boîtes/plaques de culture cellulaire non adhérentes ;
étiquetage de deux boîtes/plaques de culture cellulaire comme boîte/plaque de culture de différentiation cellulaire, les cellules hPNF-MSC étant différentiées en agrégats de cellules progénitrices pancréatiques dans les boîtes/plaques de culture de différentiation cellulaire ;
étiquetage d'une boîte/plaque de culture cellulaire comme boîte/plaque témoin, la boîte/plaque témoin étant une boîte/plaque de culture non induite ;
supplémentation de la boîte/plaque de culture cellulaire témoin (boîte/plaque de culture non induite) avec un milieu de culture de cellules, le milieu de culture de cellules comprenant l'élimination du milieu Eagle modifié de Dulbecco (DMEM-KO) ;
supplémentation des deux plaques/boîtes de différentiation cellulaire avec un milieu de culture, le milieu de culture de cellules comprenant l'élimination du milieu Eagle modifié de Dulbecco (DMEM-KO), une albumine de sérum bovin (BSA) en concentration de 1,5 %vol de l'insuline-transferrine-sélénium (ITS) en concentration de 1X ;
incubation des deux boîtes/plaques de culture de différentiation cellulaire et d'une boîte/plaque de culture cellulaire témoin à 37°C pendant quatre jours dans un incubateur de CO₂, l'incubateur de CO₂ étant rempli de CO₂ à une concentration de 5 % ;
prélèvement d'une pluralité de cellules flottantes dans un milieu de culture dans un tube centrifuge après écoulement des quatre jours ;
possibilité donnée aux cellules de se déposer dans le tube centrifuge pendant 15-20 minutes, les cellules se déposant pour former un culot et le milieu de culture formant un surnageant et le culot de cellules étant prélevé ;
décantage du surnageant ;
dans lequel le culot de cellules comprend des agrégats de cellules et dans lequel les agrégats de cellules sont ré-ensemencés dans un milieu comprenant un DMEM-KO, un BSA à une concentration de 1,5 %vol un ITS à une concentration de 1X, 100 nM d'amide de nicotine à une concentration de 100 nM, de la taurine à une concentration de 3 nM et un peptide 1 de type glucagon (GLP 1) à une concentration de 100 nM ;
soumission des cellules à une analyse de coloration à la dithizone (DTZ) et à une analyse d'immunofluorescence ; et
dans lequel les agrégats de cellules sont des agrégats de cellules en îlot.

19. Procédé selon la revendication 18, dans lequel les agrégats de cellules en îlot sont **caractérisés par** la coloration à la dithizone (DTZ) et dans lequel les agrégats de cellules en îlot sont positifs à la coloration DTZ et dans lequel la coloration DTZ est un agent chélatant du zinc colorant de manière sélective les cellules bêta pancréatiques.

20. Procédé selon la revendication 18, dans lequel les agrégats de cellules en îlot sont **caractérisés par** une coloration par immunofluorescence pour le peptide C et dans lequel les agrégats de cellules montrent l'expression du peptide C et dans lequel l'expression du peptide C confirme la génération d'agrégats de cellules en îlot fonctionnels.
